# EUROPEAN PATENT APPLICATION

(11) **EP 1 837 404 A1**
(43) Date of publication of application: **26.09.2007**
(21) Application number: 06111703.2
(22) Date of filing: 24.03.2006
(51) Int. Cl.: C12Q 1/68, G01N 33/573

(54) **Mutant lysosomal type 5 P-type ATPase ATP13A2 and its use for diagnosis and therapy of neurodegenerative disorders**

(71) Applicant: UNIVERSITÄT ZU KÖLN, 50923 Köln (DE); Rheinische Friedrich-Wilhelms-Universität Bonn, 53113 Bonn (DE)
(72) Inventor: Kubisch, Christian, Prof. Dr., 53129 Bonn (DE); Ramirez, Alfredo, Dr., 53115 Bonn (DE)
(74) Representative: von Kreisler Selting Werner

(57) **Abstract**

The present invention provides mutant forms of human lysosomal type 5 P-type ATPase ATP13A2, nucleic acid sequences coding for said mutant ATPase, and their use for diagnosis and therapy of neurodegenerative disorders, especially of parkinsonism and dementia. Test systems for testing candidate active agents for their therapeutic potential in diseases connected with ATP13A2 are also provided.

SEQUENCE LISTING

<110>  Universität Köln
       Universität Bonn

<120>  Mutant lysosomal type 5 P-type ATPase and its use for diagnosis
       and therapy of neurodegenerative disorders

<130>  053147ep

<160>  82

<170>  PatentIn version 3.3

<210>  1
<211>  3543
<212>  DNA
<213>  Homo sapiens

<400>  1
atgagcgcag acagcagccc tctcgtgggc agcacgccca ccggttatgg gaccctgacg      60

atagggacat caatagatcc cctcagctcc tcagtttcat ccgtgaggct cagcggctac     120

tgtggcagtc catggagggt catcggctat cacgtcgtgg tctggatgat ggctgggatc     180

cctttgctgc tcttccgttg gaagcccctg tggggggtgc ggctgcggct ccggccctgc      240

aacctggccc acgccgaaac actcgttatc gaaataagag acaaagagga tagttcctgg     300

cagctcttca ctgtccaggt gcagactgag gccatcggcg agggcagcct ggagccgtcc     360

ccacagtccc aggcagagga tggccggagc caggcggcag ttggggcggt accagagggt     420

gcctggaagg atacggccca gctccacaag agcgaggagg cggtgagtgt cggacagaag     480

cgggtgctgc ggtattacct cttccagggc cagcgctata tctggatcga gacccagcaa     540

gccttctacc aggtcagcct cctggaccat ggccgctctt gtgacgacgt ccaccgctcc     600

cgccatggcc tcagcctcca ggaccaaatg gtgaggaagg ccatttacgg ccccaacgtg     660

atcagcatac cggtcaagtc ctacccccag ctgctggtgg acgaggcact gaacccctac     720

tatgggttcc aggccttcag catcgcgctg tggctggctg accactacta ctggtacgcc     780

ctgtgcatct tcctcatttc ctccatctcc atctgcctgt cgctgtacaa gaccagaaag     840

caaagccaga ctctaaggga catggtcaag ttgtccatgc gggtgtgcgt gtgccggcca     900

gggggagagg aagagtgggt ggactccagt gagctagtgc ccggagactg cctggtgctg     960

ccccaggagg gtgggctgat gccctgtgat gccgccctgg tggccggcga gtgcatggtg    1020

aatgagagct ctctgacagg agagagcatt ccagtgctga agacggcact gccggagggg    1080

ctggggccct actgtgcaga gacacaccgg cggcacacac tcttctgcgg gaccctcatc    1140

ttgcaggccc gggcctatgt gggaccgcac gtcctggcag tggtgacccg cacagggttc    1200

tgcacggcaa aggggggcct ggtgagctcc atcttgcacc cccggcccat caacttcaag    1260

ttctataaac acagcatgaa gtttgtggct gccctctctg tcctggctct cctcggcacc    1320

atctacagca tcttcatcct ctaccgaaac cgggtgcctc tgaatgagat tgtaatccgg 1380

gctctcgacc tggtgaccgt ggtggtgcca cctgccctgc ctgctgccat gactgtgtgc 1440

acgctctacg cccagagccg actgcggaga cagggcattt tctgcatcca cccactgcgc 1500

atcaacctgg ggggcaagct gcagctggtg tgtttcgaca agacgggcac cctcactgag 1560

gacggcttag acgtgatggg ggtggtgccc ctgaaggggc aggcattcct gcccctggtc 1620

ccagagcctc gccgcctgcc tgtggggccc ctgctccgag cactggccac ctgccatgcc 1680

ctcagccggc tccaggacac ccccgtgggc gaccccatgg acttgaagat ggtggagtct 1740

actggctggg tcctggagga agagccggct gcagactcag catttgggac ccaggtcttg 1800

gcagtgatga gacccccact ttgggagccc cagctgcagg caatggagga gcccccggtg 1860

ccagtcagcg tcctccaccg cttccccttc tcttcggctc tgcagcgcat gagtgtggtg 1920

gtggcgtggc caggggccac tcagcccgag gcctacgtca aaggctcccc ggagctggtg 1980

gcagggctct gcaaccccga gacagtgccc accgacttcg cccagatgct gcagagctat 2040

acagctgctg gctaccgtgt cgtggccctg gccagcaagc cactgcccac tgtgcccagc 2100

ctggaggcag cccagcaact gacgagggac actgtggaag gagacctgag cctcctgggg 2160

ctgctggtca tgaggaacct actgaagccg cagacaacgc cagttatcca ggctctgcga 2220

aggacccgca tccgcgccgt catggtgaca ggggacaacc tgcagacagc ggtgactgtg 2280

gcccggggct gtggcatggt ggccccccag gagcatctga tcatcgtcca cgccacccac 2340

cctgagcggg gtcagcctgc ctctctcgag ttcctgccga tggagtcccc cacagccgtg 2400

aatggcgtta aggatcctga ccaggctgca agctacaccg tggagccaga cccccgatcc 2460

aggcacctgg ccctcagcgg gcccaccttt ggtatcattg tgaagcactt ccccaagctg 2520

ctgcccaagg tcctggtcca gggcactgtc tttgcccgca tggcccctga gcagaagaca 2580

gagctggtgt gcgagctaca gaagcttcag tactgcgtgg gcatgtgcgg agacggcgcc 2640

aatgactgtg gggccctgaa ggcggctgat gtcggcatct cgctgtccca ggcagaagcc 2700

tcagtggtct cacccttcac ctcgagcatg gccagtattg agtgcgtgcc catggtcatc 2760

agggaggggc gctgttccct tgacacttcg ttcagcgtct tcaagtacat ggctctgtac 2820

agcctgaccc agttcatctc cgtcctgatc ctctacacga tcaacaccaa cctgggtgac 2880

ctgcagttcc tggccatcga cctggtcatc accaccacag tggcagtgct catgagccgc 2940

acggggccag cgctggtcct gggacgggtg cggccaccgg gggcgctgct cagcgtgccc 3000

gtgctcagca gcctgctgct gcagatggtc ctggtgaccg gcgtgcagct agggggctac 3060

ttcctgaccc tggcccagcc atggttcgtg cctctgaaca ggacagtggc cgcaccagac 3120

aacctgccca actacgagaa caccgtggtc ttctctctgt ccagcttcca gtacctcatc 3180

ctggctgcag ccgtgtccaa gggggcgccc ttccgccggc cgctctacac caatgtgccc 3240

ttcctggtgg ccctggcgct cctgagctcc gtcctggtgg gccttgtcct ggtccccggc 3300

ctcctgcagg ggccgctggc gctgaggaac atcactgaca ccggcttcaa gctgctgctg 3360

ctgggtctgg tcaccctcaa cttcgtgggg gccttcatgc tggagagcgt gctagaccag 3420

tgcctccccg cctgcctgcg ccgcctccgg cccaagcggg cctccaagaa gcgcttcaag 3480

cagctggaac gagagctggc cgagcagccc tggccgccgc tgcccgccgg ccccctgagg 3540

tag 3543

<210> 2
<211> 1180
<212> PRT
<213> Homo sapiens

<400> 2

<210>  3
<211>  25815
<212>  DNA
<213>  Homo sapiens

<400>  3
gcgccggggc cggcgatgag cgcgaggagc cggcatgagc gcaggtgagt gcggagtccg      60

ggtcggcccc gagcccggcg cggtccccgg cccgccagtt gtcgtcaccc cttggaggga     120

gaggggaggc gacgcccccc accgcggggt cgcgagccca ggaagcggga cctggttggg     180

gatcctggcc cgtcccctcc cttttgacgg ggactcggcc ccggtttctt cccagagacg     240

ccggggctgg ggatggcgcc gccgctcgca gccggtccct cccctcccct gcacccctca     300

gccgcgggat tggaagccgg gcgcttgtgc aagagggtcc tggggccgtg cggggacggg     360

gaggccaccc cacgcccact taatcggatc ccaccccgag tgacggatgt ccaggtgggg     420

gagacggctg catcctcggg cctctgggca acccctctgg gctgaatgaa tgaatgaatg     480

gcgttggggg agtcgggact ccatttcctg ggtagatgtc ctgggccaca gcgccactcc     540

tgacctcctg cctcagaccc cagagaaaac ccgtcacacc cagggccaag ggccacaccc     600

tccatgcaac ccatccctgg ccagatgtcc tacgcccact cctccagggt ctaatttaaa     660

tcccacctgc cgggaatggg gccagtcctc ctcccctatc cagaaaggtc tcatctcagt     720

gtctgtcact cagcccccag ccccacagcc tagagtgcac cccccggcga gggaggggaa     780

ggaatgagga aagacagccc ctagaatcca gatccaaaga tgggaagatg gttcacacac     840

actcccgcct cagcctggca gaagacagga gctccccgcc gccgggaacc acagccaggg     900

aggaggcagc cgccctgggc cgctgccagg tcaccagcag gaagccaagg cttccgcagg     960

aaataagagg tcccaaatgg ctctagcccc tctgatccac agtgctggtg acactgcccc    1020

gagctgtgac ctcagacgag gttcaagtcc agtcttggcc cctcatagct gtgtgacttt    1080

gtgcttgtca atctactcct ctgggcctcg ttttaaatgg gaatcctaat ctgtactttt    1140

catggaatgg ctgtgggaaa gaatgtgccc attcagacac actcatttcc agcttctccc    1200

ctttcaagct ctatgaccct ggacaaggga ttcacatgct gttcctcagt ttccccaggt    1260

gtacaatggg gataataata gtcactaccc cagagggttg tgtgtgaagc ttcattgagt    1320

tagcacactg aagggcttcc gcagggcatg gtgagcactc catgggagct gctgttcatc    1380

agaagagcaa tgacaggcca ggcgtggtgg ctcacgcctg taatcccagc actttgggag    1440

cctgaggcag gcggattact tgaggtcagg agttcaagac cagtctggcc aacatgggga    1500

aaccccgtct ctactaaaaa tacaaaaatt agctgagtgt ggtggcatgt gcctgtagtc 1560

tcagctactt caggaactga ggcaggagaa ttgcttgaac ctgggaggtg gagggtgcag 1620

tgagctgaga tctctccact gcactccagc ctgggagacg ggcagggggg aaggggggaa 1680

cgggggggga agactccatc tcaaaaaaaa gagcaatgcc atcatgaagg tgctctcgcc 1740

cccccacaca gcctgtgggt gcctgttctg ggctggtctg agcccacaga agccctgggg 1800

agactgggct gtggctgggg ataaacagat gtactgcagg agagagtgcc ctgggcttga 1860

tccctgcagg gaggggctct ggaggtttcc acagctgtga cttcctccct caagttccac 1920

tccccttttc ctctttgcca aggttgactg agagagtgtc attgtgaagt gacagtcata 1980

cgattgccat gaggaagaac ttcccagggg gaccgctgtt tacatactgg ggactctgcc 2040

ctctttttga agaacctttt cttcataaag agtcttgagg ccgggtgtgg tggctcacac 2100

ctgtaatccc agcactttgg gaggctgagg taggaggatc agcatgaggc cgggagttca 2160

agatcagcct gggcaacaaa atgagaccct gtccctacta aaaaaaaatt agcctggtgc 2220

agtggcacct gcctgtagtg ccagctactt gggaggcaga ggcaagagta cctcctttag 2280

cctaggaggt caaggctcca gtgagctagg atcatgccac tgtgctccag cctgggtgac 2340

agagtgagac cctgtctctt ttaaaaaaaa aaaaaaaaaa aaaaaggaag aggctcaagc 2400

ccctcccccc aactcccagc acctttgcct tcaagctctg ttgtgtccaa gagacagaca 2460

ttcattcatt cagtcagtcg gtcaatcagt gtttcctgac ccctgagcac atggcaggta 2520

ctgtgagcag taagggcagt gacacccatc tgagcctgat gctgagggga atgtcaggag 2580

tagatgtgct gggcagagag gacctgggag taaggccagc cctgttccag ctttgagaca 2640

agtctgaggg caactgcccc agaggctcag tgctctcgtt tgtcaaactg ggtaagagag 2700

cccaccttgt gggttggtgc gggaggaaat caaatcagca ccgcagggat ggctcctttc 2760

ccatcatccc tgagcccgct gggctgttgg cctgagggtt tttttttttt tttttgagtc 2820

ttgctctgtc accctggctg gagtgcagtg gcgtgatctc agctcactgc aagctccacc 2880

ttctgggttc acaccattct cccgcctcag cctcccaagt agctgggact acaggcgcct 2940

gccaccacgc ccggctaatt tggtttttat atttttagta gagacagggt ttcaccgtgt 3000

tagccaggat ggtctcagct agtctccacc tcctgacctc gtgatccgcc ccccttgggc 3060

tcccaaaggg ctgggattac aggcgtgagc cactgcgcac ggccggcctg agggttttaa 3120

ggtacaagga gcaactgttg aatgggccta gggagccgtg ggacagccag ggcttcagac 3180

agggtgatgt gaaaggagaa gagagcctgg gaaatggata agcagaagcc acgcccccct 3240

gacctgggag ccgggtcctg cccacctgtc cacgcaaccc ccccaggaac ttcaaatgct 3300

tctacctccc tgtgtgctgg atgcttcggg actatcatcc tagagatgag aaatggaagc 3360

ccagagaggc aaaggcactt gctgtatgaa acacagcatc aaaaaagaaa ttcttaggaa 3420

caaatgatgg gcaaaaagac aaaaccctgt ctctacaaaa aaaattagct gggcatggtg 3480

gcctgtgcct gtagtcccag ctatttggag gctgaggtgg gagaattgct tgagcctggg 3540

aggtcaaggc tgcagtgagc tatgatcgca acactgcact ccagcctggg tgacagagag 3600

agactgtctc aaaaaaagac ttatacacga aaactgtaag ctgggcatgg tggctcacgc 3660

ctgtaatccc agcactttgg gaagccgagg ctacaagagt gataagtaaa taaatgaatg 3720

aatgaatgaa tgaaaggaca tcacaaccga ggttacctgg cagggttgat acgttagcca 3780

tgtaccctgt gcacagtgag ccctctagga gtgttgaagc cagagtgctt ggggatgggg 3840

ctgctcagag gctgggggtg ctgtgcccac agcagctgcc tggggcactc tggactcttt 3900

gggtagaaat tttagaaggg ctcgccgggc gctgtggctc acgcctgtaa tcccagcact 3960

ttgggaggcc gaggcgggcg gatcacgagg tcgggagatc gagaccatcc tggctaacac 4020

ggtgaaaccc catctctact aaaaatacaa aaaattagcc gggcgtggtg gcgggcgcct 4080

gtagtcccag ctacttggga ggctgaggca ggagaatggt gtgaacccgg aaggcagagc 4140

ttgcagtgag ccgagatcgc gccactgcac tccagcctgg gtgacagagc gagactcgct 4200

ctcaaaaaaa aaaaaaaaaa aaggaaaaat tttagaaggg ctcagattgg gggatgaagg 4260

agggagccca gaaaatcagc agcctacccc gcacccgccc agcttttcca gcttagtgag 4320

ctttttcaga ggctcttgtg aaaccacaga tgcctagcac catccctacc ccggcctgag 4380

gagttgatcc agccagtctg ggagggcccc tggcatctgc atatgtaaaa ggtgctccag 4440

gtggtctggg accgtagatg aaccctgaca gaggctgtcc aagggtgggg ctggtgagct 4500

ctacccgctc tggagcccca gaacatccac tggctgcctg ccctgctaag gccacagctc 4560

tacagcacag gaggaaggag tgaaaagaat gtctgtggcc gggcgcagtg gctcagcctg 4620

tcagcccagc actcagggag gcaaggcggg tggatcacct gaggtcggca gtttgagacc 4680

accctgacca gcatggtgaa accccgtctc tactgaaaat acaaaactta gctgggcgcc 4740

tgtaatccca gctactcggg aggctgaggc aggataatcg cttgaacctg ggaggcagag 4800

gttgtagtga gccgagatca tgccattgta ttccagactg ggcgacagag caagacttca 4860

tctcaaaaaa aaaagaatat ctgcaataga accacctgat accattcccc aagtctcagt 4920

ttacccatct gtaaagatag gcctgcaggg ctgaccgttt attagtccag acttgacttt 4980

gcatctcctc actgccagac ctgggctggg cacgggcatg gaatgaagat aagcatccct 5040

gacccctgcc ctcagggtgg ggactggatg aggatagaaa agtgagaccc agagctccaa 5100

ggaagctaat ggctggatgc acgagcacca tgggggcttg gggcactgtc caggccccat 5160

tctccaattc tgggccatac ccagcagaaa gaccgacaca cgcaaggaaa caagacacta 5220

ctggccaagc acggtggctc atgcctgtaa tcccagcact ttgggaggcc aaagtgggca 5280

gatcacttga ggtcaggagt tcgagaccag cctggccaac atggtgaaac cccatctcta 5340

ctaaaaatac aaaaattagc tgggcatggt ggtgcatgcc tataatccca gctactcggg 5400

aggctgaggc aggagaatcg cttgaacctg ggaggcaaag gttgcagtga gctgagatca 5460

agtgactgca ctccagcctg ggtgacagag tgacactccg tctcaaaaaa aaaaaaaaga 5520

tacggtaagt aagaaccgac ggaaagaaga gccagataaa ggacacagga agacatgagc 5580

tttgggctct gtcagaccag gtgcttactg ttttgtgttt tgttttgttt agttttttga 5640

gatggagtct cactctgttg ctcaggcagg ggtgcagtgg tgtgatcttg cctcactgca 5700

acctctacct cccaggttca agtgattccc ctgcctcagc ctcctgagta gctgagatta 5760

caggcacgcg ctgccacgcc cggctaattt tttttatttt tagtagagat gggtttcacc 5820

atgttagcca ggttgatctc gaacttctaa cctcaggtga tccacccgcc tcggcctccc 5880

aaagtgctgg gattacaggc gtgagccacc gcgcccaggc tgatgtttat tgtttttaag 5940

gaagcaagag gagaaggtgc ctccccaaat gacctttctc ttttctgtcc acagacagca 6000

gccctctcgt gggcagcacg cccaccggtt atgggaccct gacgataggg acatcaatag 6060

atcccctcag ctcctcagtt tcatccgtgg taagtgggca gagtggggtg gggaaaagct 6120

gcagggggtg aatgcccagg cccacccaga caccccaact tcctactcca gccccgttcc 6180

caccccgacc tgggccctct ctcgctgcgt tcgcagaggc tcagcggcta ctgtggcagt 6240

ccatggaggg tcatcggcta tcacgtcgtg gtctggatga tggctgggat ccctttgctg 6300

ctcttccgtt ggaagcccct gtggggggtg cggctgcggc tccggccctg caacctggcc 6360

cacgccgaaa cactcgttat cgaaataaga gacaaagagg tgagagtcag gctgagcctg 6420

gggctagaga agcgcagtga gggtcagggt cgggggtgga ggatgcttgg gtgccaaccc 6480

ggccactgac tttccatggg acattaggca ggtcccgctc ttctctggac ctcagctttc 6540

ccatctgagc attgggcaga agggacgggt cgggtggttt ctggagggct ctccatagcc 6600

ttgatttctg ccttctccag caggggccgc ctggctgtag gacagggcct gggaggggag 6660

gggactcttc tgagactgag tccatacctc tgtgttccag gatagttcct ggcagctctt 6720

cactgtccag gtgcagactg aggccatcgg cgagggcagg tgaggcagcc ccattgcccc 6780

ttcccagaag cccctgctac cctggcctcc ccaacttccc caaccctcgg cccccccata 6840

aaggctgact ctggcgcctc ccatcctagg gagggaagat ggctctgtga gcacagggag 6900

gtgccagggc agctcccaga gctcgagagt aaccctcacc tgcttcccca gcctggagcc 6960

gtccccacag tcccaggcag aggatggccg gagccaggcg gcagttgggg cggtaccaga 7020

gggtgcctgg aaggatacgg cccagctcca caagagcgag gaggcggtga gtgtcggaca 7080

ggtgggttct gaaaaagtcc ttgcccctct ctcggcctcg gtttcatgat ctgtgcaatg 7140

ggagagtcag acttcatgtt ctcccaccac cttttagttc tgttgtccca aacatccagg 7200

atgctgtctg agcacgttta cactttcagg cctctcccca cctctctgtc tggtgacacc 7260

agtccctgcc ccctaccccc atccctcggc tcccagcata agcacactgc ttctggggcc 7320

ggggccagct tccaacccat cctctcctct ctcccaccca gaagcgggtg ctgcggtatt 7380

acctcttcca gggccagcgc tatatctgga tcgagaccca gcaagccttc taccaggtca 7440

ggtaggagtc agggcctgtg tggctcatgt cacctcccag agtggcactg gtttgggcac 7500

agacggtggc atggcatggc ctgggctccc ctctcagctc tgatgctttt gctgtctgaa 7560

accttgggca ggttactgag cccctccagg cctgggatct ctgtctataa atagggcatg 7620

aagataactt ctgcctccta ggattgtttt gatggtgaaa tagataataa tttatgtaga 7680

gcttggcacc gaatgtgatc tgaggttgtg acaccactgt gaatcagact atcagctctg 7740

tactcagact gtctgcttgg ggtttagtca ctgctctgcc acctacaaaa ggcttaagaa 7800

acatgagctc aggctgggtg cggtggctca cccctgtaat cccagcactt tgggaagcca 7860

agacaggtgg atcacgaggt caggagttca agatcagcct ggccaagatg ctgaaacccc 7920

atctctacta aaaacacaaa aattagctgg gcgtgatggc atgtgcctgt aatcccagct 7980

acttgggagg ctgaggcagg aaaatcgctt gaacctgggc ggcagaggtt gcagtgagcc 8040

aacatcgcgc cactgcactc tagcctggat gacagagcat gactccatct caaaaaaaaa 8100

aaaaaagaaa aagaaacaca agctcaccac acaccttagt ttgtgtgtat gtgtgtgtgt 8160

gtgtgtgtgt gtgtgtgtgt gtgtgtggtt tttttggttt gtttgtttgt tttttaagat 8220

ggagtttcac tcttgttgcc cgggctggag tgcaatgccg cattctcggc tcactgcacc 8280

tccgcctcct gggttcaagc gattctcctg cctcagcctc ccgagtagtt gggattacag 8340

gtgcatgcca ccacacccag ctaatttttg catgttttgt agggacgggg tttcaccatg 8400

ttggccaggc tgttctcaaa tacctgacct caggtgatcc acccgcctca gcctcccaaa 8460

gtgctagaat tacaggcatg agccaccggg ccctgctcac acactttagt ttgaatgccg 8520

cagaagcaga cccccagaca agggctcaag ggcagggagt ttgttttgaa gctgatccca 8580

ggcagtgctg gggaagggat ggaagcacgg agggaggtta tgctggctgc cacagctgtg 8640

gacacccgga cttcagtcgc ctggggatct ccttgggcca gcgtagacca cacacctctg 8700

ggcgacctca cacagggagt gaggacagtt accatcaaat tccctgccat tatcagtcac 8760

gggacacaaa gtcttccacg ctttccgggc tcacaggcag gggattcaca tgcagccaga 8820

aggcctcagg taaagacagg tgttttcagg gtccagctgt catgtggagg tgaggcagag 8880

gggagatggg gatggcagca acagcctctg ctgcatgatg gggataatag caaaaatcac 8940

ccgtgcattc atcacttagc aagtggcagg cacgtccaaa gcagtccccg aggcttgccc 9000

cacttaatct acccatggcc ctatgtggta ggtgctgtca ctgcccccat tttgcagatg 9060

agaaaactga ggcacagaga ggttaagtga cttgcccagg gtatcatcag tgtaaatagt 9120

gcccttatca ctaaataaca gctactcttt tcactgttca tattattagc atcatctctc 9180

gctgttctcc ttcttcagtg acttggtttg gaacccagtg agaggaacaa aagggcaaag 9240

ccaagccttg ggttcaaggg ggcttacttg ctcccaggag ctcagtgtgg cagaacgggg 9300

atggggggcc tagagcccat agtctccagc ctgcacaggg tgggggatgc agacctcagg 9360

ccccatggcc catgcgtgtc ccctcgcacc cacccccagc ctcctggacc atggccgctc 9420

ttgtgacgac gtccaccgct cccgccatgg cctcagcctc caggaccaaa tggtgaggtc 9480

agtgctgccc tggaccgggg tgccctgaga actggggctc cttgtgttgc cccccaatgt 9540

ggttgccttc cagtcacctg ccttctctgg gcctcagttc ccccagctga aagtggggag 9600

ctccttcacc ccgggagggt ccagccacag ctcctccttc tgatggcttg gcctcatccc 9660

ctcttccagg aaggccattt acggccccaa cgtgatcagc ataccggtca agtcctaccc 9720

ccagctgctg gtggacgagg tagggctgtc cctgggtctt gccccagccc tgcctggcgt 9780

gctgtgtgac cttggcccag ggcttgcctc ctctgggcca ctttacggtg gcaaccagac 9840

cagctgtccc cttctccata gtggatcgtc atctcagaac ctgccgcagt cagcctagcc 9900

agagagacca ggtccaggaa ttggaggttc tgcccaaggc ttgggtcaca gattggggcc 9960

ccactcatag ggattggagg ccaagaggca aatccacgca gcctgagcat ctatggggtc 10020

cccagagaca cgccctagag cctgggtcct ctctgtgcgt cctccacttt catgctccca 10080

acagtcccgt gggttgggcc tgggactgca ctgttcacac ctgagaaatc caagcttcag 10140

ttggaggctg tgacatggcc aaggtcacac agggacaaag agaagccagt gacctggaca 10200

caactagaat aaagtgtgaa catcccagcc ctctgatcgg gggcaggcac catgaaagag 10260

atctttgagt ctggccccat agaaatcagt ctgtcactta gaagctatgt ggttttttgt 10320

tttttgtttg ttgttttttt ttttgagaca gagttttgct cttgttgccc aggctggagt 10380

gcaatggcgt gatctcggct cactgcgatg tccacctcct gggttcaagt gattctcctg 10440

cctgagcctc ctacatagct gggactacag gcgcctgcca ccacgcccag ctaatttttt 10500

tttttttttg tatttttagt agatacgggg attcactatg ttggctaggc tggtctcaaa 10560

ctcctgacct cagatgaccc acctgcctcg gccttgcaaa gtgctgggat tataggcatg 10620

agccaccgca cctggctgga actatgtggt tttaggccag tggcttcacc tgtccatgcc 10680

ttggtttcct catctgtaga atggagctaa tggtggtacc ggccttgtag aattgtcatt 10740

gccattgtat gacacttaga taaagcactt ggcgtgtagt gggggttcag taaataccct 10800

tatcctattt atttatttgt ttattttttt tgagacaggg tctccctctg tcactcaggc 10860

tggagtgcag tggcgcgatc tcagctcact gcaccctcca cctccccagc ttaagtgatc 10920

ctcccacctc agcctcctga gtagctggga gcgtaggcac aggccacccc atcaggctaa 10980

tttttgtatt ttttgtagat atggggtttc gccatgttgc ccaggctggt cataaaccct 11040

tgtcctcttt agagcggaga aactgagggc cctggggatg tctggttggg tggggatggg 11100

attgaccagg agaaaggcct ggctccagtc ctttggcagc cactgggccc tctgctccca 11160

tgggcacagg agacccagcc tgtggctggg ctgccctggg ggacgcggcc ctgacgctgc 11220

ctcccgcccc tcccccaggc actgaacccc tactatgggt tccaggcctt cagcatcgcg 11280

ctgtggctgg ctgaccacta ctactggtac gccctgtgca tcttcctcat ttcctccatc 11340

tccatctgcc tgtcgctgta caagaccaga aaggtgagtg ccataaggtg ggtggggacg 11400

ggacaggtgg gtccccaggg gcccccaccc actcacggcc ctcttgtccc ctgcccacag 11460

caaagccaga ctctaaggga catggtcaag ttgtccatgc gggtgtgcgt gtgccggcca 11520

gggggaggtg aggagctagg gcccaggggg cgggggcagg agtgggaggg tgggggggca 11580

tggctggggc tggccccacc tagctgagct cctctcctgc ctgcctgcag aggaagagtg 11640

ggtggactcc agtgagctag tgcccggaga ctgcctggtg ctgccccagg agggtgggct 11700

gatgccctgt gatgccgccc tggtggccgg cgagtgcatg gtgaatgaga gctctctgac 11760

aggtgagccc ccagcccctg gcctgcagct tccttcctcc cctgccatcc aaccctttgc 11820

cctgcggcaa aagttttcat ttgagtttcc ttttcctttt ttgttttttt gtgagacgga 11880

gtttcactct gttgtccagg ctggagtgca gtgacatgat ctcagctcac tgcagcctcc 11940

accccccagg ttcaagtgat tcttgtgcct cagcctcctg agtagctggg accacaggtg 12000

cataccatca cgcccagcta attttttatt tttagtagag atggagtttc actatgttgc 12060

ccaggctggt cttgaactcc tgacctcagg ttatctgccc acctcggcct cccaaggtgc 12120

tgggatttca ggcgtgtgcc agcgctccgc ctcctcttcc tttttctctc aagcaacaat 12180

cccagcttta ggacttgcta acctcacacc cctgggccgt taccacgtat catctccgtg 12240

cctcagttcc ttcttctgta agatggctgt cagtcactcc tcctggacag agttgtgcaa 12300

agtgcttagg agcgtgagag gtgcagcata aatggccagc cacccagtgc atctgggcta 12360

tttctccttt tttttttttt tttttttttt ttgagatgga gtttcactct tgttgccccg 12420

gctggagtac catggtgcga tctcagctca ctgcaacctc cacctcctgg gttcaagcga 12480

ttctcctgcc ttagccaccc aagtacctgg gattacaggt gtgcgccacc atacccggct 12540

aattttgtat ttttagttga gatggggttt caccacgttg gtcagactgg tcttgaactc 12600

ctgacctcaa gtgatctgcc cgccccagcc tcccaaagtg ctgggattac aagcatgaac 12660

catagcacct ggctgcatct gagctgtttc tataccagtg cccaccttca ggtcataaag 12720

attccagatg gcacaggaga gtgggaagcc ctctcccaga gtgagagact gcggtcccca 12780

ttcactaccc cacagtcccc ttcccttccc ctccccaggg cagccactgt gaagggtgtg 12840

aggtgtcttc cacgcctttt tctgcattac gtgtgtgcac atgggctgat ttgcctctcc 12900

ttttgttctg aacgtgggat tgtatgctgt gtattgttcc atactttgct ttttctcctg 12960

gcatccttcc acaagcacgg atggccccac ctcatggctg catgagagtc cacaatacgg 13020

atgtatcgta actgactcaa ccggcccgct cgccatgagt atttagaatg tgattttcca 13080

gccattgcaa aggacgccac aggagcagct ttgtcctgtg tctcttgatg tgtgcaagta 13140

tttctttctc tgagatgtgt tatattttgg tggtagatac tggagagagt ttactaaaaa 13200

ccacaggata gtggtttttt tccagggaag ttttctggct ggcattgtat ttgtatcaca 13260

aatcgtaggc caggcgcagt ggctcgcacc tgtaatccca acactttggc aggcagaggt 13320

gggtggattg cctgaggtga ggagttcgaa aacagcctgg aaatcatgtt gaaaccccat 13380

ctctaccaaa aatacaaaaa attagccagg tgtagtggtg cacgtctatc gtcccaggta 13440

cttgggaggc tgaggtggga ggatcgtttg agcctaggag gtggaggttg cagtgagccg 13500

agatcgcacc actgcactcc agcctgggtg acagagtaag actccgtctc agaaaacaaa 13560

aaaaaagtag taattgtgat cagtatgatt tgtgggcaca cgccctgagc cctgccctcc 13620

acaaactgtc cacacatact tcatctgctc atctcttgtg aggcagacat ggcaggattg 13680

tctgactccc ttgcaggaat gtgcacacag taggtgctca atagtcccag cctgcaggca 13740

ttcagtaagg gagtgccact gtttgctagg cagcctgccc aggacttcac agggccatgg 13800

cgagcatgca gtgcttttct cactccaact catggaaata tttgccccct ccatgtaaat 13860

atttctctcc cctccccctc ctccttcccc ctccccttcc ttaccccatc ccctcccctc 13920

ccctcctctt ttgagacaga gtttcactct gttgtccagg ctggagtgca gtggcacgat 13980

ctcggctcac tgcaacctcc gcctctctgg ttcaagttat taccctgcct tagcctccca 14040

agtagctggg attacagatg cccaccacaa cacccggcta atttttgtat tttagtagag 14100

acagggtttc accatgttgg ccagcctggt cccgaactcc tggcctcaag tgatctgccc 14160

acctcagcct cccaaagtgt tgggattaca ggcgtgagcc accgcatctg gccccttgta 14220

aatatttctg ttggttggag ttagaaaagc actccatgca tgttataaaa taattccagc 14280

agaaagagaa cgactccccc accctgcccc actgcacaca tgatgcccac acatgctctg 14340

tctgacccac ccccccccgc tggttccagg gagctggctg ccacctgctt ccaggagtgt 14400

ctccctgtgc ctttatatct ccgtcctctc tgagcctcag tgttcctctc tgtaaaatgg 14460

ggctgtgatt tcttgttcac acctggttac agagttaact gagacttaga gcttgtggga 14520

caattctaca cagaactcct ggtttgctgg tgggagggag gggtgtggag tggtcctcac 14580

accacgttcc ccaccaggag agagcattcc agtgctgaag acggcactgc cggaggggct 14640

ggggccctac tgtgcagaga cacaccggcg gcacacactc ttctgcggga ccctcatctt 14700

gcaggcccgg gcctatgtgg gaccgcacgt cctggcagtg gtgacccgca caggtatgag 14760

ccgggaggct gggcttgaga gagatcccgg gctcagcacc cttgggagag gtggagtgag 14820

gcagggtggg acctggaacc ctggacccca cgcccacctc tgctgctgcc ctgctgtggg 14880

acctcgggcc agttgccccc catctggggt ctgagcattc ccatcttagc agaagttggg 14940

ccagactaag gccaagcatt gcaagatctg gaggagccgc cggggttaca gtgctggagc 15000

gggcagggat gtagcgggca ggcaggagcc ttccctgtgt ctggattttc agtggaacca 15060

cccagtgttt agatgctatc cactagttcc ttgtttaaag cccttaaaaa gcctacaaga 15120

gccgggccag acctgttagg actccacact gctgatggct aatttcacaa acatcatgtc 15180

ttgcaactcc agaggcacgg gtcgggaggg ctaggggtgg ggaaggcctg ccctcaccct 15240

ggcttccctg ccttggggtc cccgcctcct ccccagggtt ctgcacggca aaggggggcc 15300

tggtgagctc catcttgcac ccccggccca tcaacttcaa gttctataaa cacagcatga 15360

agtttgtggc tgccctctct gtcctgggtg agtggccccc tgcaccctcc tctgcccata 15420

gcccccccca cctgggtccc aaatcccact cagtccttcg ctgtccccac agctctcctc 15480

ggcaccatct acagcatctt catcctctac cgaaaccggg taagccttgg gggtggggtg 15540

gggggtgctg ccagcagggc ccaggggcca accagccagg tgctcacatt catggtgccc 15600

cctgccaggt gcctctgaat gagattgtaa tccgggctct cgacctggtg accgtggtgg 15660

tgccacctgc cctgcctgct gccatgactg tgtgcacgct ctacgcccag agccgactgc 15720

ggagacaggg cattttctgc atccacccac tgcgcatcaa cctggggggc aagctgcagc 15780

tggtgtgttt cgacaaggtg ggccgcaggt ggggttggt gcaggggagc gggacagcca 15840

caggcgcccc agtgaggcct ccacacccgc gttattctct cagcagcacc cacaggatgc 15900

caggtcctgt ctagggatgg aaacccccag aggatccacc ccatcccact cctgccctca 15960

ctgggctcac ggtctggcag atgaaatgtc tagaatcatg tattcatgtg ttcaagaact 16020

ggtggggggc cgggcgcggt ggctcacgtc tgtaatccca gcactttggg aggctgaggc 16080

agatacatca cttgaggtca ggagttcaag accagcctga ccaacatggc aaaaccccgt 16140

ctactaaaaa tacaaaagtt agccaggcat gatggcacat gcttgtaatc ccagctactc 16200

gggaggctga ggcaggagaa tcgcagaggt tgcagtgagc caagatcatg ccattgtact 16260

ccagcctggg tgacagagca aaactcaaaa aaaaaaaaaa attactggtg gggacctggg 16320

gccctagtgt gcatcccgtg ctgctcccaa cccttggggc ccagcagtga ccaacacagg 16380

catccttgtg gtcactggag tgtgtgtgtc ccccaccaaa gtaagtgatc caggagtgca 16440

caaaggagct gctggggctg aggagtgggc agtgggtttt aggaaggaag ccagtgggat 16500

agcttgatgg gttctagatg gggacacttc cttagagaga aggcagtcaa ggaagccccc 16560

tgagcaggtc cctcagtgac ctcagtgagg ggcaggtcga ggagaagcaa gggtaaaagg 16620

tgggagtgtc atagcacaga tgtaccgtgt gcctggggca cagggagtgg cgtggcacag 16680

atgtgctgcg tacctgagca cagggagtgg tggagcagga cagggccggg taggcagtgg 16740

cctggttgac agaggctagg tgacattcag aggctgtcgg gggccacaga gcgcttttaa 16800

actggggagg gggcccagcg cagtggctca tgcctggaat cccggcactt cgggaggccg 16860

aggcgggtgg atcatgaggt caggagttca agaccagcct ggccaatatg gtgaaacccc 16920

gtctctactg aaaatataaa aaattagcca ggcacggtga catatgcctg ttgtcccagt 16980

tactcgggag gctggggcag gagaatcgct tgaacccggg aggcagaggt tgcagtgagc 17040

tgagatcaca ccactgcact ccagcctggg caacagagtg agactccgtc tcaaaaaaaa 17100

ataaataaac cggggagggg gatgaccaga ttcttatagt tcccaggcta gccaaggagc 17160

cagacgcgta cttgggcagt tagagtctag gatggggaac gcaggcactg actcatgcgc 17220

tcattcaaca gatatattga ggctgggcgt ggtggctcat gcctgtaatc ccagcactct 17280

gggaggccaa ggcgggtgga ttgcctgagg tcgggagttc gagaccagcc tgaccaacat 17340

ggtgaaaccc cgtctctact aaaaatacaa aaattaggtg ggcgaggtag cgcgtgccta 17400

taatcctagc tgctcgggag gctgaggcag gagaatcact tgaacccggg aggtggaggt 17460

tgcagtgaac tgagattgcg ccactgcact ccagcgcggg cgacagagca agactccatc 17520

tcaaaaaata aaaataaata aaaataaaaa taaataaata aataaataaa taaatgagag 17580

agagagagcg agccaaccgt gggccgggtc cttaaccaca atgcacaagt gtggggaagt 17640

gaagaggcca tgggtaggag cccagagcaa accacctgcc ctaacttggg agttaaggag 17700

gaagacaccc tggaagagat gatgctagac agagagacgg tgggggctgg ggagggggac 17760

cccagcagag ggaatcacat gtgccaagga cagcagtcaa gaaaggtcat cttttggctc 17820

cagctcgagt tcctggggtt cccatagggt ccgccgggcc tggggtttgg aggcccagct 17880

cagcagggta cccccatccc aggactcatc gctgccccac ccacctgtct cccacagacg 17940

ggcaccctca ctgaggacgg cttagacgtg atgggggtgg tgcccctgaa ggggcaggca 18000

ttcctgcccc tggtcccaga gcctcgccgc ctgcctgtgg ggcccctgct ccgagcactg 18060

gccacctgcc atgccctcag ccggctccag gacacccccg tgggcgaccc catggacttg 18120

aagatggtgg agtctactgg ctgggtgagg aggccaagca ggtcagcccc ctgcctctgg 18180

gcagtggggc ctaacgaatc ccaccctgtc ttccctctcc atctttcagg gtggtctctt 18240

attatgccca ccattaataa taataggcca ggcgcagtgg ctcacacctg taacgtggct 18300

cccggcgctt tgggaggcca agacgggagg atcacttgag cccgggaccc cagcctgacc 18360

tacatggcaa aaccccatct ctgccaaaaa tacaaaaatt agccgggcgt ggtggtgcat 18420

gcctgtagtc ccaggaggct gaggtgggag gattaattga gcccgggagg gcgaggctac 18480

agggagctgt gatcacgcta ctgcactcca gcctgggtaa cagaacgaga ccctgtctca 18540

aaaaataata atgataggcc aggtgcagtg actcatgcct gtaattccag cactttggga 18600

ggctgaggtg ggcagatcac ctgaggtcgg aagtttgaaa gcagcctggc caacatagtg 18660

aaaccccgtc tgtaataaaa atacaaaaat tagccaagtg cggtggaaca tgcctgtagt 18720

cccagctact tgggaggctg agggcaggag aatcatttga atccgggagg cagaggttgc 18780

agtaagccga gatcacacca ctgcactcca gcctaggtca cagagtaaga ctctgtctta 18840

aaataaaagt aacataataa taatggcaag gcctcaggct gagtgcctac tatgcgccag 18900

cgcacggcca cagaggcctg gtctcatttc ctcttcccca agccctagca agcaagctca 18960

aatgttgctt cacggatgct gaggctggga gatttggagt gacgtattcc aggtcaccca 19020

acagcttgta agtagcaaag ctagagtttg aatttgagtc caccgggctc caaagcccac 19080

attcttaacc caccattcag tctcccatga aggaagtctt ccctgagtct agctcaaatc 19140

tctctgtgag ccaaagcccc cagacaccaa acttccctgc ctgcctgcca ggtcctggag 19200

gaagagccgg ctgcagactc agcatttggg acccaggtct tggcagtgat gagaccccca 19260

ctttgggagc cccagctgca ggcaatggtg agctgagggg ggcctgttgg gtgcagggca 19320

gaggggtggg ctccccagcc agcacctcag ctgcctctcc aaccctgcag gaggagcccc 19380

cggtgccagt cagcgtcctc caccgcttcc ccttctcttc ggctctgcag cgcatgagtg 19440

tggtggtggc gtggccaggg gccactcagc ccgaggccta cgtcaaaggc tccccggagc 19500

tggtggcagg gctctgcaac cccgagacag gtgcaggggg aagcccctga gtccccctgg 19560

ttgggcattg gcacagaatg gggtgggtac ccagcctctg tccctgccat cccctccttg 19620

tgacacctgc ctctccctgg cagtgcccac cgacttcgcc cagatgctgc agagctatac 19680

agctgctggc taccgtgtcg tggccctggc cagcaagcca ctgcccactg tgcccagcct 19740

ggaggcagcc cagcaactga cgaggtgggc ctgtctcctg tcccacccac tctgaggact 19800

aggcagaggg aggctctcac gcaggcagag cccaaggaag atgccctgcc tggtggctgg 19860

gagaggggcc ctggccactg catgacctct gatccaggtc tgcccaccct ccagggacac 19920

tgtggaagga gacctgagcc tcctggggct gctggtcatg aggaacctac tgaagccgca 19980

gacaacgcca gttatccagg ctctgcgaag gacccgcatc cgcgccgtca tggtgacagg 20040

tacaatggca tgtaggggcc cgggctagca gggcagaatc cgccccttcc tggcagagaa 20100

gaggggcacc agggattcct tttgggggca tctttttggg cccttgcttc aggccaggtc 20160

ctttttaaac atttcgtgaa tcttcacaat atcccccccg caagtggtaa ttgtcacccc 20220

tgtttgacag atgaggaaac tgagactcag aggagtggag tgacttccaa ggaacccatg 20280

tgcgctgtca gacagcctgg gttccaggtg gcacctctga gaaatgggga taatgatgtc 20340

agcctcagag gctgtgacaa gctcaaggag caggaagaga aagattctgg ccgggcacgg 20400

tacttcatgc ctgtaatccc agcactttgg gaggccaaag gagaaggatg tcttgaaccc 20460

tggagttcaa gaccaacctg ggcaacatgg caaaactccg tctctacaaa gaatacgaaa 20520

atggccgggc acagtgtctc acgcctgtaa tcccagcact ttgggaggcc gacgcgggcg 20580

gatcacttga ggtcaggagt tcgagaccaa cctggccaac atagtgaaac cccgtctcta 20640

ctaaaaatac aaaaaattag ccaggtatgg tggtggatgc ctgtaatccc agctactcgg 20700

gaggctgagg caggagaatc acttcaaccc gggaggtgga ggttgcagtg agctgagatc 20760

acgccatttc actccagcct gggcaacaga gagagaccca gtctcaaaaa aaaaaagatt 20820

ctagctaggg tgagacagca ggagctaaga ggtctgtgtc cttgtcctcc ccgcccctgc 20880

acacacacat atgtgcacac acattcatgg gcacttggca gcagagtcac acagcagtaa 20940

ggagtatatg tagcctgggc acatggctcg tgcctgtaat cccagcactc cgggaggccg 21000

aggcaggggg atcacctgag gccaggagtt caagaccaga ctggccaaca tggtgaaacc 21060

ccatctctac taaaaatgca aaaaaaatta gcctggtgtg gtcgcaccct cctgtaatcc 21120

cagctactca agaggctgag gcaggagaat tgcttgagcc caggaggaag gaggttgcag 21180

tgagccgaga tcaagccact gcactccagc ctgggccaca gagagagact cttgtcttaa 21240

aaaaagaaaa aaaaggagta tatgtggagc tccagcccac ggctccaggt tctcctaaga 21300

cccttcaatg cctggatcta cagatgagaa aactgaggca aagggatgtt ggtaacctgc 21360

ctcagctcac ttggctggtg gtggcagacg gatttcatcc agcctgtttt cccatctgta 21420

aggatcagga tgaggatggc ctccccggat agcctcagac cctcaccttg ccccataacc 21480

cccaggggac aacctgcaga cagcggtgac tgtggcccgg ggctgtggca tggtggcccc 21540

ccaggagcat ctgatcatcg tccacgccac ccaccctgag cggggtcagc ctgcctctct 21600

cgagttcctg ccgatggagt cccccacagc cgtgaatggc gttaaggtga ggctaaccca 21660

gagttccagc tgtacctcac ccagtgaccc cacccctgtg tcacctgacc caggccccaa 21720

ctctccaggg tgggcagggg gagctaagct gagcttcctg ggcccccagg atcctgacca 21780

ggctgcaagc tacaccgtgg agccagaccc ccgatccagg cacctggccc tcagcgggcc 21840

cacctttggt atcattgtga agcacttccc caagctgctg cccaaggtag ggccgccccc 21900

agctccctgg ccgccctgcg cctcccctgc tctgtctcct tcctcacggc tcgctctccc 21960

tggtgggtgc ctcagccctc ccccatctcc ctgtgctccc aggtcctggt ccagggcact 22020

gtctttgccc gcatggcccc tgagcagaag acagagctgg tgtgcgagct acagaagctt 22080

cagtgagtgc tcggcagagg gtgtgggcaa gggcctgcgg agactgagct tgtccgttcg 22140

tctgttcatc tgtccttccc cagggccctg tctccctcct caggcaccag agagctgtca 22200

cccctcctca ccaaaagcag gcaggctctc ccaggtcttt catgctaaaa ataataattg 22260

taggctgggt gcggtggctc attcctgtaa tcccacctct ttgagtggcc gaggtgggag 22320

gatcacttga gcccaggagt ttgaggccag cctgggcaac aaagtgagac cccatctctt 22380

attaaaaatt taaaaattag ccaggtgtgg tgacacaccg gtcatcccag ctactcaaga 22440

ggctgaggca ggagggttgc ttgagcctgg gaggctgcgg ctgcagtgag ccatgatcac 22500

accactgcac ttcagcctgg gcaacagagc gagactccgt ctcaaaaaat agtaataggc 22560

caggcgcggt ggctcatgcc agtaatccca gcgctttggg aggctgaggc aggcagatca 22620

cctgaggtca ggagttcaag accagcctgg ccaacatggt gaaaccctgt ctctactaaa 22680

aatacaaaaa ttagttgggc atggtggcgc acaccagtaa tcccagctac tcaagaggct 22740

gagggcactt gggcagcaga ggaggttgta gtgagcagag atcacgccac tgcactctag 22800

cctgggcaac agagggagac tctgtctcaa aaaataatag gccgggcacg gtggctcatg 22860

cctgtaatcc cagcactttg ggaggccgag gcgggtggat cacctgagat caggagttta 22920

agaccagcct ggccaacatg gtgaaacccc atctctacta aaaatacaaa aaattagcca 22980

ggcgtggtgg cgggcgtctg taatcccagc tacttgggag gctgaagcag gagaatcact 23040

tgagcccagg aggcagaggt tgcagtgagc tgagatcatg ccattacact ccagcctggg 23100

caacaagagt gaaactccgt ctcaaaaaat aataataata atgataataa taataataat 23160

tgcatctatt cactgagcgc ctcctgtaca ccaggcacca tgtggcatta ggggcccagc 23220

tgtcatcata ttctgccagc ccacccatgg ggtggtgggg taattcccca ggccacacct 23280

gtccacacct ctcttcaggt actgcgtggg catgtgcgga gacggcgcca atgactgtgg 23340

ggccctgaag gcggctgatg tcggcatctc gctgtcccag gcagaagcct cagtggtctc 23400

acccttcacc tcgagcatgg ccagtattga gtgcgtgccc atggtcatca ggtaaggcag 23460

gcagggaccg gtgggtgagg gctcagcagg gctggtcagc caacctgggt cccctaatgt 23520

ccgtgccctg ccacccaggg aggggcgctg ttcccttgac acttcgttca gcgtcttcaa 23580

gtacatggct ctgtacagcc tgacccagtt catctccgtc ctgatcctct acacggtgag 23640

tatctgcaga gctccgtacc cagagccgtc ccaggactag ggaggggaca cagccctgtg 23700

ccttggagct ggcaggtccc agaatagatg acgtgggccc agatcctgac cctgccactc 23760

tccggctgtg tggttggaga ggtggcaagg cttgggggag atgagaaaaa caccaggcac 23820

ggagcctggt acccactagg tcctcacaaa tggcagcagc tgtcatgggc taacatcttg 23880

accgcaactc tgggagctcc ccaatctgca gggaagactg aacggaggca gggccaaaac 23940

cctcgccccc gccccatccc cacaggtctg cccatttccc cagatgcact tgctttgccc 24000

cataccttgg ctgtacctcc cacaccctac ttggaggagc tctcccctcc cctgctctgg 24060

gcatggcctg ctcaccccca gcgaatgagg tgacactgca gggcggcgca catcttatac 24120

ttgggtctgt ccctgtgtct gcctgaaagc gaggagctcc ctgagggcag gaactgtgca 24180

tgccctggta gctataggga ctccgcaagg cagagccgaa gcctcccctt cgggccggga 24240

gtttgttaca gggagggaaa cctcccctca gatttaggcc cccaaggtca gcacaggtgc 24300

cctttccatc agaccagggg tttgtctcac atcaagagcg gcacctcctc cgctgggctg 24360

gcaggagagt acggccccca ttagactggg ggatgcctta cccatcagat tccttcgggg 24420

caggggtggg gcctctgcta tcagactagg ggctgccagg gccaggtctt agtttcccct 24480

ctgtcctccc ttccctgtgg cagatcaaca ccaacctggg tgacctgcag ttcctggcca 24540

tcgacctggt catcaccacc acagtggcag tgctcatgag ccgcacgggg ccagcgctgg 24600

tcctgggacg ggtgcggcca ccgggggcgc tgctcagcgt gcccgtgctc agcagcctgc 24660

tgctgcagat ggtcctggtg accggcgtgc agctaggggg ctacttcctg accctggccc 24720

agccatggtg agtagggagc tgactgacca cccccatccc gccccttccc tgtccacccc 24780

acgttcgttc cctgaccccc atccctgtac ccccaggttc gtgcctctga acaggacagt 24840

ggccgcacca gacaacctgc ccaactacga gaacaccgtg gtcttctctc tgtccagctt 24900

ccagtacctc atcctggctg cagccgtgtc caagggggcg cccttccgcc ggccgctcta 24960

caccaatggt gccactgcgg gcgcgggcgg gagatgctgg cggagggagg gaggagccct 25020

ggggtgctgg ggaggcagat gggagagagg tgccggccga gtggaagact ggcgtgcaca 25080

cgtgtgtctg tgggtgccgg gggggcgtgg ctcctgcctg agagactctc ctgctcccag 25140

tgcccttcct ggtggccctg gcgctcctga gctccgtcct ggtgggcctt gtcctggtcc 25200

ccggcctcct gcaggggccg ctggcgctga ggaacatcac tgacaccggc ttcaagctgc 25260

tgctgctggg tctggtcacc ctcaacttcg tgggggcctt catgctggag gtggggcccg 25320

ccctgggtct cagcagcggg agagaaggga ggtgggggtg cattgacccc agctcagccc 25380

cggctgcccc tgacaccctc cctgcccttt gcagagcgtg ctagaccagt gcctccccgc 25440

ctgcctgcgc cgcctccggc ccaagcgggc ctccaagaag cgcttcaagc agctggaacg 25500

agagctggcc gagcagccct ggccgccgct gcccgccggc cccctgaggt agtgcaggcc 25560

cacgggcacc ccagacactg gaactccctg cctctgagcc accaactgga cccctctcca 25620

gcaacaccac cgccaccacc tcccacatcc ctgaggttgg cgactgtcta cactcctccc 25680

ccgagaccac ccccaccctg gggaagcgtt gactactgtc ccctaccttg gaccatcccg 25740

cgtaggggtg gcagccccca gctcccctca gtgctgctgt cagtgtagca aataaagtca 25800

tgatattttc ctggc 25815

<210> 4
<211> 3432
<212> DNA
<213> Homo sapiens

<400> 4

atgagcgcag acagcagccc tctcgtgggc agcacgccca ccggttatgg gaccctgacg 60

atagggacat caatagatcc cctcagctcc tcagtttcat ccgtgaggct cagcggctac 120

tgtggcagtc catggagggt catcggctat cacgtcgtgg tctggatgat ggctgggatc 180

cctttgctgc tcttccgttg gaagcccctg tggggggtgc ggctgcggct ccggccctgc 240

aacctggccc acgccgaaac actcgttatc gaaataagag acaaagagga tagttcctgg 300

cagctcttca ctgtccaggt gcagactgag gccatcggcg agggcagcct ggagccgtcc 360

ccacagtccc aggcagagga tggccggagc caggcggcag ttggggcggt accagagggt 420

gcctggaagg atacggccca gctccacaag agcgaggagg cggtgagtgt cggacagaag 480

cgggtgctgc ggtattacct cttccagggc cagcgctata tctggatcga gacccagcaa 540

gccttctacc aggtcagcct cctggaccat ggccgctctt gtgacgacgt ccaccgctcc 600

cgccatggcc tcagcctcca ggaccaaatg gtgaggaagg ccatttacgg ccccaacgtg 660

atcagcatac cggtcaagtc ctacccccag ctgctggtgg acgaggcact gaacccctac 720

tatgggttcc aggccttcag catcgcgctg tggctggctg accactacta ctggtacgcc 780

ctgtgcatct tcctcatttc ctccatctcc atctgcctgt cgctgtacaa gaccagaaag 840

caaagccaga ctctaaggga catggtcaag ttgtccatgc gggtgtgcgt gtgccggcca 900

gggggagagg aagagtgggt ggactccagt gagctagtgc ccggagactg cctggtgctg 960

ccccaggagg gtgggctgat gccctgtgat gccgccctgg tggccggcga gtgcatggtg 1020

aatgagagct ctctgacagg agagagcatt ccagtgctga agacggcact gccggagggg 1080

ctggggccct actgtgcaga gacacaccgg cggcacacac tcttctgcgg gaccctcatc 1140

ttgcaggccc gggcctatgt gggaccgcac gtcctggcag tggtgacccg cacagctctc 1200

ctcggcacca tctacagcat cttcatcctc taccgaaacc gggtgcctct gaatgagatt 1260

gtaatccggg ctctcgacct ggtgaccgtg gtggtgccac ctgccctgcc tgctgccatg 1320

actgtgtgca cgctctacgc ccagagccga ctgcggagac agggcatttt ctgcatccac 1380

ccactgcgca tcaacctggg gggcaagctg cagctggtgt gtttcgacaa gacgggcacc 1440

ctcactgagg acggcttaga cgtgatgggg gtggtgcccc tgaaggggca ggcattcctg 1500

cccctggtcc cagagcctcg ccgcctgcct gtggggcccc tgctccgagc actggccacc 1560

tgccatgccc tcagccggct ccaggacacc cccgtgggcg accccatgga cttgaagatg 1620

gtggagtcta ctggctgggt cctggaggaa gagccggctg cagactcagc atttgggacc 1680

caggtcttgg cagtgatgag acccccactt tgggagcccc agctgcaggc aatggaggag 1740

cccccggtgc cagtcagcgt cctccaccgc ttccccttct cttcggctct gcagcgcatg 1800

agtgtggtgg tggcgtggcc aggggccact cagcccgagg cctacgtcaa aggctccccg 1860

gagctggtgg cagggctctg caaccccgag acagtgccca ccgacttcgc ccagatgctg 1920

cagagctata cagctgctgg ctaccgtgtc gtggccctgg ccagcaagcc actgcccact 1980

gtgcccagcc tggaggcagc ccagcaactg acgagggaca ctgtggaagg agacctgagc 2040

ctcctggggc tgctggtcat gaggaaccta ctgaagccgc agacaacgcc agttatccag 2100

gctctgcgaa ggacccgcat ccgcgccgtc atggtgacag gggacaacct gcagacagcg 2160

gtgactgtgg cccggggctg tggcatggtg gccccccagg agcatctgat catcgtccac 2220

gccacccacc ctgagcgggg tcagcctgcc tctctcgagt tcctgccgat ggagtccccc 2280

acagccgtga atggcgttaa ggatcctgac caggctgcaa gctacaccgt ggagccagac 2340

ccccgatcca ggcacctggc cctcagcggg cccacctttg gtatcattgt gaagcacttc 2400

cccaagctgc tgcccaaggt cctggtccag ggcactgtct ttgcccgcat ggcccctgag 2460

cagaagacag agctggtgtg cgagctacag aagcttcagt actgcgtggg catgtgcgga 2520

gacggcgcca atgactgtgg ggccctgaag gcggctgatg tcggcatctc gctgtcccag 2580

gcagaagcct cagtggtctc acccttcacc tcgagcatgg ccagtattga gtgcgtgccc 2640

atggtcatca gggaggggcg ctgttccctt gacacttcgt tcagcgtctt caagtacatg 2700

gctctgtaca gcctgaccca gttcatctcc gtcctgatcc tctacacgat caacaccaac 2760

ctgggtgacc tgcagttcct ggccatcgac ctggtcatca ccaccacagt ggcagtgctc 2820

atgagccgca cggggccagc gctggtcctg ggacgggtgc ggccaccggg ggcgctgctc 2880

agcgtgcccg tgctcagcag cctgctgctg cagatggtcc tggtgaccgg cgtgcagcta 2940

gggggctact tcctgaccct ggcccagcca tggttcgtgc ctctgaacag gacagtggcc 3000

gcaccagaca acctgcccaa ctacgagaac accgtggtct tctctctgtc cagcttccag 3060

tacctcatcc tggctgcagc cgtgtccaag ggggcgccct tccgccggcc gctctacacc 3120

aatgtgccct tcctggtggc cctggcgctc ctgagctccg tcctggtggg ccttgtcctg 3180

gtccccggcc tcctgcaggg gccgctggcg ctgaggaaca tcactgacac cggcttcaag 3240

ctgctgctgc tgggtctggt caccctcaac ttcgtggggg ccttcatgct ggagagcgtg 3300

ctagaccagt gcctccccgc ctgcctgcgc cgcctccggc ccaagcgggc ctccaagaag 3360

cgcttcaagc agctggaacg agagctggcc gagcagccct ggccgccgct gcccgccggc 3420

cccctgaggt ag 3432

<210> 5
<211> 1143
<212> PRT
<213> Homo sapiens

<400> 5

Met Ser Ala Asp Ser Ser Pro Leu Val Gly Ser Thr Pro Thr Gly Tyr
1               5                   10                  15

Gly Thr Leu Thr Ile Gly Thr Ser Ile Asp Pro Leu Ser Ser Ser Val
            20                  25                  30

Ser Ser Val Arg Leu Ser Gly Tyr Cys Gly Ser Pro Trp Arg Val Ile
        35                  40                  45

Gly Tyr His Val Val Val Trp Met Met Ala Gly Ile Pro Leu Leu Leu
    50                  55                  60

Phe Arg Trp Lys Pro Leu Trp Gly Val Arg Leu Arg Leu Arg Pro Cys
65                  70                  75                  80

Asn Leu Ala His Ala Glu Thr Leu Val Ile Glu Ile Arg Asp Lys Glu
                85                  90                  95

Asp Ser Ser Trp Gln Leu Phe Thr Val Gln Val Gln Thr Glu Ala Ile
            100                 105                 110

Gly Glu Gly Ser Leu Glu Pro Ser Pro Gln Ser Gln Ala Glu Asp Gly
        115                 120                 125

Arg Ser Gln Ala Ala Val Gly Ala Val Pro Glu Gly Ala Trp Lys Asp
    130                 135                 140

Thr Ala Gln Leu His Lys Ser Glu Glu Ala Val Ser Val Gly Gln Lys
145                 150                 155                 160

Arg Val Leu Arg Tyr Tyr Leu Phe Gln Gly Gln Arg Tyr Ile Trp Ile
                165                 170                 175

Glu Thr Gln Gln Ala Phe Tyr Gln Val Ser Leu Leu Asp His Gly Arg
            180                 185                 190

Ser Cys Asp Asp Val His Arg Ser Arg His Gly Leu Ser Leu Gln Asp
        195                 200                 205

Gln Met Val Arg Lys Ala Ile Tyr Gly Pro Asn Val Ile Ser Ile Pro
    210                 215                 220

Val Lys Ser Tyr Pro Gln Leu Leu Val Asp Glu Ala Leu Asn Pro Tyr
225                 230                 235                 240

Tyr Gly Phe Gln Ala Phe Ser Ile Ala Leu Trp Leu Ala Asp His Tyr
                245                 250                 255

Tyr Trp Tyr Ala Leu Cys Ile Phe Leu Ile Ser Ser Ile Ser Ile Cys

            260                 265                 270

Leu Ser Leu Tyr Lys Thr Arg Lys Gln Ser Gln Thr Leu Arg Asp Met
        275                 280                 285

Val Lys Leu Ser Met Arg Val Cys Val Cys Arg Pro Gly Gly Glu Glu
    290                 295                 300

Glu Trp Val Asp Ser Ser Glu Leu Val Pro Gly Asp Cys Leu Val Leu
305                 310                 315                 320

Pro Gln Glu Gly Gly Leu Met Pro Cys Asp Ala Ala Leu Val Ala Gly
                325                 330                 335

Glu Cys Met Val Asn Glu Ser Ser Leu Thr Gly Glu Ser Ile Pro Val
            340                 345                 350

Leu Lys Thr Ala Leu Pro Glu Gly Leu Gly Pro Tyr Cys Ala Glu Thr
        355                 360                 365

His Arg Arg His Thr Leu Phe Cys Gly Thr Leu Ile Leu Gln Ala Arg
    370                 375                 380

Ala Tyr Val Gly Pro His Val Leu Ala Val Val Thr Arg Thr Ala Leu
385                 390                 395                 400

Leu Gly Thr Ile Tyr Ser Ile Phe Ile Leu Tyr Arg Asn Arg Val Pro
                405                 410                 415

Leu Asn Glu Ile Val Ile Arg Ala Leu Asp Leu Val Thr Val Val Val
            420                 425                 430

Pro Pro Ala Leu Pro Ala Ala Met Thr Val Cys Thr Leu Tyr Ala Gln
        435                 440                 445

Ser Arg Leu Arg Arg Gln Gly Ile Phe Cys Ile His Pro Leu Arg Ile
    450                 455                 460

Asn Leu Gly Gly Lys Leu Gln Leu Val Cys Phe Asp Lys Thr Gly Thr
465                 470                 475                 480

Leu Thr Glu Asp Gly Leu Asp Val Met Gly Val Val Pro Leu Lys Gly
                485                 490                 495

Gln Ala Phe Leu Pro Leu Val Pro Glu Pro Arg Arg Leu Pro Val Gly
            500                 505                 510

Pro Leu Leu Arg Ala Leu Ala Thr Cys His Ala Leu Ser Arg Leu Gln
        515                 520                 525

Asp Thr Pro Val Gly Asp Pro Met Asp Leu Lys Met Val Glu Ser Thr
    530                 535                 540

Gly Trp Val Leu Glu Glu Glu Pro Ala Ala Asp Ser Ala Phe Gly Thr
545                 550                 555                 560

Gln Val Leu Ala Val Met Arg Pro Pro Leu Trp Glu Pro Gln Leu Gln
                565                 570                 575

Ala Met Glu Glu Pro Pro Val Pro Val Ser Val Leu His Arg Phe Pro
            580                 585                 590

Phe Ser Ser Ala Leu Gln Arg Met Ser Val Val Val Ala Trp Pro Gly
        595                 600                 605

Ala Thr Gln Pro Glu Ala Tyr Val Lys Gly Ser Pro Glu Leu Val Ala
    610                 615                 620

Gly Leu Cys Asn Pro Glu Thr Val Pro Thr Asp Phe Ala Gln Met Leu
625                 630                 635                 640

Gln Ser Tyr Thr Ala Ala Gly Tyr Arg Val Val Ala Leu Ala Ser Lys
                645                 650                 655

Pro Leu Pro Thr Val Pro Ser Leu Glu Ala Ala Gln Gln Leu Thr Arg
            660                 665                 670

Asp Thr Val Glu Gly Asp Leu Ser Leu Leu Gly Leu Leu Val Met Arg
        675                 680                 685

Asn Leu Leu Lys Pro Gln Thr Thr Pro Val Ile Gln Ala Leu Arg Arg
    690                 695                 700

Thr Arg Ile Arg Ala Val Met Val Thr Gly Asp Asn Leu Gln Thr Ala
705                 710                 715                 720

Val Thr Val Ala Arg Gly Cys Gly Met Val Ala Pro Gln Glu His Leu
                725                 730                 735

Ile Ile Val His Ala Thr His Pro Glu Arg Gly Gln Pro Ala Ser Leu
            740                 745                 750

Glu Phe Leu Pro Met Glu Ser Pro Thr Ala Val Asn Gly Val Lys Asp
        755                 760                 765

Pro Asp Gln Ala Ala Ser Tyr Thr Val Glu Pro Asp Pro Arg Ser Arg
    770                 775                 780

His Leu Ala Leu Ser Gly Pro Thr Phe Gly Ile Ile Val Lys His Phe
785                 790                 795                 800

Pro Lys Leu Leu Pro Lys Val Leu Val Gln Gly Thr Val Phe Ala Arg
                805                 810                 815

Met Ala Pro Glu Gln Lys Thr Glu Leu Val Cys Glu Leu Gln Lys Leu
            820                 825                 830

Gln Tyr Cys Val Gly Met Cys Gly Asp Gly Ala Asn Asp Cys Gly Ala
        835                 840                 845

Leu Lys Ala Ala Asp Val Gly Ile Ser Leu Ser Gln Ala Glu Ala Ser
    850                 855                 860

Val Val Ser Pro Phe Thr Ser Ser Met Ala Ser Ile Glu Cys Val Pro
865                 870                 875                 880

Met Val Ile Arg Glu Gly Arg Cys Ser Leu Asp Thr Ser Phe Ser Val
                885                 890                 895

Phe Lys Tyr Met Ala Leu Tyr Ser Leu Thr Gln Phe Ile Ser Val Leu
            900                 905                 910

Ile Leu Tyr Thr Ile Asn Thr Asn Leu Gly Asp Leu Gln Phe Leu Ala
        915                 920                 925

Ile Asp Leu Val Ile Thr Thr Thr Val Ala Val Leu Met Ser Arg Thr
    930                 935                 940

Gly Pro Ala Leu Val Leu Gly Arg Val Arg Pro Pro Gly Ala Leu Leu
945                 950                 955                 960

Ser Val Pro Val Leu Ser Ser Leu Leu Leu Gln Met Val Leu Val Thr
                965                 970                 975

Gly Val Gln Leu Gly Gly Tyr Phe Leu Thr Leu Ala Gln Pro Trp Phe
            980                 985                 990

Val Pro Leu Asn Arg Thr Val Ala  Ala Pro Asp Asn Leu  Pro Asn Tyr
        995                 1000                1005

Glu Asn  Thr Val Val Phe Ser  Leu Ser Ser Phe Gln  Tyr Leu Ile
    1010                1015                1020

Leu Ala Ala Ala Val Ser Lys Gly Ala Pro Phe Arg Arg Pro Leu
    1025                1030                1035

Tyr Thr Asn Val Pro Phe Leu Val Ala Leu Ala Leu Leu Ser Ser
    1040                1045                1050

Val Leu Val Gly Leu Val Leu Val Pro Gly Leu Leu Gln Gly Pro
    1055                1060                1065

Leu Ala Leu Arg Asn Ile Thr Asp Thr Gly Phe Lys Leu Leu Leu
    1070                1075                1080

Leu Gly Leu Val Thr Leu Asn Phe Val Gly Ala Phe Met Leu Glu
    1085                1090                1095

Ser Val Leu Asp Gln Cys Leu Pro Ala Cys Leu Arg Arg Leu Arg
    1100                1105                1110

Pro Lys Arg Ala Ser Lys Lys Arg Phe Lys Gln Leu Glu Arg Glu
    1115                1120                1125

Leu Ala Glu Gln Pro Trp Pro Pro Leu Pro Ala Gly Pro Leu Arg
    1130                1135                1140

<210> 6
<211> 3542
<212> DNA
<213> Homo sapiens

<400> 6
atgagcgcag acagcagccc tctcgtgggc agcacgccca ccggttatgg gaccctgacg      60

atagggacat caatagatcc cctcagctcc tcagtttcat ccgtgaggct cagcggctac     120

tgtggcagtc catggagggt catcggctat cacgtcgtgg tctggatgat ggctgggatc     180

cctttgctgc tcttccgttg gaagcccctg tggggggtgc ggctgcggct ccggccctgc     240

aacctggccc acgccgaaac actcgttatc gaaataagag acaaagagga tagttcctgg     300

cagctcttca ctgtccaggt gcagactgag gccatcggcg agggcagcct ggagccgtcc     360

ccacagtccc aggcagagga tggccggagc caggcggcag ttggggcggt accagagggt     420

gcctggaagg atacggccca gctccacaag agcgaggagg cggtgagtgt cggacagaag     480

cgggtgctgc ggtattacct cttccagggc cagcgctata tctggatcga gacccagcaa     540

gccttctacc aggtcagcct cctggaccat ggccgctctt gtgacgacgt ccaccgctcc     600

cgccatggcc tcagcctcca ggaccaaatg gtgaggaagg ccatttacgg ccccaacgtg     660

atcagcatac cggtcaagtc ctacccccag ctgctggtgg acgaggcact gaacccctac     720

tatgggttcc aggccttcag catcgcgctg tggctggctg accactacta ctggtacgcc 780

ctgtgcatct tcctcatttc ctccatctcc atctgcctgt cgctgtacaa gaccagaaag 840

caaagccaga ctctaaggga catggtcaag ttgtccatgc gggtgtgcgt gtgccggcca 900

gggggagagg aagagtgggt ggactccagt gagctagtgc ccggagactg cctggtgctg 960

ccccaggagg gtgggctgat gccctgtgat gccgccctgg tggccggcga gtgcatggtg 1020

aatgagagct ctctgacagg agagagcatt ccagtgctga agacggcact gccggagggg 1080

ctggggccct actgtgcaga gacacaccgg cggcacacac tcttctgcgg gaccctcatc 1140

ttgcaggccc gggcctatgt gggaccgcac gtcctggcag tggtgacccg cacagggttc 1200

tgcacggcaa aggggggcct ggtgagctcc atcttgcacc cccggcccat caacttcaag 1260

ttctataaac acagcatgaa gtttgtggct gccctctctg tcctggctct cctcggcacc 1320

atctacagca tcttcatcct ctaccgaaac cgggtgcctc tgaatgagat tgtaatccgg 1380

gctctcgacc tggtgaccgt ggtggtgcca cctgccctgc ctgctgccat gactgtgtgc 1440

acgctctacg cccagagccg actgcggaga cagggcattt tctgcatcca cccactgcgc 1500

atcaacctgg ggggcaagct gcagctggtg tgtttcgaca agacgggcac cctcactgag 1560

gacggcttag acgtgatggg ggtggtgccc ctgaaggggc aggcattcct gcccctggtc 1620

ccagagcctc gccgcctgcc tgtggggccc ctgctccgag cactggccac ctgccatgcc 1680

ctcagccggc tccaggacac ccccgtgggc gaccccatgg acttgaagat ggtggagtct 1740

actggctggg tcctggagga agagccggct gcagactcag catttgggac ccaggtcttg 1800

gcagtgatga gacccccact ttgggagccc cagctgcagg caatggagga gcccccggtg 1860

ccagtcagcg tcctccaccg cttccccttc tcttcggctc tgcagcgcat gagtgtggtg 1920

gtggcgtggc caggggccac tcagcccgag gcctacgtca aaggctcccc ggagctggtg 1980

gcagggctct gcaaccccga gacagtgccc accgacttcg cccagatgct gcagagctat 2040

acagctgctg gctaccgtgt cgtggccctg gccagcaagc cactgcccac tgtgcccagc 2100

ctggaggcag cccagcaact gacgagggac actgtggaag gagacctgag cctcctgggg 2160

ctgctggtca tgaggaacct actgaagccg cagacaacgc cagttatcca ggctctgcga 2220

aggacccgca tccgcgccgt catggtgaca ggggacaacc tgcagacagc ggtgactgtg 2280

gcccggggct gtggcatggt ggccccccag gagcatctga tcatcgtcca cgccacccac 2340

cctgagcggg gtcagcctgc ctctctcgag ttcctgccga tggagtcccc cacagccgtg 2400

aatggcgtta aggatcctga ccaggctgca agctacaccg tggagccaga cccccgatcc 2460

aggcacctgg ccctcagcgg gcccaccttt ggtatcattg tgaagcactt ccccaagctg 2520

ctgcccaagg tcctggtcca gggcactgtc tttgcccgca tggcccctga gcagaagaca 2580

gagctggtgt gcgagctaca gaagcttcag tactgcgtgg gcatgtgcgg agacggcgcc    2640

aatgactgtg gggccctgaa ggcggctgat gtcggcatct cgctgtccca ggcagaagcc    2700

tcagtggtct cacccttcac ctcgagcatg gccagtattg agtgcgtgcc catggtcatc    2760

agggaggggc gctgttccct tgacacttcg ttcagcgtct tcaagtacat ggctctgtac    2820

agcctgaccc agttcatctc cgtcctgatc ctctacacga tcaacaccaa cctgggtgac    2880

ctgcagttcc tggccatcga cctggtcatc accaccacag tggcagtgct catgagccgc    2940

acggggccag cgctggtcct gggacgggtg cggccaccgg gggcgctgct cagcgtgccc    3000

gtgctcagca gcctgctgct gcagatggtc ctggtgaccg gcgtgcagct agggggtact    3060

tcctgaccct ggcccagcca tggttcgtgc ctctgaacag gacagtggcc gcaccagaca    3120

acctgcccaa ctacgagaac accgtggtct tctctctgtc cagcttccag tacctcatcc    3180

tggctgcagc cgtgtccaag ggggcgccct tccgccggcc gctctacacc aatgtgccct    3240

tcctggtggc cctggcgctc ctgagctccg tcctggtggg ccttgtcctg gtccccggcc    3300

tcctgcaggg gccgctggcg ctgaggaaca tcactgacac cggcttcaag ctgctgctgc    3360

tgggtctggt caccctcaac ttcgtggggg ccttcatgct ggagagcgtg ctagaccagt    3420

gcctccccgc ctgcctgcgc cgcctccggc ccaagcgggc ctccaagaag cgcttcaagc    3480

agctggaacg agagctggcc gagcagccct ggccgccgct gcccgccggc cccctgaggt    3540

ag                                                                  3542

<210>  7
<211>  1021
<212>  PRT
<213>  Homo sapiens

<400>  7

Met Ser Ala Asp Ser Ser Pro Leu Val Gly Ser Thr Pro Thr Gly Tyr
1               5                   10                  15

Gly Thr Leu Thr Ile Gly Thr Ser Ile Asp Pro Leu Ser Ser Ser Val
            20                  25                  30

Ser Ser Val Arg Leu Ser Gly Tyr Cys Gly Ser Pro Trp Arg Val Ile
        35                  40                  45

Gly Tyr His Val Val Val Trp Met Met Ala Gly Ile Pro Leu Leu Leu
    50                  55                  60

Phe Arg Trp Lys Pro Leu Trp Gly Val Arg Leu Arg Leu Arg Pro Cys
65                  70                  75                  80

Asn Leu Ala His Ala Glu Thr Leu Val Ile Glu Ile Arg Asp Lys Glu

                85                  90                  95

Asp Ser Ser Trp Gln Leu Phe Thr Val Gln Val Gln Thr Glu Ala Ile
            100                 105                 110

Gly Glu Gly Ser Leu Glu Pro Ser Pro Gln Ser Gln Ala Glu Asp Gly
        115                 120                 125

Arg Ser Gln Ala Ala Val Gly Ala Val Pro Glu Gly Ala Trp Lys Asp
    130                 135                 140

Thr Ala Gln Leu His Lys Ser Glu Glu Ala Val Ser Val Gly Gln Lys
145                 150                 155                 160

Arg Val Leu Arg Tyr Tyr Leu Phe Gln Gly Gln Arg Tyr Ile Trp Ile
                165                 170                 175

Glu Thr Gln Gln Ala Phe Tyr Gln Val Ser Leu Leu Asp His Gly Arg
            180                 185                 190

Ser Cys Asp Asp Val His Arg Ser Arg His Gly Leu Ser Leu Gln Asp
        195                 200                 205

Gln Met Val Arg Lys Ala Ile Tyr Gly Pro Asn Val Ile Ser Ile Pro
    210                 215                 220

Val Lys Ser Tyr Pro Gln Leu Leu Val Asp Glu Ala Leu Asn Pro Tyr
225                 230                 235                 240

Tyr Gly Phe Gln Ala Phe Ser Ile Ala Leu Trp Leu Ala Asp His Tyr
                245                 250                 255

Tyr Trp Tyr Ala Leu Cys Ile Phe Leu Ile Ser Ser Ile Ser Ile Cys
            260                 265                 270

Leu Ser Leu Tyr Lys Thr Arg Lys Gln Ser Gln Thr Leu Arg Asp Met
        275                 280                 285

Val Lys Leu Ser Met Arg Val Cys Val Cys Arg Pro Gly Gly Glu Glu
    290                 295                 300

Glu Trp Val Asp Ser Ser Glu Leu Val Pro Gly Asp Cys Leu Val Leu
305                 310                 315                 320

Pro Gln Glu Gly Gly Leu Met Pro Cys Asp Ala Ala Leu Val Ala Gly
                325                 330                 335

Glu Cys Met Val Asn Glu Ser Ser Leu Thr Gly Glu Ser Ile Pro Val
            340                 345                 350

Leu Lys Thr Ala Leu Pro Glu Gly Leu Gly Pro Tyr Cys Ala Glu Thr
        355                 360                 365

His Arg Arg His Thr Leu Phe Cys Gly Thr Leu Ile Leu Gln Ala Arg
    370                 375                 380

Ala Tyr Val Gly Pro His Val Leu Ala Val Val Thr Arg Thr Gly Phe
385                 390                 395                 400

Cys Thr Ala Lys Gly Gly Leu Val Ser Ser Ile Leu His Pro Arg Pro
                405                 410                 415

Ile Asn Phe Lys Phe Tyr Lys His Ser Met Lys Phe Val Ala Ala Leu
            420                 425                 430

Ser Val Leu Ala Leu Leu Gly Thr Ile Tyr Ser Ile Phe Ile Leu Tyr
        435                 440                 445

Arg Asn Arg Val Pro Leu Asn Glu Ile Val Ile Arg Ala Leu Asp Leu
    450                 455                 460

Val Thr Val Val Val Pro Pro Ala Leu Pro Ala Ala Met Thr Val Cys
465                 470                 475                 480

Thr Leu Tyr Ala Gln Ser Arg Leu Arg Arg Gln Gly Ile Phe Cys Ile
                485                 490                 495

His Pro Leu Arg Ile Asn Leu Gly Gly Lys Leu Gln Leu Val Cys Phe
            500                 505                 510

Asp Lys Thr Gly Thr Leu Thr Glu Asp Gly Leu Asp Val Met Gly Val
        515                 520                 525

Val Pro Leu Lys Gly Gln Ala Phe Leu Pro Leu Val Pro Glu Pro Arg
    530                 535                 540

Arg Leu Pro Val Gly Pro Leu Leu Arg Ala Leu Ala Thr Cys His Ala
545                 550                 555                 560

Leu Ser Arg Leu Gln Asp Thr Pro Val Gly Asp Pro Met Asp Leu Lys
                565                 570                 575

Met Val Glu Ser Thr Gly Trp Val Leu Glu Glu Glu Pro Ala Ala Asp
            580                 585                 590

Ser Ala Phe Gly Thr Gln Val Leu Ala Val Met Arg Pro Pro Leu Trp
        595                 600                 605

Glu Pro Gln Leu Gln Ala Met Glu Glu Pro Pro Val Pro Val Ser Val
    610                 615                 620

Leu His Arg Phe Pro Phe Ser Ser Ala Leu Gln Arg Met Ser Val Val
625                 630                 635                 640

Val Ala Trp Pro Gly Ala Thr Gln Pro Glu Ala Tyr Val Lys Gly Ser
                645                 650                 655

Pro Glu Leu Val Ala Gly Leu Cys Asn Pro Glu Thr Val Pro Thr Asp
            660                 665                 670

Phe Ala Gln Met Leu Gln Ser Tyr Thr Ala Ala Gly Tyr Arg Val Val
        675                 680                 685

Ala Leu Ala Ser Lys Pro Leu Pro Thr Val Pro Ser Leu Glu Ala Ala
    690                 695                 700

Gln Gln Leu Thr Arg Asp Thr Val Glu Gly Asp Leu Ser Leu Leu Gly
705                 710                 715                 720

Leu Leu Val Met Arg Asn Leu Leu Lys Pro Gln Thr Thr Pro Val Ile
                725                 730                 735

Gln Ala Leu Arg Arg Thr Arg Ile Arg Ala Val Met Val Thr Gly Asp
            740                 745                 750

Asn Leu Gln Thr Ala Val Thr Val Ala Arg Gly Cys Gly Met Val Ala
        755                 760                 765

Pro Gln Glu His Leu Ile Ile Val His Ala Thr His Pro Glu Arg Gly
    770                 775                 780

Gln Pro Ala Ser Leu Glu Phe Leu Pro Met Glu Ser Pro Thr Ala Val
785                 790                 795                 800

Asn Gly Val Lys Asp Pro Asp Gln Ala Ala Ser Tyr Thr Val Glu Pro
                805                 810                 815

Asp Pro Arg Ser Arg His Leu Ala Leu Ser Gly Pro Thr Phe Gly Ile
            820                 825                 830

Ile Val Lys His Phe Pro Lys Leu Leu Pro Lys Val Leu Val Gln Gly
        835                 840                 845

Thr Val Phe Ala Arg Met Ala Pro Glu Gln Lys Thr Glu Leu Val Cys
    850                 855                 860

Glu Leu Gln Lys Leu Gln Tyr Cys Val Gly Met Cys Gly Asp Gly Ala
865                 870                 875                 880

Asn Asp Cys Gly Ala Leu Lys Ala Ala Asp Val Gly Ile Ser Leu Ser
                885                 890                 895

Gln Ala Glu Ala Ser Val Val Ser Pro Phe Thr Ser Ser Met Ala Ser
            900                 905                 910

Ile Glu Cys Val Pro Met Val Ile Arg Glu Gly Arg Cys Ser Leu Asp
        915                 920                 925

Thr Ser Phe Ser Val Phe Lys Tyr Met Ala Leu Tyr Ser Leu Thr Gln
    930                 935                 940

Phe Ile Ser Val Leu Ile Leu Tyr Thr Ile Asn Thr Asn Leu Gly Asp
945                 950                 955                 960

Leu Gln Phe Leu Ala Ile Asp Leu Val Ile Thr Thr Thr Val Ala Val
                965                 970                 975

Leu Met Ser Arg Thr Gly Pro Ala Leu Val Leu Gly Arg Val Arg Pro
            980                 985                 990

Pro Gly Ala Leu Leu Ser Val Pro  Val Leu Ser Ser Leu  Leu Leu Gln
        995                 1000                1005

Met Val  Leu Val Thr Gly Val  Gln Leu Gly Gly Thr  Ser
    1010                1015                1020

<210>  8
<211>  3565
<212>  DNA
<213>  Homo sapiens

<400>  8
   atgagcgcag acagcagccc tctcgtgggc agcacgccca ccggttatgg gaccctgacg      60
   atagggacat caatagatcc cctcagctcc tcagtttcat ccgtgaggct cagcggctac     120
   tgtggcagtc catggagggt catcggctat cacgtcgtgg tctggatgat ggctgggatc     180
   cctttgctgc tcttccgttg gaagcccctg tggggggtgc ggctgcggct ccggccctgc     240
   aacctggccc acgccgaaac actcgttatc gaaataagag acaaagagga tagttcctgg     300
   cagctcttca ctgtccaggt gcagactgag gccatcggcg agggcagcct ggagccgtcc     360
   ccacagtccc aggcagagga tggccggagc caggcggcag ttggggcggt accagagggt 420
   gcctggaagg atacggccca gctccacaag agcgaggagg cggtgagtgt cggacagaag 480
   cgggtgctgc ggtattacct cttccagggc cagcgctata tctggatcga gacccagcaa 540
   gccttctacc aggtcagcct cctggaccat ggccgctctt gtgacgacgt ccaccgctcc 600
   cgccatggcc tcagcctcca ggaccaaatg gtgaggaagg ccatttacgg ccccaacgtg 660
   atcagcatac cggtcaagtc ctacccccag ctgctggtgg acgaggcact gaacccctac 720
   tatgggttcc aggccttcag catcgcgctg tggctggctg accactacta ctggtacgcc 780
   ctgtgcatct tcctcatttc ctccatctcc atctgcctgt cgctgtacaa gaccagaaag 840
   caaagccaga ctctaaggga catggtcaag ttgtccatgc gggtgtgcgt gtgccggcca 900
   gggggagagg aagagtgggt ggactccagt gagctagtgc ccggagactg cctggtgctg 960
   ccccaggagg gtgggctgat gccctgtgat gccgccctgg tggccggcga gtgcatggtg 1020
   aatgagagct ctctgacagg agagagcatt ccagtgctga agacggcact gccggagggg 1080
   ctggggccct actgtgcaga gacacaccgg cggcacacac tcttctgcgg gaccctcatc 1140
   ttgcaggccc gggcctatgt gggaccgcac gtcctggcag tggtgacccg cacagggttc 1200
   tgcacggcaa aggggggcct ggtgagctcc atcttgcacc cccggcccat caacttcaag 1260
   ttctataaac acagcatgaa gtttgtggct gccctctctg tcctggctct cctcggcacc 1320
   atctacagca tcttcatcct ctaccgaaac cgggtgcctc tgaatgagat tgtaatccgg 1380
   gctctcgacc tggtgaccgt ggtggtgcca cctgccctgc ctgctgccat gactgtgtgc 1440
   acgctctacg cccagagccg actgcggaga cagggcattt tctgcatcca cccactgcgc 1500
   atcaacctgg ggggcaagct gcagctggtg tgtttcgaca agacgggcac cctcactgag 1560
   gacggcttag acgtgatggg ggtggtgccc ctgaaggggc aggcattcct gcccctggtc 1620
   ccagagcctc gccgcctgcc tgtggggccc ctgccgcctg cctgtggggc ccctgctccg 1680
   agcactggcc acctgccatg ccctcagccg gctccaggac acccccgtgg gcgaccccat 1740
   ggacttgaag atggtggagt ctactggctg ggtcctggag gaagagccgg ctgcagactc 1800
   agcatttggg acccaggtct tggcagtgat gagaccccca ctttgggagc cccagctgca 1860
   ggcaatggag gagcccccgg tgccagtcag cgtcctccac cgcttcccct tctcttcggc 1920
   tctgcagcgc atgagtgtgg tggtggcgtg gccaggggcc actcagcccg aggcctacgt 1980
   caaaggctcc ccggagctgg tggcagggct ctgcaacccc gagacagtgc ccaccgactt 2040
   cgcccagatg ctgcagagct atacagctgc tggctaccgt gtcgtggccc tggccagcaa 2100
   gccactgccc actgtgccca gcctggaggc agcccagcaa ctgacgaggg acactgtgga 2160
   aggagacctg agcctcctgg ggctgctggt catgaggaac ctactgaagc cgcagacaac 2220
   gccagttatc caggctctgc gaaggacccg catccgcgcc gtcatggtga caggggacaa 2280
   cctgcagaca gcggtgactg tggcccgggg ctgtggcatg gtggcccccc aggagcatct 2340
   gatcatcgtc cacgccaccc accctgagcg gggtcagcct gcctctctcg agttcctgcc 2400
   gatggagtcc cccacagccg tgaatggcgt taaggatcct gaccaggctg caagctacac 2460
   cgtggagcca gacccccgat ccaggcacct ggccctcagc gggcccacct ttggtatcat 2520
   tgtgaagcac ttccccaagc tgctgcccaa ggtcctggtc cagggcactg tctttgcccg 2580
   catggcccct gagcagaaga cagagctggt gtgcgagcta cagaagcttc agtactgcgt 2640
   gggcatgtgc ggagacggcg ccaatgactg tggggccctg aaggcggctg atgtcggcat 2700
   ctcgctgtcc caggcagaag cctcagtggt ctcacccttc acctcgagca tggccagtat 2760
   tgagtgcgtg cccatggtca tcagggaggg gcgctgttcc cttgacactt cgttcagcgt 2820
   cttcaagtac atggctctgt acagcctgac ccagttcatc tccgtcctga tcctctacac 2880
   gatcaacacc aacctgggtg acctgcagtt cctggccatc gacctggtca tcaccaccac 2940
   agtggcagtg ctcatgagcc gcacggggcc agcgctggtc ctgggacggg tgcggccacc 3000
   gggggcgctg ctcagcgtgc ccgtgctcag cagcctgctg ctgcagatgg tcctggtgac 3060
   cggcgtgcag ctagggggct acttcctgac cctggcccag ccatggttcg tgcctctgaa 3120
   caggacagtg gccgcaccag acaacctgcc caactacgag aacaccgtgg tcttctctct 3180
   gtccagcttc cagtacctca tcctggctgc agccgtgtcc aagggggcgc ccttccgccg 3240
   gccgctctac accaatgtgc ccttcctggt ggccctggcg ctcctgagct ccgtcctggt 3300
   gggccttgtc ctggtccccg gcctcctgca ggggccgctg gcgctgagga acatcactga 3360
   caccggcttc aagctgctgc tgctgggtct ggtcaccctc aacttcgtgg gggccttcat 3420
   gctggagagc gtgctagacc agtgcctccc cgcctgcctg cgccgcctcc ggcccaagcg 3480
   ggcctccaag aagcgcttca agcagctgga acgagagctg gccgagcagc cctggccgcc 3540
   gctgcccgcc ggccccctga ggtag 3565

<210> 9
<211> 788
<212> PRT
<213> Homo sapiens

<400> 9

Met Ser Ala Asp Ser Ser Pro Leu Val Gly Ser Thr Pro Thr Gly Tyr
1               5                   10                  15

Gly Thr Leu Thr Ile Gly Thr Ser Ile Asp Pro Leu Ser Ser Ser Val
            20                  25                  30

Ser Ser Val Arg Leu Ser Gly Tyr Cys Gly Ser Pro Trp Arg Val Ile

35                  40                  45

Gly Tyr His Val Val Val Trp Met Met Ala Gly Ile Pro Leu Leu Leu
    50                  55                  60

Phe Arg Trp Lys Pro Leu Trp Gly Val Arg Leu Arg Leu Arg Pro Cys
65                  70                  75                  80

Asn Leu Ala His Ala Glu Thr Leu Val Ile Glu Ile Arg Asp Lys Glu
                85                  90                  95

Asp Ser Ser Trp Gln Leu Phe Thr Val Gln Val Gln Thr Glu Ala Ile
            100                 105                 110

Gly Glu Gly Ser Leu Glu Pro Ser Pro Gln Ser Gln Ala Glu Asp Gly
        115                 120                 125

Arg Ser Gln Ala Ala Val Gly Ala Val Pro Glu Gly Ala Trp Lys Asp
    130                 135                 140

Thr Ala Gln Leu His Lys Ser Glu Glu Ala Val Ser Val Gly Gln Lys
145                 150                 155                 160

Arg Val Leu Arg Tyr Tyr Leu Phe Gln Gly Gln Arg Tyr Ile Trp Ile
                165                 170                 175

Glu Thr Gln Gln Ala Phe Tyr Gln Val Ser Leu Leu Asp His Gly Arg
            180                 185                 190

Ser Cys Asp Asp Val His Arg Ser Arg His Gly Leu Ser Leu Gln Asp
        195                 200                 205

Gln Met Val Arg Lys Ala Ile Tyr Gly Pro Asn Val Ile Ser Ile Pro
    210                 215                 220

Val Lys Ser Tyr Pro Gln Leu Leu Val Asp Glu Ala Leu Asn Pro Tyr
225                 230                 235                 240

Tyr Gly Phe Gln Ala Phe Ser Ile Ala Leu Trp Leu Ala Asp His Tyr
                245                 250                 255

Tyr Trp Tyr Ala Leu Cys Ile Phe Leu Ile Ser Ser Ile Ser Ile Cys
            260                 265                 270

Leu Ser Leu Tyr Lys Thr Arg Lys Gln Ser Gln Thr Leu Arg Asp Met
        275                 280                 285

Val Lys Leu Ser Met Arg Val Cys Val Cys Arg Pro Gly Gly Glu Glu
    290                 295                 300

Glu Trp Val Asp Ser Ser Glu Leu Val Pro Gly Asp Cys Leu Val Leu
305                 310                 315                 320

Pro Gln Glu Gly Gly Leu Met Pro Cys Asp Ala Ala Leu Val Ala Gly
                325                 330                 335

Glu Cys Met Val Asn Glu Ser Ser Leu Thr Gly Glu Ser Ile Pro Val
            340                 345                 350

Leu Lys Thr Ala Leu Pro Glu Gly Leu Gly Pro Tyr Cys Ala Glu Thr
        355                 360                 365

His Arg Arg His Thr Leu Phe Cys Gly Thr Leu Ile Leu Gln Ala Arg
    370                 375                 380

Ala Tyr Val Gly Pro His Val Leu Ala Val Val Thr Arg Thr Gly Phe
385                 390                 395                 400

Cys Thr Ala Lys Gly Gly Leu Val Ser Ser Ile Leu His Pro Arg Pro
                405                 410                 415

Ile Asn Phe Lys Phe Tyr Lys His Ser Met Lys Phe Val Ala Ala Leu
            420                 425                 430

Ser Val Leu Ala Leu Leu Gly Thr Ile Tyr Ser Ile Phe Ile Leu Tyr
        435                 440                 445

Arg Asn Arg Val Pro Leu Asn Glu Ile Val Ile Arg Ala Leu Asp Leu
    450                 455                 460

Val Thr Val Val Val Pro Pro Ala Leu Pro Ala Ala Met Thr Val Cys
465                 470                 475                 480

Thr Leu Tyr Ala Gln Ser Arg Leu Arg Arg Gln Gly Ile Phe Cys Ile
                485                 490                 495

His Pro Leu Arg Ile Asn Leu Gly Gly Lys Leu Gln Leu Val Cys Phe
            500                 505                 510

Asp Lys Thr Gly Thr Leu Thr Glu Asp Gly Leu Asp Val Met Gly Val
        515                 520                 525

Val Pro Leu Lys Gly Gln Ala Phe Leu Pro Leu Val Pro Glu Pro Arg
    530                 535                 540

Arg Leu Pro Val Gly Pro Leu Pro Pro Ala Cys Gly Ala Pro Ala Pro
545                 550                 555                 560

Ser Thr Gly His Leu Pro Cys Pro Gln Pro Ala Pro Gly His Pro Arg
                565                 570                 575

Gly Arg Pro His Gly Leu Glu Asp Gly Gly Val Tyr Trp Leu Gly Pro
            580                 585                 590

Gly Gly Arg Ala Gly Cys Arg Leu Ser Ile Trp Asp Pro Gly Leu Gly
        595                 600                 605

Ser Asp Glu Thr Pro Thr Leu Gly Ala Pro Ala Ala Gly Asn Gly Gly
    610                 615                 620

Ala Pro Gly Ala Ser Gln Arg Pro Pro Pro Leu Pro Leu Leu Phe Gly
625                 630                 635                 640

Ser Ala Ala His Glu Cys Gly Gly Gly Val Ala Arg Gly His Ser Ala
                645                 650                 655

Arg Gly Leu Arg Gln Arg Leu Pro Gly Ala Gly Gly Arg Ala Leu Gln
            660                 665                 670

Pro Arg Asp Ser Ala His Arg Leu Arg Pro Asp Ala Ala Glu Leu Tyr
        675                 680                 685

Ser Cys Trp Leu Pro Cys Arg Gly Pro Gly Gln Gln Ala Thr Ala His
    690                 695                 700

Cys Ala Gln Pro Gly Gly Ser Pro Ala Thr Asp Glu Gly His Cys Gly
705                 710                 715                 720

Arg Arg Pro Glu Pro Pro Gly Ala Ala Gly His Glu Glu Pro Thr Glu
                725                 730                 735

Ala Ala Asp Asn Ala Ser Tyr Pro Gly Ser Ala Lys Asp Pro His Pro
            740                 745                 750

Arg Arg His Gly Asp Arg Gly Gln Pro Ala Asp Ser Gly Asp Cys Gly
        755                 760                 765

Pro Gly Leu Trp His Gly Gly Pro Pro Gly Ala Ser Asp His Arg Pro
    770                 775                 780

Arg His Pro Pro
785

<210>  10
<211>  22
<212>  DNA
<213>  artificial

<220>
<223>  ATP1F primer

<400>  10
ggactgcggc agtgtcggag cc                                                22


<210>  11
<211>  20
<212>  DNA
<213>  artificial

<220>
<223>  primer

<400>  11
gagcctggtg gcccaggtgc                                                   20


<210>  12
<211>  20
<212>  DNA
<213>  artificial

<220>
<223>  primer

<400>  12
cgcttcctgg gctcgcgacc                                                   20


<210>  13
<211>  22
<212>  DNA
<213>  artificial

<220>
<223>  primer

<400>  13
gctgcgagcg gcggcgccat cc                                                22


<210>  14
<211>  22
<212>  DNA
<213>  artificial

<220>
<223>  primer

<400>  14
agcaagagga gaaggtgcct cc                                                22


<210>  15

<211>  22
<212>  DNA
<213>  artificial

<220>
<223>  primer

<400>  15
ggttggcacc caagcatcct cc                                              22


<210>  16
<211>  23
<212>  DNA
<213>  artificial

<220>
<223>  primer

<400>  16
ccttgatttc tgccttctcc agc                                             23


<210>  17
<211>  22
<212>  DNA
<213>  artificial

<220>
<223>  primer

<400>  17
gcacagatca tgaaaccgag gc                                              22


<210>  18
<211>  23
<212>  DNA
<213>  artificial

<220>
<223>  primer

<400>  18
cagcataagc acactgcttc tgg                                             23


<210>  19
<211>  22
<212>  DNA
<213>  artificial

<220>
<223>  primer

<400>  19
catgccatgc caccgtctgt gc                                              22


<210>  20
<211>  22
<212>  DNA
<213>  artificial

<220>
<223>  primer

<400>  20
atagtctcca gcctgcacag gg                                                22


<210>  21
<211>  22
<212>  DNA
<213>  artificial

<220>
<223>  primer

<400>  21
ctggttgcca ccgtaaagtg gc                                                22


<210>  22
<211>  22
<212>  DNA
<213>  artificial

<220>
<223>  primer

<400>  22
cacaggagac ccagcctgtg gc                                                22


<210>  23
<211>  24
<212>  DNA
<213>  artificial

<220>
<223>  primer

<400>  23
cctggacaac agagtgaaac tccg                                              24


<210>  24
<211>  22
<212>  DNA
<213>  artificial

<220>
<223>  primer

<400>  24
cctctctgag cctcagtgtt cc                                                22


<210>  25
<211>  23
<212>  DNA
<213>  artificial

<220>
<223>  primer

<400>  25
ggcccaactt ctgctaagat ggg                                              23


<210>  26
<211>  24
<212>  DNA
<213>  artificial

<220>
<223>  primer

<400>  26
ttgcaactcc agaggcacgg gtcg                                             24


<210>  27
<211>  24
<212>  DNA
<213>  artificial

<220>
<223>  primer

<400>  27
tagacaggac ctggcatcct gtgg                                             24


<210>  28
<211>  23
<212>  DNA
<213>  artificial

<220>
<223>  primer

<400>  28
agagggaatc acatgtgcca agg                                              23


<210>  29
<211>  20
<212>  DNA
<213>  artificial

<220>
<223>  primer

<400>  29
gcccagctca gcagggtacc                                                  20


<210>  30
<211>  22
<212>  DNA
<213>  artificial

<220>
<223>  primer

<400>  30
tgggcataat aagagaccac cc                                               22

<210>  31
<211>  22
<212>  DNA
<213>  artificial

<220>
<223>  primer

<400>  31
cgttacaggt gtgagccact gc                                                22


<210>  32
<211>  24
<212>  DNA
<213>  artificial

<220>
<223>  primer

<400>  32
acgtattcca ggtcacccaa cagc                                              24


<210>  33
<211>  23
<212>  DNA
<213>  artificial

<220>
<223>  primer

<400>  33
accaggcagg gcatcttcct tgg                                               23


<210>  34
<211>  22
<212>  DNA
<213>  artificial

<220>
<223>  primer

<400>  34
ccaaggaaga tgccctgcct gg                                                22


<210>  35
<211>  23
<212>  DNA
<213>  artificial

<220>
<223>  primer

<400>  35
atgggttcct tggaagtcac tcc                                               23


<210>  36

<211>  22
<212>  DNA
<213>  artificial

<220>
<223>  primer

<400>  36
ggcagacgga tttcatccag cc                                                22


<210>  37
<211>  22
<212>  DNA
<213>  artificial

<220>
<223>  primer

<400>  37
ggacaagctc agtctccgca gg                                                22


<210>  38
<211>  23
<212>  DNA
<213>  artificial

<220>
<223>  primer

<400>  38
cccagctgtc atcatattct gcc                                               23


<210>  39
<211>  23
<212>  DNA
<213>  artificial

<220>
<223>  primer

<400>  39
cccacgtcat ctattctggg acc                                               23


<210>  40
<211>  22
<212>  DNA
<213>  artificial

<220>
<223>  primer

<400>  40
gggcctctgc tatcagacta gg                                                22


<210>  41
<211>  22
<212>  DNA
<213>  artificial

<220>
<223>  primer

<400>  41
gtgcacgcca gtcttccact cg                                                22


<210>  42
<211>  22
<212>  DNA
<213>  artificial

<220>
<223>  primer

<400>  42
cgagtggaag actggcgtgc ac                                                22


<210>  43
<211>  22
<212>  DNA
<213>  artificial

<220>
<223>  primer

<400>  43
tggctcagag gcagggagtt cc                                                22


<210>  44
<211>  24
<212>  DNA
<213>  artificial

<220>
<223>  primer

<400>  44
cacggaggga ctgcggcagt gtcg                                              24


<210>  45
<211>  24
<212>  DNA
<213>  artificial

<220>
<223>  primer

<400>  45
tgctgcggta ttacctcttc cagg                                              24


<210>  46
<211>  24
<212>  DNA
<213>  artificial

<220>
<223>  primer

<400>  46
agcggtggac gtcgtcacaa gagc                                              24


<210>  47
<211>  23
<212>  DNA
<213>  artificial

<220>
<223>  primer

<400>  47
cctggtgagc tccatcttgc acc                                               23


<210>  48
<211>  24
<212>  DNA
<213>  artificial

<220>
<223>  cATP5R primer

<400>  48
ggatgaagat gctgtagatg gtgc                                              24


<210>  49
<211>  24
<212>  DNA
<213>  artificial

<220>
<223>  cATP6F primer

<400>  49
atacagctgc tggctaccgt gtcg                                              24


<210>  50
<211>  24
<212>  DNA
<213>  artificial

<220>
<223>  primer

<400>  50
ggcttcagta ggttcctcat gacc                                              24


<210>  51
<211>  24
<212>  DNA
<213>  artificial

<220>
<223>  cATP7R primer

<400>  51
ggcagaagcc tcagtggtct cacc                                              24

<210> 52
<211> 24
<212> DNA
<213> artificial

<220>
<223> cATP9R primer

<400> 52
gaactgggtc aggctgtaca gagc 24


<210> 53
<211> 24
<212> DNA
<213> artificial

<220>
<223> cATP10R primer

<400> 53
tccagttggt ggctcagagg cagg 24


<210> 54
<211> 20
<212> DNA
<213> artificial

<220>
<223> cATP12F primer

<400> 54
gtgctgaaga cggcactgcc 20


<210> 55
<211> 20
<212> DNA
<213> artificial

<220>
<223> cATP15R primer

<400> 55
acacaccagc tgcagcttgc 20


<210> 56
<211> 19
<212> DNA
<213> artificial

<220>
<223> Atp13a2F1 primer

<400> 56
tgacgacagg gacgtcaat 19


<210> 57

<211>  20
<212>  DNA
<213>  artificial

<220>
<223>  Atp13a2R1 primer

<400>  57
ggacgtcatc acaggtacga                                                    20


<210>  58
<211>  19
<212>  DNA
<213>  artificial

<220>
<223>  Atp13a2F2 primer

<400>  58
ctcctcagct tcatccgtg                                                     19


<210>  59
<211>  19
<212>  DNA
<213>  artificial

<220>
<223>  Atp13a2R2 primer

<400>  59
tttccatcca gacgtagcg                                                     19


<210>  60
<211>  20
<212>  DNA
<213>  artificial

<220>
<223>  Gad65/67F1 primer

<400>  60
catacgcaga cagcacgttt                                                    20


<210>  61
<211>  20
<212>  DNA
<213>  artificial

<220>
<223>  Gad65/67R1 primer

<400>  61
aaaagattcc atcgccagag                                                    20


<210>  62
<211>  20
<212>  DNA
<213>  artificial

<220>
<223>  Gad65/67F2 primer

<400>  62
gggatgtcaa ctacgcgttt                                                    20


<210>  63
<211>  20
<212>  DNA
<213>  artificial

<220>
<223>  Gad65/67R2 primer

<400>  63
cacaaataca ggggcgatct                                                    20


<210>  64
<211>  20
<212>  DNA
<213>  artificial

<220>
<223>  DatF1 primer

<400>  64
ttctcctctg gcttcgttgt                                                    20


<210>  65
<211>  20
<212>  DNA
<213>  artificial

<220>
<223>  DatR1 primer

<400>  65
tggattgggt gtgaacagtc                                                    20


<210>  66
<211>  20
<212>  DNA
<213>  artificial

<220>
<223>  DatF2 primer

<400>  66
caacctgtac tggcggctat                                                    20


<210>  67
<211>  20
<212>  DNA
<213>  artificial

<220>
<223>  DatR2 primer

<400>  67
gcataggcca gtttctctcg                                                    20


<210>  68
<211>  22
<212>  DNA
<213>  artificial

<220>
<223>  ATPdelstR primer

<400>  68
cgtgggcctg cacaacctca gg                                                 22


<210>  69
<211>  22
<212>  DNA
<213>  artificial

<220>
<223>  cATP11F primer

<400>  69
agtacctcat cctggctgca gc                                                 22


<210>  70
<211>  44
<212>  DNA
<213>  artificial

<220>
<223>  V1+delstop primer

<400>  70
cctgaggttg tgcaggccca cgaagggcaa ttctgcagat atcc                         44


<210>  71
<211>  18
<212>  DNA
<213>  artificial

<220>
<223>  BGHrev primer

<400>  71
tagaaggcac agtcgagg                                                      18


<210>  72
<211>  23
<212>  DNA
<213>  artificial

<220>
<223>  cATP12R primer

<400>  72
ctgtgcgggt caccactgcc agg                                                23

<210>  73
<211>  46
<212>  DNA
<213>  artificial

<220>
<223>  cATP14F primer

<400>  73
cctggcagtg gtgacccgca cagctctcct cggcaccatc tacagc                    46


<210>  74
<211>  45
<212>  DNA
<213>  artificial

<220>
<223>  ATPins16R primer

<400>  74
ggccccacag gcaggcggca ggggccccac aggcaggcgg cgagg                     45


<210>  75
<211>  47
<212>  DNA
<213>  artificial

<220>
<223>  ATPins16F primer

<400>  75
gcccctgccg cctgcctgtg gggcccctgc tccgagcact ggccacc                   47


<210>  76
<211>  24
<212>  DNA
<213>  artificial

<220>
<223>  ATP26delcR primer

<400>  76
ggtcaggaag taccccctag ctgc                                            24


<210>  77
<211>  24
<212>  DNA
<213>  artificial

<220>
<223>  ATp26delcF primer

<400>  77
gcagctaggg ggtacttcct gacc                                            24


<210>  78

<211>  66
<212>  DNA
<213>  artificial

<220>
<223>  V5NHrev primer

<400>  78
cgtagaatcg agaccgagga gagggttagg gataggctta cccatgccgg ctcctcgcgc      60

tcatcg                                                                  66


<210>  79
<211>  67
<212>  DNA
<213>  artificial

<220>
<223>  V5NHforw primer

<400>  79
ggtaagccta tccctaaccc tctcctcggt ctcgattcta cgagcgcaga cagcagccct      60

ctcgtgg                                                                 67


<210>  80
<211>  23
<212>  DNA
<213>  artificial

<220>
<223>  ENO-2F primer

<400>  80
ggcactctac caggactttg tca                                               23


<210>  81
<211>  24
<212>  DNA
<213>  artificial

<220>
<223>  ENO-2R primer

<400>  81
gatccctaca ttggctgtga actt                                              24


<210>  82
<211>  29
<212>  DNA
<213>  artificial

<220>
<223>  ENO-2 probe

<400>  82
actatcctgt ggtctccatt gaggaccca                                         29

## Description

The present invention provides mutant forms of human lysosomal type 5 P-type ATPase ATP13A2, nucleic acid sequences coding for said mutant ATPase, and their use for diagnosis and therapy of neurodegenerative disorders, especially of parkinsonism and dementia. Test systems for testing candidate active agents for their therapeutic potential in diseases connected with ATP13A2 are also provided.

### Background of the Invention

Neurodegenerative disorders like Parkinson's and Alzheimer's disease cause motor and cognitive dysfunction and belong to a heterogeneous group of common and disabling disorders (Nussbaum, R.L. and Ellis, C.E., New Engl. J. Med. 348:1356 (2003)). They are often progressive and irreversible and most cases develop at an advanced age. Although nearly all neurodegenerative disorders have a genetic cause or component, the genetic basis of late-onset neurodegenerative disorders is mostly unknown. However, there are rare monogenic forms that typically show a much earlier age of onset of the disease. Although the complex molecular pathophysiology of neurodegeneration is still largely unknown, major progress has been made by elucidating the genetic defects in these Mendelian forms (Vila, M. and Przedborski, S., Nature Med. 10 Suppl:S58 (2004)). This has lead to the discovery of common pathophysiological pathways involved in neurodegeneration such as enhanced oxidative stress, protein misfolding and aggregation, as well as dysfunction of the ubiquitin-proteasome system (Ciechanover, A., Nature Rev. Mol. Cell. Biol. 6:79 (2005); Cookson, M.R., Annu. Rev. Biochem. 74:29 (2005); Dawson, T.M. and V.L., Science 302:819 (2003); Selkoe, D.J., Nature Cell Biol. 6:1054 (2004)).

Autosomal recessive parkinsonism with pyramidal degeneration and dementia (Kufor-Rakeb syndrome (KRS), PARK9) is a rare hereditary form with a juvenile onset and unknown genetic cause. In addition to typical signs of Parkinson's disease (PD), affected family members of the first described Jordanian family show symptoms of a more widespread neurodegeneration including dementia (Najim al-Din, A.S. et al., Acta Neurol. Scand. 89:347 (1994); Williams, D.R. et al., Mov. Disord. Epub ahead of print Jun 28 (2005)). In this single family the disease locus has been linked to a 9 cM (centiMorgan) spanning region on the short arm of chromosome 1 within a cluster of early-onset PD loci (1p36; Hampshire, D.J. et al., J. Med. Genet. 38:680 (2001)). Up to now, two monogenic PD genes in this region have been identified, namely DJ-1 (Bonifati, V. et al, Science 299:256 (2003)) and PINK1 (Valente, E. M. et al., Science 304:1158 (2004)). Also other monogenic neurodegeneration genes have been identified, however, the pathogenesis of these complex disorders is still not fully understood. Thus, the identification of further genes involved is of pivotal interest for the proper understanding of disease etiology, pathogenesis, and therapy.

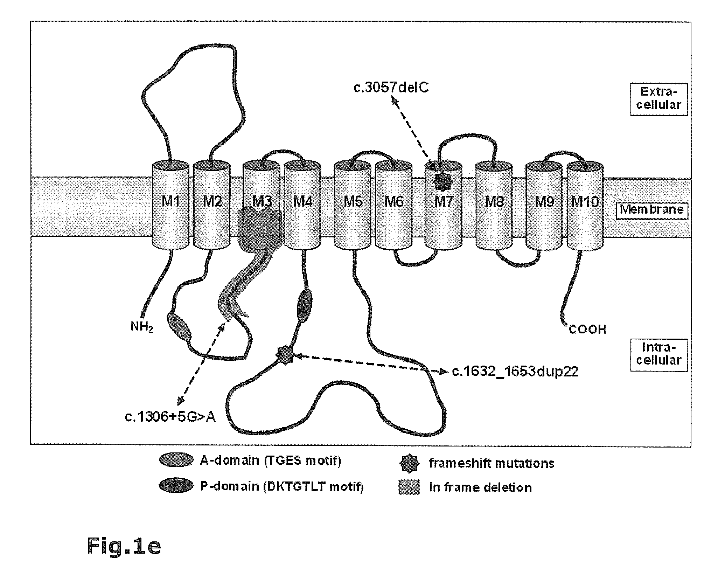

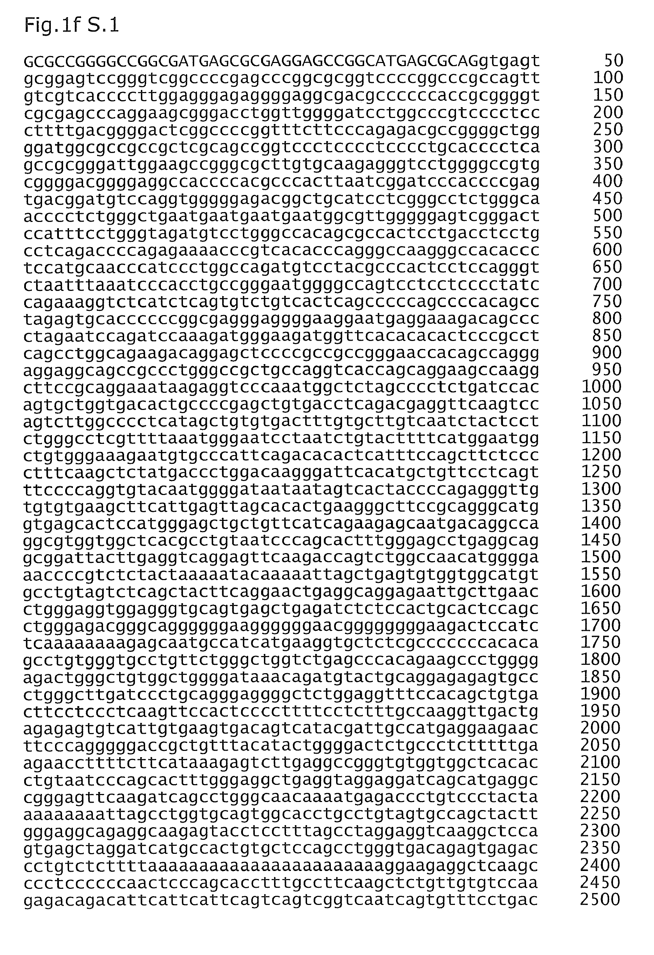

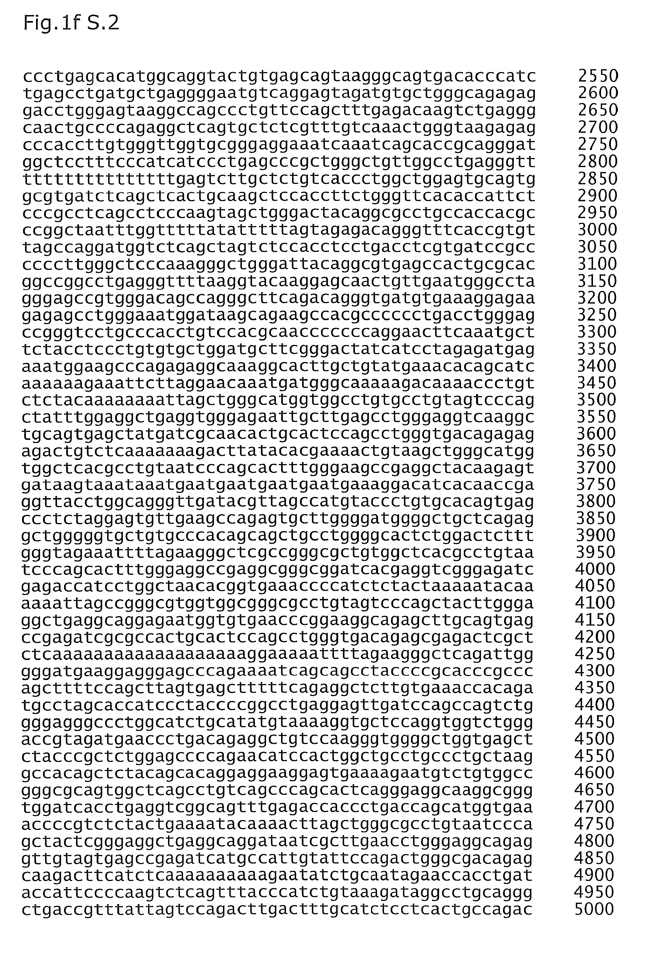

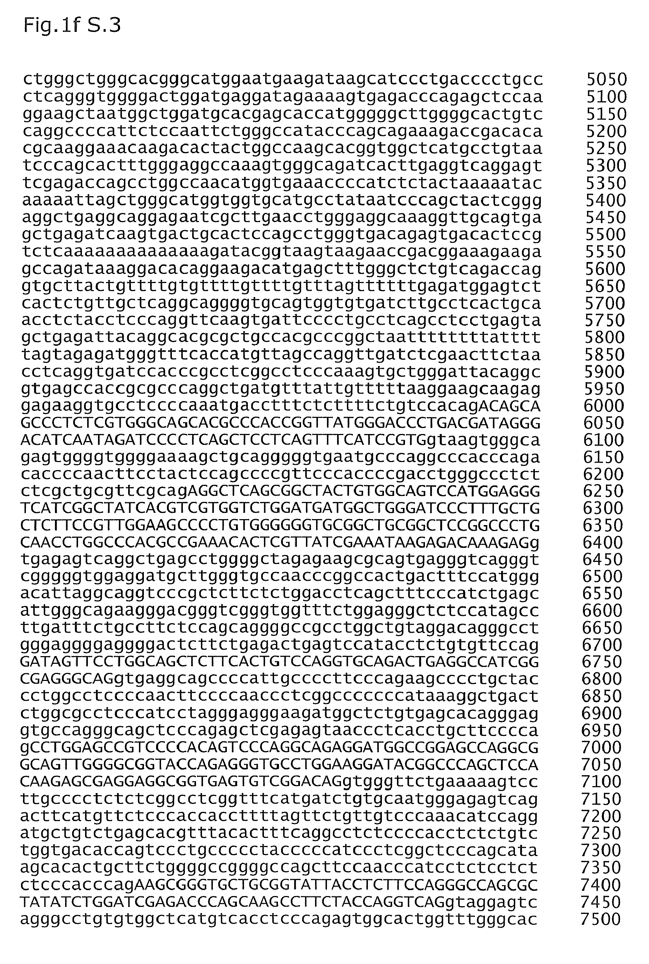

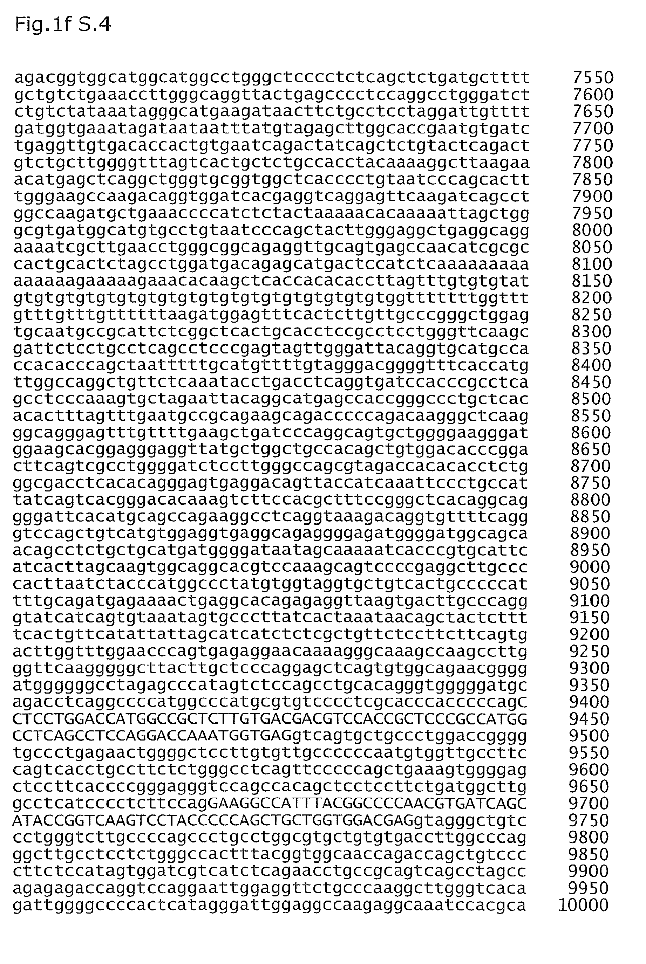

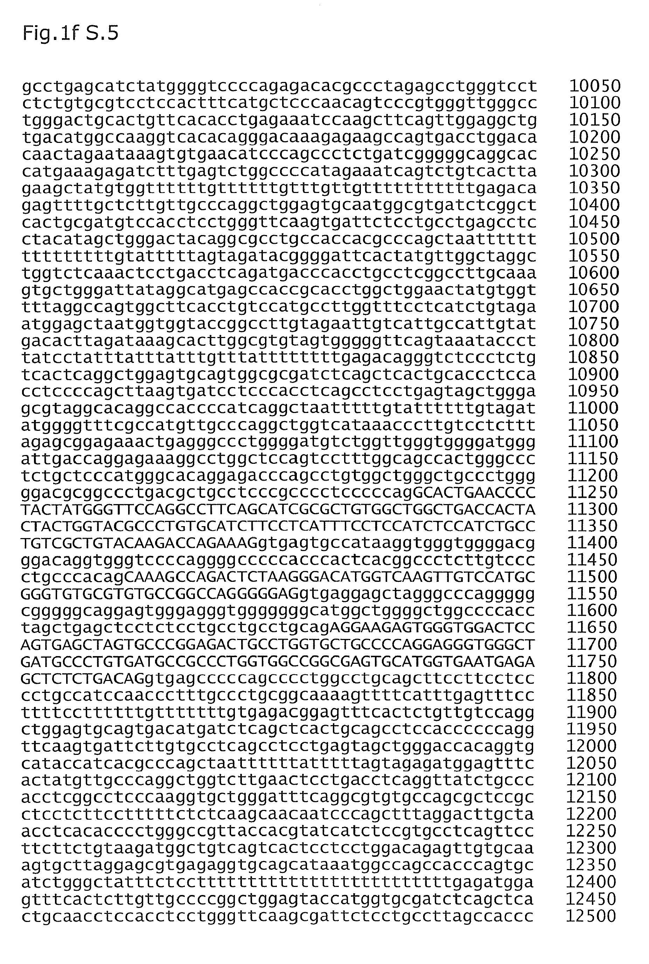

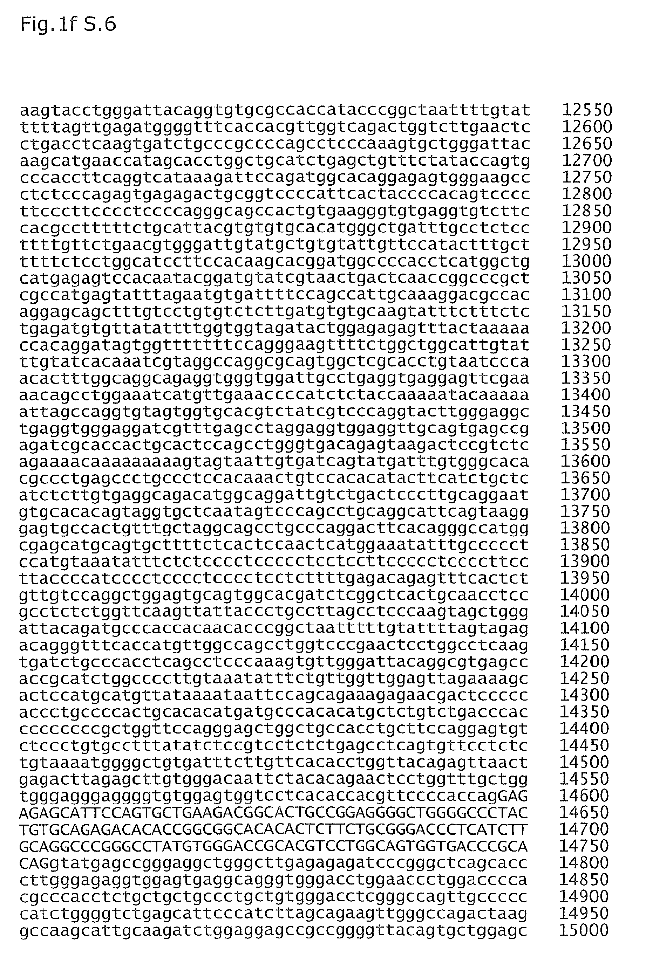

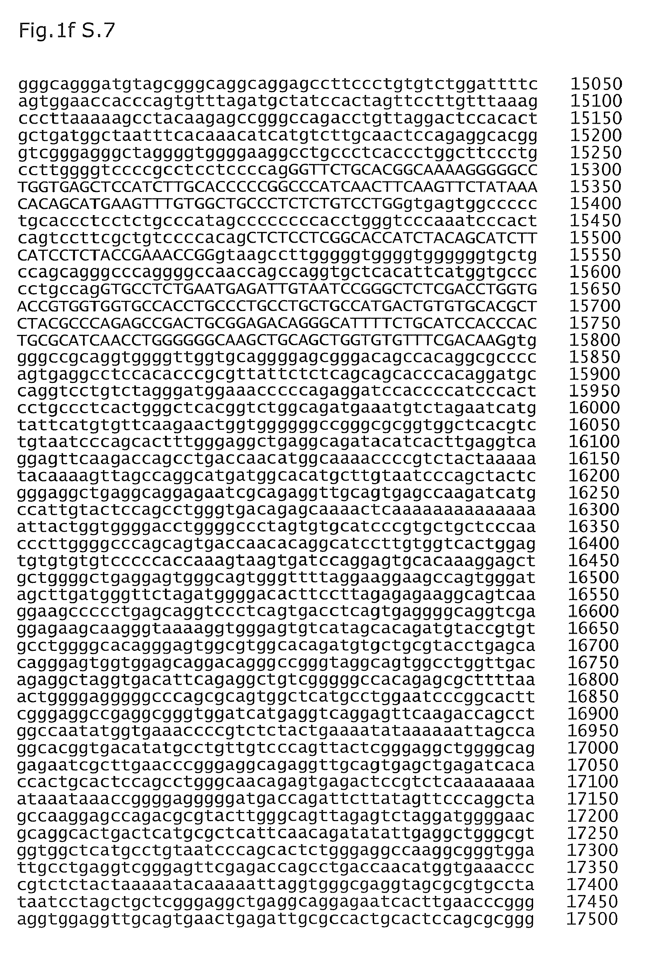

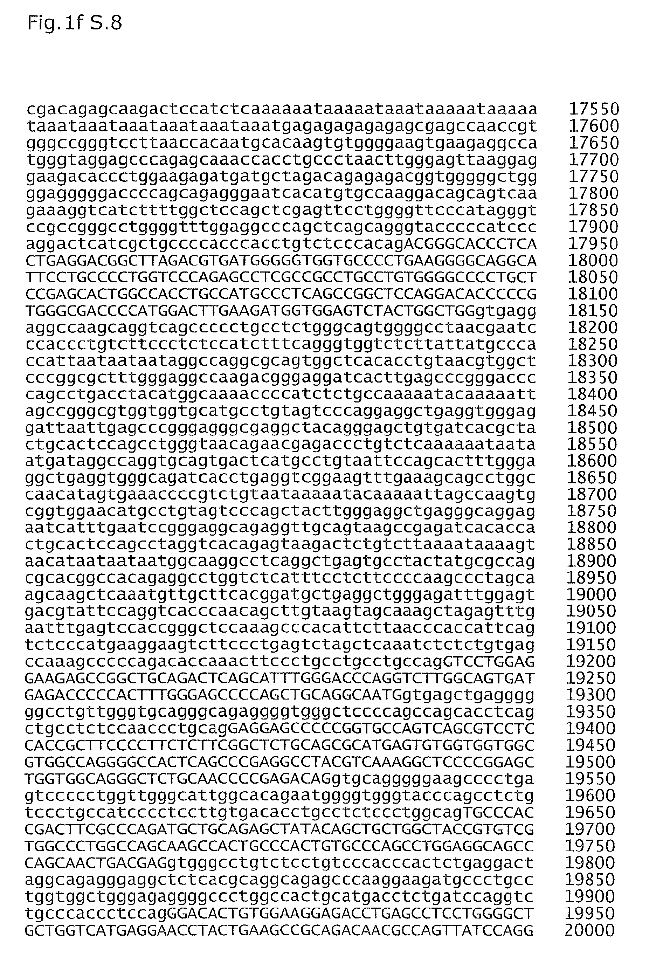

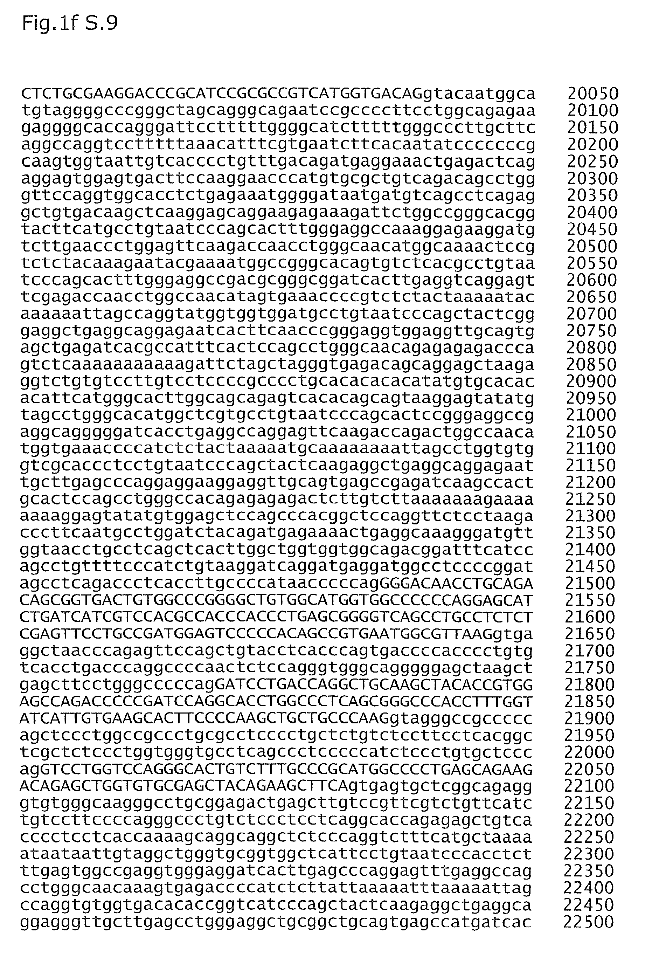

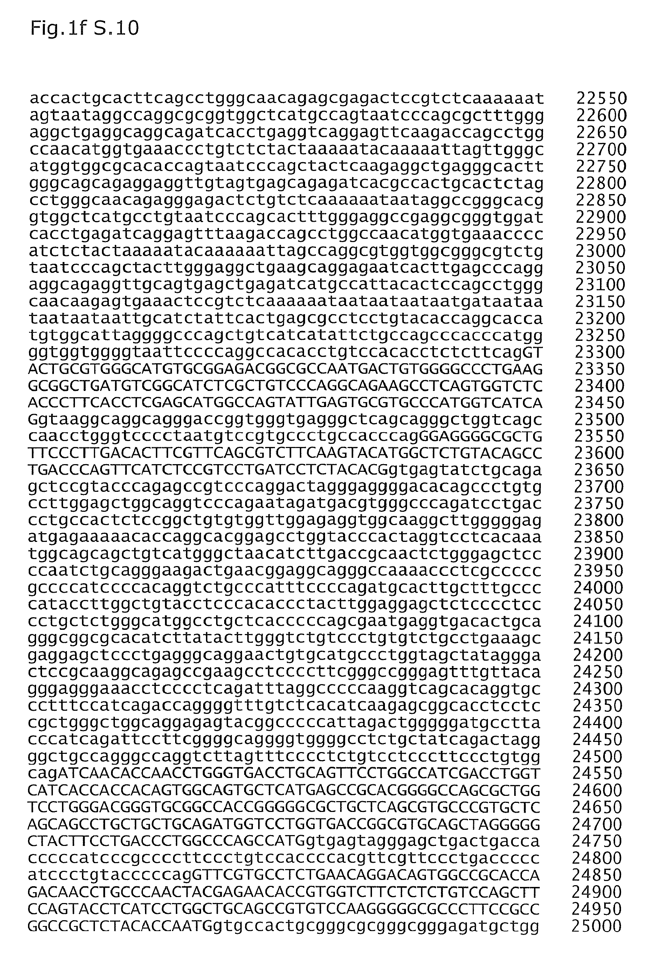

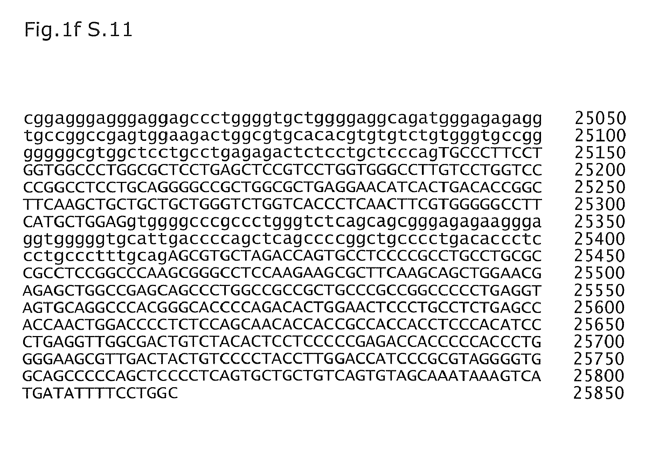

According to database analyses, there is a predicted human Type 5 P-type ATPase, named ATP13A2, which is located in the critical KRS-interval on chromosome 1p36. The cDNA encoding ATP13A2 consists of 3543 nucleotides (SEQ ID NO: 1) coding for a protein of 1180 amino acids (SEQ ID NO:2). Wild type (WT) ATP13A2 shares the characteristic functional A- and P-domains as well as the predicted 10-transmembrane (TM) domain topology with other members of this ATPase class (Fig. 1E). Its predicted molecular weight is 129 kDa. The human gene which encodes WT ATP13A2 is *ATP13A2.* It is organized in 29 exons and 28 introns (see Fig. 1F) and is localized on chromosome 1:17.057.758-17.083.573 (according to NCBI-Build 35, NM_022089). It is represented by SEQ ID NO:3. The *ATP13A2* gene has not been comprehensively analyzed on a molecular level up to now, but has just been predicted on the basis of EST and homology data (Schultheis, P.J. et al., Biochem. Biophys. Res. Commun. 323:731 (2004); Kwasnicka-Crawford, D.A. et al., Genomics 86:182 (2005)).

The "P-type" ATPases are a family of ATP directed pumps involved in transmembrane transport of ionic molecules (Axelsen, K.B. and Palmgren, M.G., J. Mol. Evol. 46:84 (1998); Kuhlbrandt, W., Nature Rev. Mol. Cell. Biol. 5:282 (2004)). Members of this family are e.g. the Na+/K+ ATPases and SERCA. The term "P type" originates from the fact that said ATPases form a phosphorylated intermediate during their reaction cycle. Based upon substrate specifity and sequence homology, P type ATPases are subdivided into 5 different branches. Type 5 P-type ATPases are P-type ATPases whose substrate (besides ATP) specifity and function are unknown so far (Axelsen, K.B. and Palmgren, M.G., J. Mol. Evol. 46:84 (1998)). Most type 5 P-type ATPases possess 10 TM domains. As P-type ATPases are transmembrane proteins, they are ideal pharmaceutical targets.

However, there is no indication in the literature that mutations of ATP13A2 and its gene may be causative for human disorders.

Currently, neurodegenerative disorders like Parkinsonism and dementia are not curable. Treatment is usually symptomatic and limited to selected symptoms. There is no cure, prophylaxis, or effective causative treatment available for these progressive disorders of the central nervous system. Thus, there is a need for early identification of neurodegenerative disorders and also for new medicaments for therapy of neurodegenerative disorders.

As insights into the complex molecular pathways involved in neurodegeneration are mainly gained by disease gene identification in hereditary forms of neurodegenerative disorders, there was a need to identify and characterize the gene involved in KRS/PARK9 and to evaluate its potential role in disease development and as new lever for diagnosis and therapy of neurodegenerative diseases.

### Summary of the Invention

It was found that loss-of-function mutations of *ATP13A2* are underlying PARK9, an autosomal recessive form of early-onset parkinsonism with pyramidal degeneration and dementia. Besides the human wild-type protein ATP13A2 also loss-of-function mutants thereof were identified. While the wild-type protein is located in the lysosome, the unstable truncated mutants are retained in the endoplasmic reticulum and are degraded by the proteasome. These findings link ATP13A2 to the protein networks implicated in severe neurodegeneration and especially Parkinsonism and dementia. Thus, a partial or complete loss-of-function mutation, preferably a mutation connected with partial or complete loss of one or more transmembrane domains, of ATP13A2 is deemed to be causative for neurodegenerative disorders including Parkinsonism and dementia, particularly for Parkinson's disease and Alzheimer's disease. Likewise, any dysfunction of the regulation of ATP13A2, as well as *ATP13A2* mutations leading to splicing errors, protein misfolding, erroneous posttranslational modifications, translocation errors, and impairment of protein function, are deemed to be causative for said neurodegenerative disorders.

The analysis of the ATP13A2/*ATP13A2* sequence and/or its expression provide tools for diagnosis and prognosis of neurodegenerative diseases, prevalently hereditary neurodegenerative diseases, preferably Parkinsonism and dementia. Furthermore, ATP13A2 and its mutant forms are targets for novel agents for treatment and prevention of neurodegenerative diseases, especially of Parkinsonism and dementia. Moreover, transgenic cells, tissues and animals which are heterologously expressing mutant ATP13A2 or are ATP13A2-knock-out mutants are useful model systems for neurodegenerative diseases correlated to ATP13A2 defects.

Thus, the present invention provides
(1) a method for prognosing, diagnosing and/or monitoring a disorder characterized by changes in the expression and/or translation of the lysosomal type-5 P type ATPase ATP13A2 (hereinafter "wild type ATP13A2") or by the presence of a loss-of-function mutant of ATP13A2 (hereinafter "mutant ATP13A2") in a biological sample isolated from a human subject, which method comprises detection of
   (i) the amount and/or activity of wild type ATP13A2 and/or of mutant ATP13A2 in the sample; and/or
   (ii) the nucleic acid sequence coding for wild type ATP13A2 or for mutant ATP13A2 in the sample, and/or
   (iii) mutations present in the ATP13A2 nucleic acid sequence of the sample in comparison to the wild type ATP13A2 nucleic acid sequence;
(2) an isolated loss-of-function mutant protein of the human ATP13A2 ("mutant ATP13A2"), which is causative for a disorder, especially for a neurodegenerative disease, more preferably for Parkinsonism or dementia, most preferably for Parkinson's disease or Alzheimer's disease;
(3) an isolated nucleic acid sequence encoding the mutant ATP13A2 as defined in (2) above;
(4) a vector comprising the nucleic acid sequence of (3) above;
(5) a cell, preferably an isolated cell which
   (i) is transfected or transformed with the vector of (4) above and/or
   (ii) heterologously comprises the nucleic acid of (3) above and/or
   (iii) is capable of heterologously expressing a mutant protein as defined in (1) or (2) above; or
   (iv) is a knock-out mutant not producing ATP13A2;
(6) an isolated tissue, tissue culture or a non-human transgenic organism comprising one or more of the cell of (5) above;
(7) use of the cell of (5) above, or the non-human transgenic organism, the tissue or tissue culture of (6) above as a test cell system, test tissue or test model organism in testing candidate active agents for their therapeutic potential in diseases correlated to ATP13A2, preferably in neurodegenerative diseases, more preferably in Parkinsonism and dementia, most preferably in Parkinson's disease and Alzheimer's disease;
(8) a method for testing candidate active agents for their therapeutic potential in diseases correlated to ATP13A2, preferably in neurodegenerative diseases, comprising the steps
   (a) administering the candidate agent to a culture of a cell as defined in (5) above, to a tissue or tissue culture or to the non-human transgenic organism as defined in (6) above; and
   (b) determining the effect of the candidate agent on said cell, tissue or non-human organism.
(9) an antibody specific for (i) the wild type ATP13A2, (ii) the mutant ATP13A2 as defined in (2) above; or (iii) the transmembrane domain(s) lacking in certain preferred embodiments of the mutant protein;
(10) a pharmaceutical or diagnostic composition comprising one or more of wild type ATP13A2, a nucleic acid encoding said wild type ATP13A2, and/or the antibody of (9) above, or a pharmaceutically or diagnostically acceptable salt thereof;
(11) use of one or more component(s) of the group consisting of wild type ATP13A2, a nucleic acid encoding said wild type ATP13A2, and/or the antibody of (9) above, and a pharmaceutically acceptable salt thereof, for preparing a medicament for prevention or treatment of neurodegenerative disorders; and
(13) a kit for performing the method of (1) above, comprising anyone of the mutant ATP13A2 of (2) above, the nucleic acid of (3) above, the vector of (4) above, the cell of (5) above, the antibody of (9) above, and/or primers or probes for a hybridization assay or PCR-based assay apt for specific detection of *ATP13A2* mutations.

### Description of the Figures

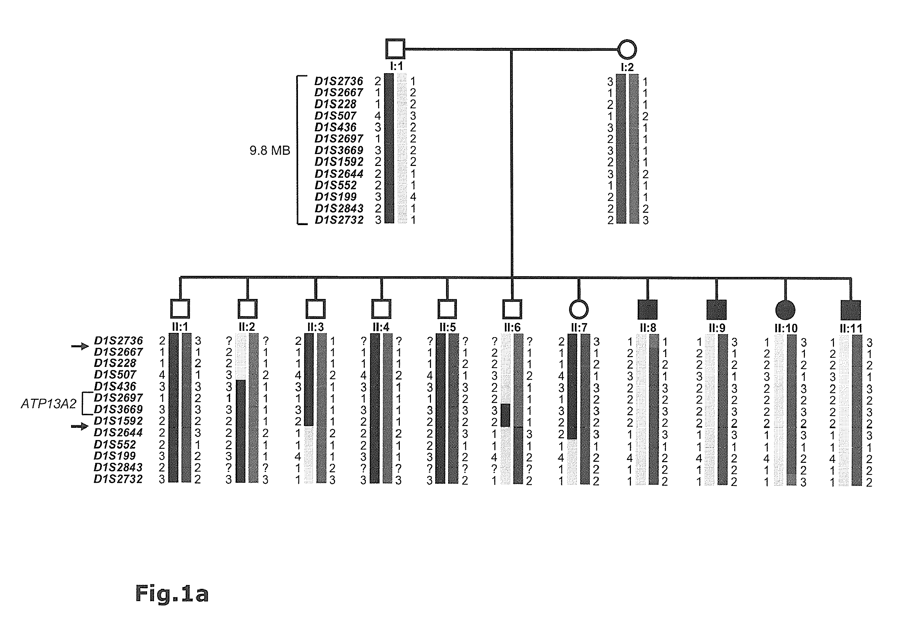

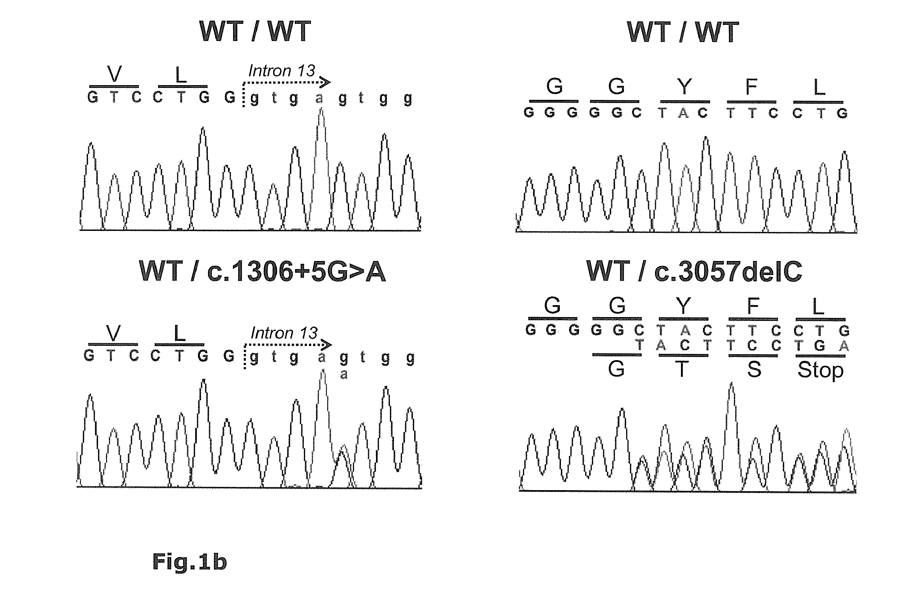

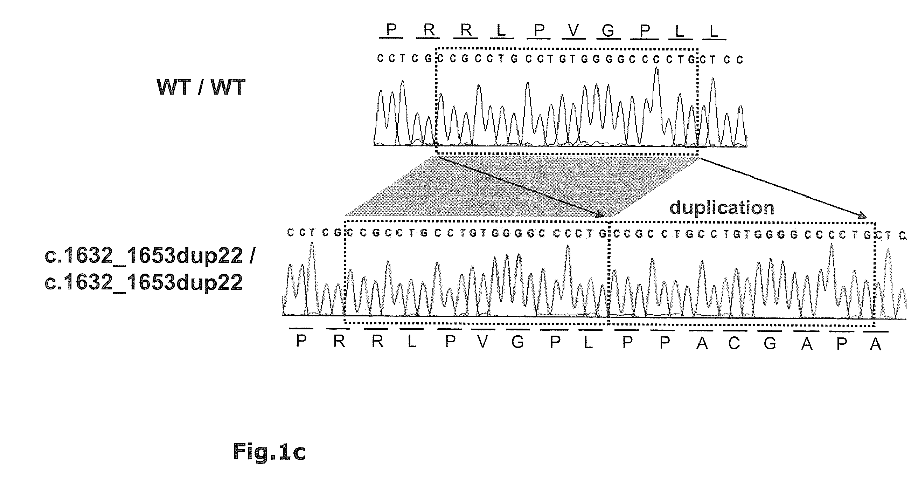

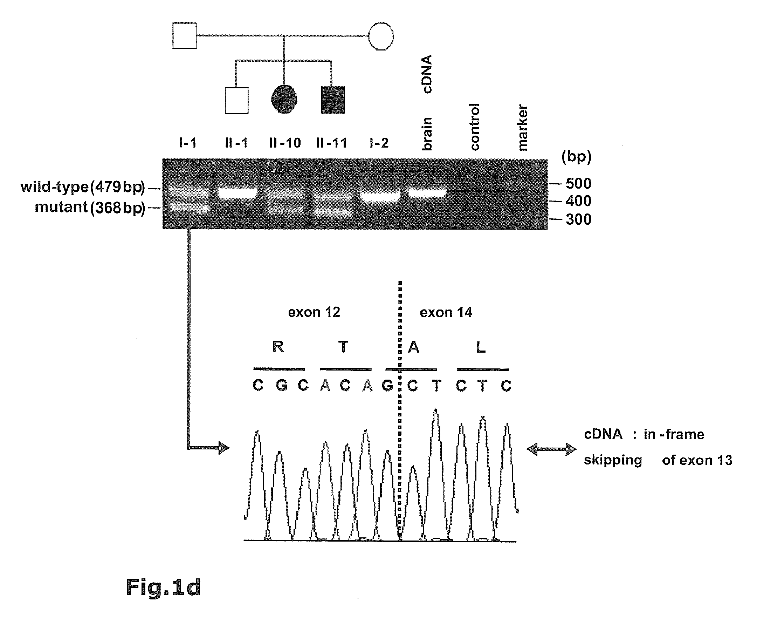

Fig. 1: Linkage analysis (Example 3) of the KRS-region and *ATP13A2* mutations.
   (A) Haplotype analysis of the KRS-locus in a Chilean family (Example 1). Affected family members are shown in black; circles and squares denote female and male individuals. The microsatellite markers are given on the left, the location of *ATP13A2* is indicated by the small bracket. Critical recombinations in II:8 (telomeric) and II:6 (centromeric) are shown by arrows.
   (B+C) Direct sequencing of mutation carriers and controls in the Chilean family (B) and the original KRS-family (Hampshire, D.J. et al., J. Med. Genet. 38:680 (2001); C). Wild-type (WT) sequences and amino acid translation are shown on top, mutations are shown below. The Chilean patients are compound heterozygous for c.1306+5G>A and c.3057deIC, the Jordanian patients are homozygous for c.1632_1653dup22.
   (D) RT-PCR analysis of the c.1306+5G>A mutation. Exons 12-14 of *ATP13A2* cDNA were amplified from whole blood of family members and control brain cDNA. In addition to the 479 bp WT product, the father and affected members show an aberrant amplicon of 368 bp. Sequencing reveals in-frame skipping of exon 13.
   (E) Topology model of ATP13A2 showing the location and effect of KRS mutations. The position of frameshift mutations is indicated by red stars, the in-frame exon skipping is shown by red shading.
   (F) ATP13A2 gene. Upper case letters: exons; lower case letters: introns. Start codon begins at position 52, stop codon ends at position 25552.

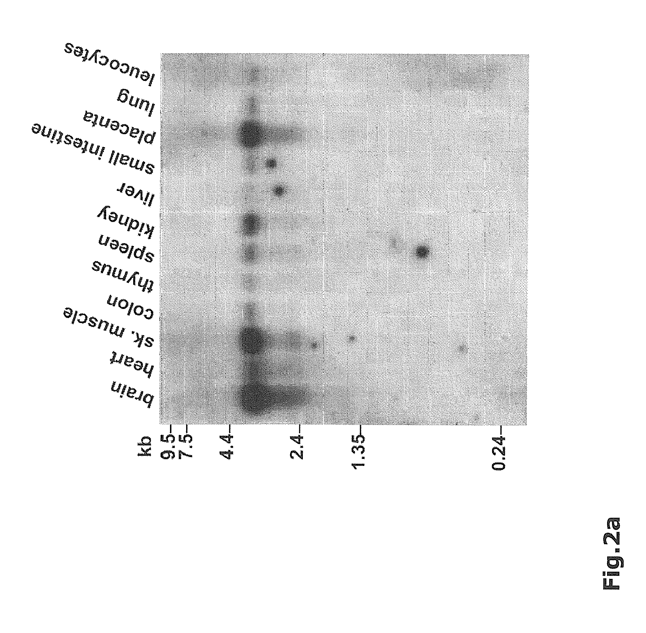

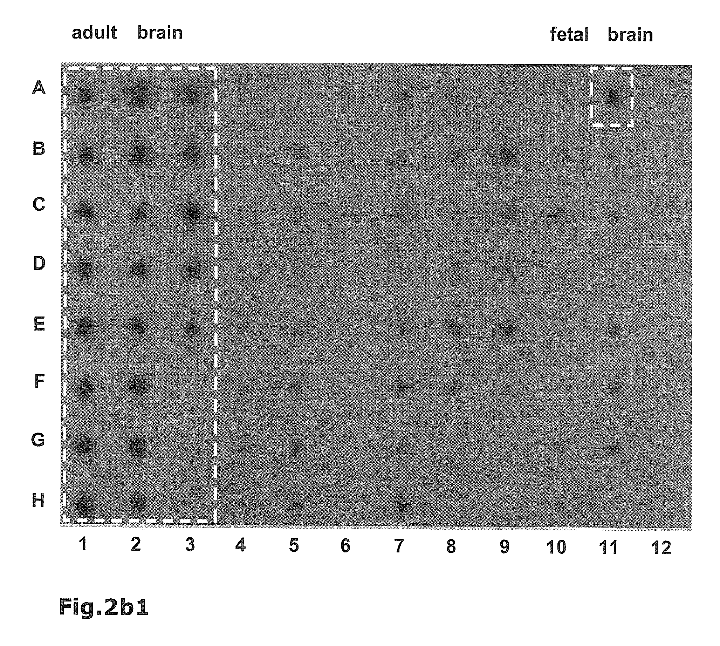

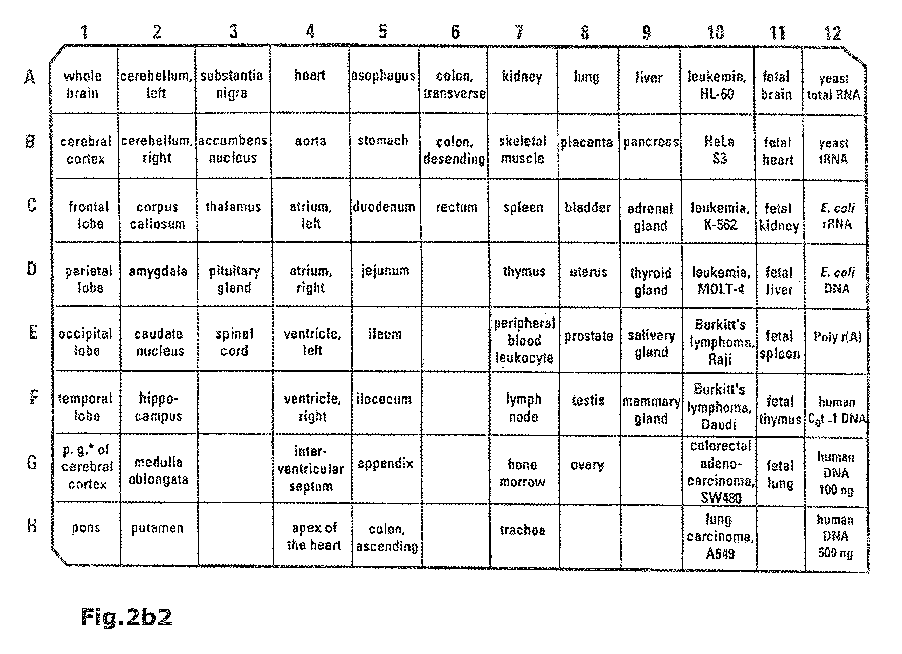

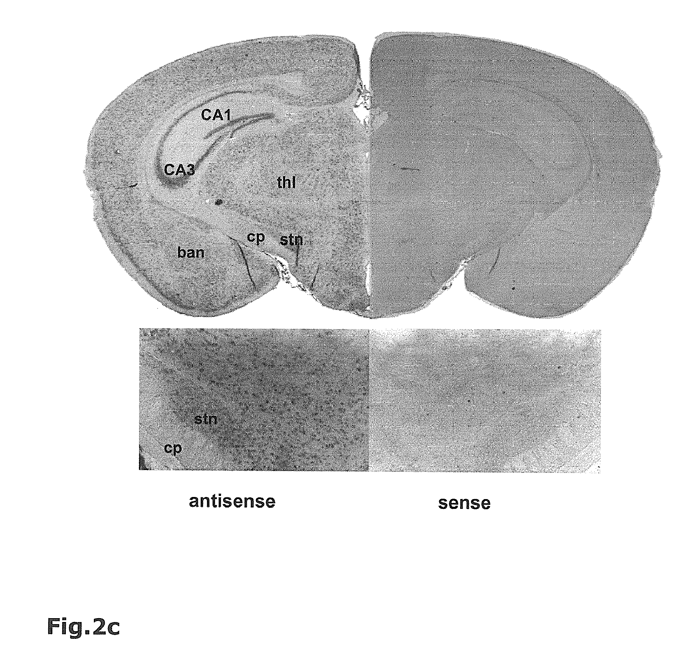

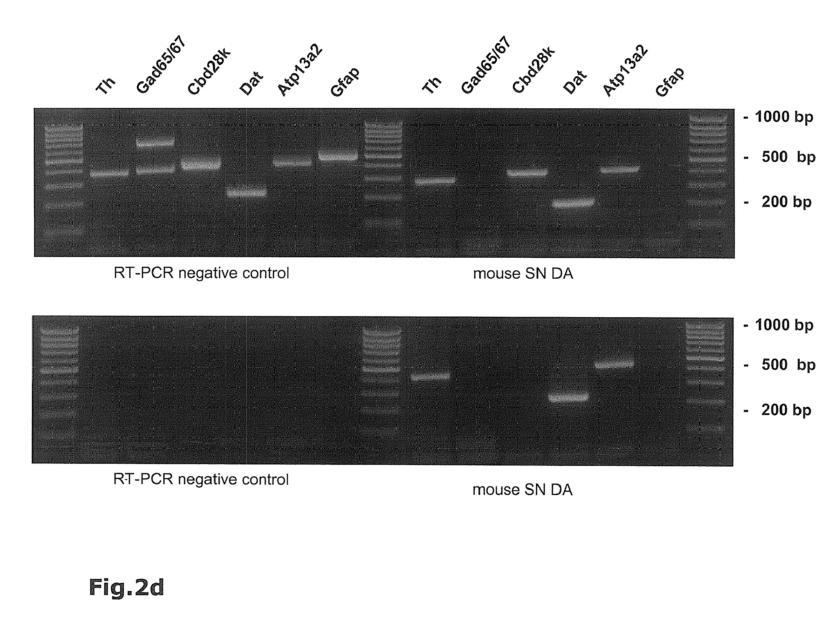

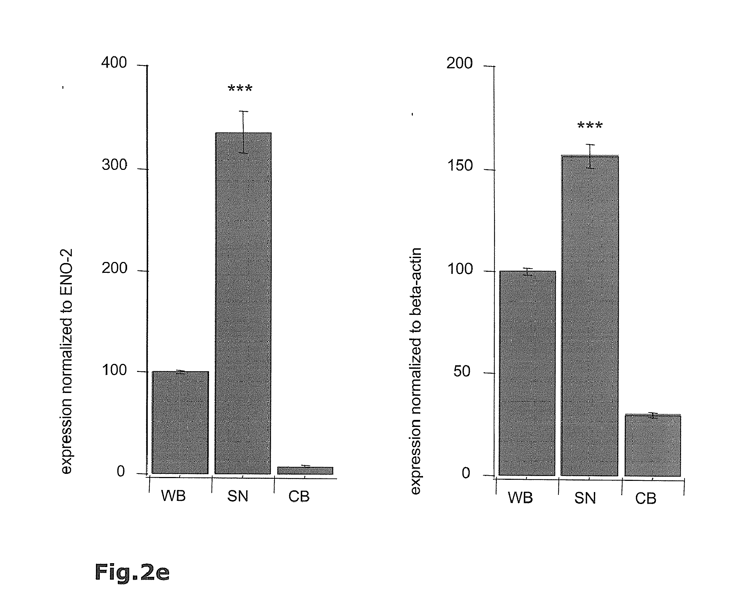

Fig. 2: Expression analysis of *ATP13A2.* Human multiple tissue (A) Northern and (B) dot blots (Clontech) show ubiquitous *ATP13A2* expression with highest expression in brain and all tested brain subregions (columns 1-3) including the substantia nigra (SN, dot A3) and fetal brain (allocation of tissues in (B) is given in Fig. 2B-2 according to BD^{™} TE Multiple Tissue Expression Array User Manual, May 2004).
   (C) *In situ* hybridization of an adult mouse brain section using a murine *Atp13a2* diglabelled antisense probe (left) or a sense control probe (right). *Atp13a2* is widely expressed in the CNS including e. g. the cortex, subthalamic nucleus (stn), thalamus (thl), and basolateral amygdaloid nucleus (ban). CA1, CA3: hippocampal CA1 and CA3 regions; cp: basal part of the cerebral peduncle.
   (D) *Atp13a2* is expressed in individual murine dopaminergic neurons from SN and ventral tegmental area (VTA). Laser-microdissected pools of individual cells: *Th-* and *Dat*-positive cells (SN): *Atp13a2* co-detection 84% (10 of 12); *Th-, Dat-* and *Cbd28k-*positive cells: 100% (12 of 12). Note absence of glia or GABAergic contamination. Electrophysically characterized individual *Th*-positive neurons (SN): *Atp13a2* co-detection 87% (n= 13 of 15); *Th-* and Cbd28k-positive neurons (VTA): 100% (n=8 of 8).
   (E) *ATP13A2* mRNA-levels are significantly higher in human SN compared to whole brain (WB) or cerebellum (CB). *ATP13A2* expression: normalized to actin: WB 100.0±1.84, SN 156±5.52, CB 30.19±1.41 (p=0.0006 SN/WB; p=0.00052 SN/CB); normalized to neuron-specific enolase ENO-2: WB 100.0±1.98, SN 335.74±20.35, CB 7.89±0.48; (p=0.0014 SN/WB; p= 0.0005 SN/CB); n=4 each.

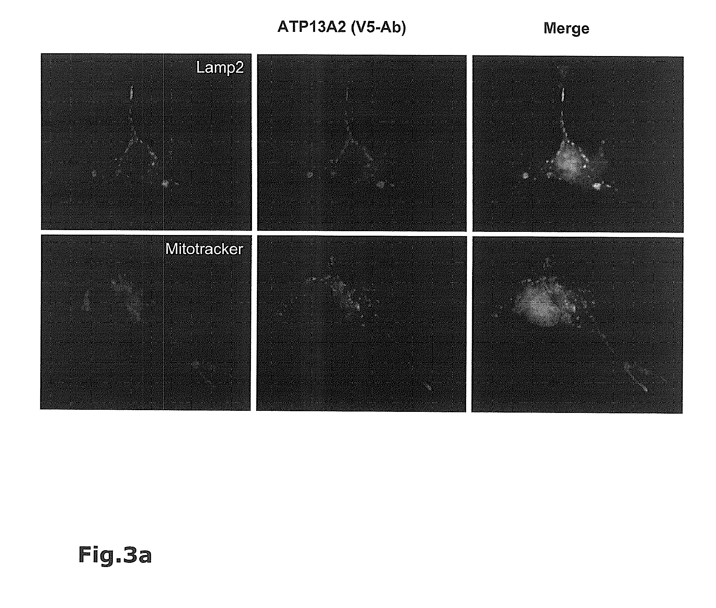

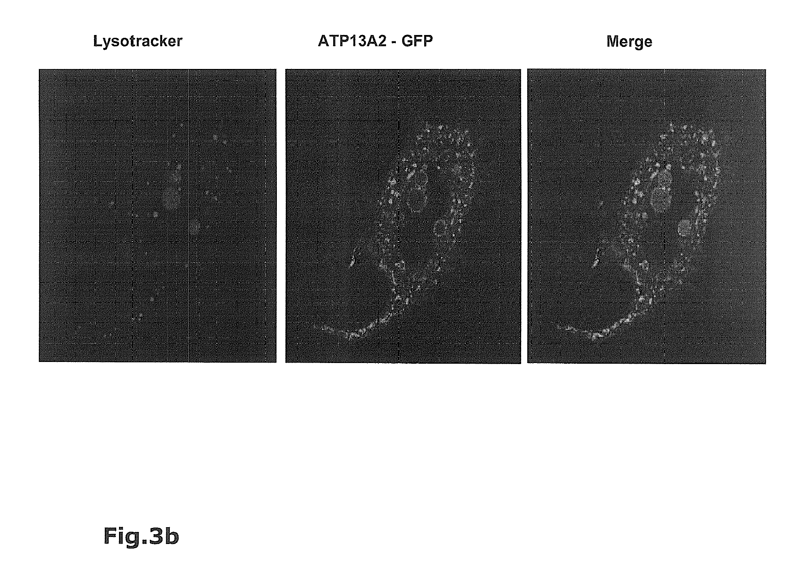

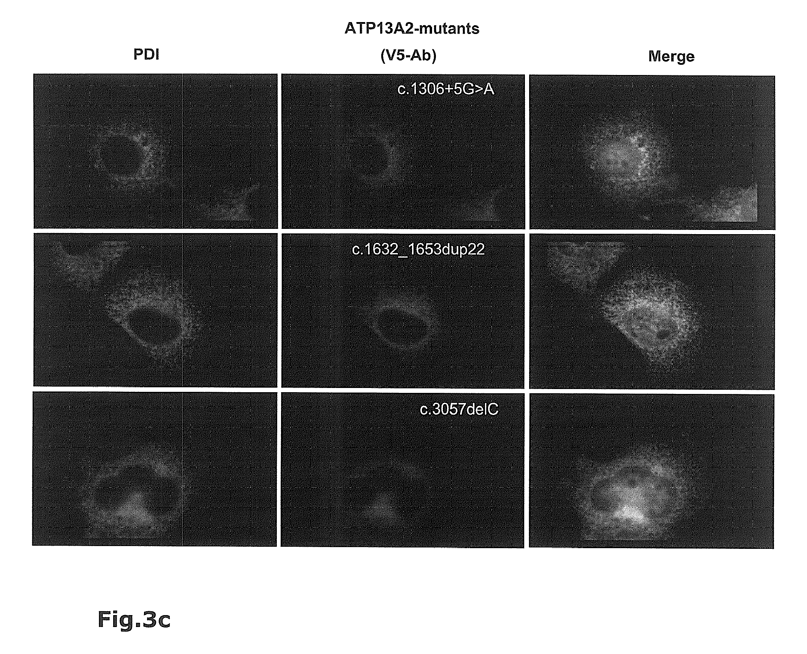

Fig. 3: Subcellular localization of ATP13A2-WT and KRS-mutants in transiently transfected COS7 cells by immunofluorescence.
   (A) Co-localization was observed between the V5-tagged WT-construct and the lysosomal marker protein Lamp2 (top). For comparison, no co-localization was found with the mitochondrial-specific dye MitoTracker Red (bottom).
   (B) The lysosomal localization of WT was confirmed by co-localization of an ATP13A2-EGFP fusion protein with LysoTracker in transiently transfected COS7 cells. Note the intralysosomal staining for LysoTracker, whereas WT-ATP13A2-EGFP stains the lysosomal membrane.
   (C) All KRS-mutants are mislocalized and co-stain with the endoplasmic-reticulum marker PDI (protein disulfide isomerase).

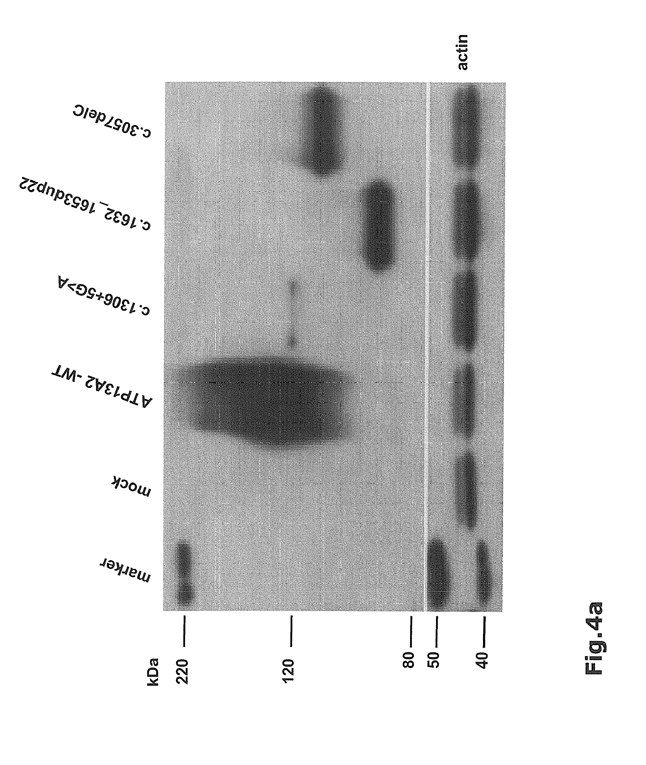

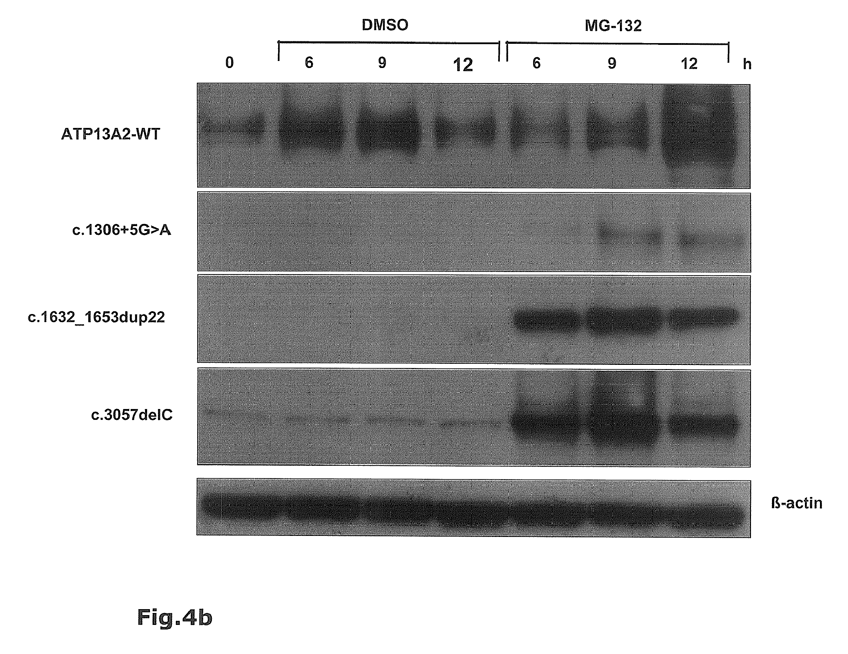

Fig. 4: Western blot analysis of ATP13A2 WT and mutants expressed from the corresponding constructs in transiently transfected COS7 cells.
   (A) Immunoblotting shows a signal in the expected size range for all constructs. The stability of mutants is severely reduced when compared to WT-levels and the actin control (bottom). Note lower amount of total protein in mock-transfected (vector pcDNA3.1 without insert) and WT-transfected samples.
   (B) The proteasomal inhibitor MG-132 (5 µM) strongly stabilizes KRS-mutants after 6-12 hours demonstrating the involvement of the proteasome in the degradation of KRS-mutants.

**Sequence Listing - Free Text**

| SEQ ID NO: | Description |
|---|---|
| 1 | *ATP13A2* WT cDNA |
| 2 | ATP13A2 WT |
| 3 | *ATP13A2* gene |
| 4 | ATP13A2 c.1306+5G>A mutation cDNA |
| 5 | ATP13A2 c.1306+5G>A mutation |
| 6 | ATP13A2 c.3057delC mutation cDNA |
| 7 | ATP13A2 c.3057delC mutation |
| 8 | ATP13A2 c.1632_1653dup22 mutation cDNA |
| 9 | ATP13A2 c.1632_1653dup22 mutation |
| 10 to 82 | primers and probes; see examples 4, 5, 9 and 10 |

### Definitions

In the framework of the present invention the following abbreviations, terms and definitions are used.

The abbreviations used are: ER, endoplasmic reticulum; GFP, green fluorescent protein; SN, substantia nigra; DA, dopaminergic; VTA, ventral tegmental area; WT, wild type; TM, transmembrane; AD, Alzheimer's Disease; PD, Parkinson's Disease.

In the context of present invention, the term "ATP13A2" refers to the wild type ATPase ATP13A2 and its amino acid sequence, more preferably to human ATPase ATP13A2, most preferably to human ATPase13A2 of SEQ ID NO:2. Said human ATP13A2 is most preferably encoded by SEQ ID NO:1 or the coding parts of SEQ ID NO:3.

*Atp13a2* refers to the murine gene encoding ATP13A2, while *ATP13A2* refers to the human gene encoding ATP13A2.

A "nucleic acid sequence coding for", e.g. ATP13A2, may be any nucleic acid, preferably DNA or RNA, coding for the respective proteins or peptides mentioned in present application. Said nucleic acid sequence is either identical or substantially identical to the native sequence of the gene (fragment) corresponding to said protein or peptide, or to the nucleic acid sequence corresponding to the amino acid sequence of said protein or peptide. In the context of present invention, a specific nucleic acid sequence refers to said sequence itself and nucleic acid sequences which are substantially identical thereto. A "substantially identical" nucleic acid sequence differs from the initial nucleic acid only by degenerated codon exchange. Thus, in a substantially identical nucleic acid sequence the codons inside the coding sequences are changed in a way which will not lead to a change of the amino acid sequence of the translation product (i.e. replacement of a codon by another codon out of its codon family).

The nucleic acid sequences of the invention are preferably used as full length sequences. However, they may also be used as addition or deletion mutants of said full length sequences for the performance of present invention, especially if they are used as primers or hybridisation probes, if non-coding parts of the sequence are removed (e.g. for cDNA-construction), or if a nucleic acid encoding a mutant ATP13A2 is generated. Thus, in one preferred aspect the allowable additions and deletions in the nucleic acids of the invention are either the removal or addition of non-coding regions, or they correspond to amino acid additions and deletions at the C- and/or N-terminus of the corresponding protein.

A "nucleic acid sequence fragment" is a part of the full length nucleic acid sequence which is shorter that full length, but which is still able to hybridize under stringent conditions to the full length nucleic acid sequence. It contains preferably at least 15 nucleotides, more preferably at least 25 nucleotides.

The term "vector" is generally understood by a person skilled in the art and comprises a DNA molecule, or its corresponding RNA molecule, derived from a plasmid, a bacterial phage, or a mammalian or insect virus, into which fragments of DNA may be inserted or cloned. A vector comprises one or more unique restriction enzyme sites and may be capable of autonomous replication. Furthermore the vector is capable of expressing the inserted or cloned fragment by providing transcription control elements, such as a promoter and transcription termination signals. The DNA fragment inserted into the vector, here the DNA coding for ATP13A2 or one of its mutants according to the invention, is functionally linked to said transcription control elements. In addition the vector may also comprise a nucleic acid sequence encoding marker proteins. Examples of suitable vectors include retroviral vectors, vaccinia vectors, adenoviral vectors, herpes virus vectors, fowl pox virus vectors, plasmids and baculovirus transfer vectors.

The terms "transfected" and "transformed" refer to the introduction of a nucleic acid into a cell. Various transfection and transformation methods are known to the person skilled in the art. The term "transfection" generally describes the introduction of a nucleic acid into a mammalian cell, whereas the term "transformation" describes the uptake of a nucleic acid by a microbial cell such as fungal cells or prokaryotes. The means by which the nucleic acids are introduced into the cell include microinjection, lipofection, electroporation, calcium phosphate transfection, DEAE-dextran transfection or infection with a recombinant virus harbouring said nucleic acid (Sambrook et al. in "Molecular Cloning. A Laboratory Manual", Cold Spring Harbor Press, Plainview, New York (1989)).

"Native" is used synonymously to "naturally occurring".

A "wild type" gene, allele, protein or organism is the allele, genotype, or phenotype that is most prevalent in populations. With reference to the wild-type gene/allele, a change, deletion, or rearrangement in said DNA sequence that may lead to the synthesis of an altered protein ("mutant protein") which is either partially or completely inactive or has a novel, non-native activity, or to the partial or complete loss of the ability to produce the protein is called a "mutation" and the corresponding altered DNA sequence is a "mutant gene". In other words, a mutation leads to a phenotype which is different from the wild type.

Preferably, a mutant ATP13A2 protein in the context of present application is a loss-of-function mutant, i.e. it has lost part or total of the native activity/function of the wild type ATP13A2. More preferably, the mutant ATP13A2 has lost its native function completely. In a further preferred aspect of present invention, the loss-of-function mutant is not localized in the lysosome of its cell of origin *in vivo* and *in vitro.*

A "KRS-mutant" in the context of present invention is a mutant ATP13A2 protein or ATP13A2 gene detectable in KRS patients. Preferably, it is represented by SEQ ID NOs:4 to 9.

The term "isolated" means that a molecule selected from the group comprising proteins, peptides and nucleic acids is/has been separated or purified from other molecules of said group, wherein said other molecules are usually associated with said isolated molecule in its organism of origin. This encompasses biochemically purified molecules, recombinantly produced and chemically synthesized molecules of said group. In case of membrane anchored proteins like WT ATP13A2, this also encompasses membrane vesicles containing the intact protein in their membrane layer. An "isolated cell" is a cell which is separate from its organism of origin. An "isolated tissue" is a tissue which is separate from its organism of origin, preferably a primary tissue.

The "organism" according to present invention includes vertebrates and invertebrates, eucaryotes and procaryotes. The term "animal" encloses all animals with the exception of humans. Preferred animals in the context of present invention are vertebrates.

The term "vertebrate" according to the present invention relates to an animal having a backbone or spinal column including mammals, birds, reptiles, amphibians and fish.

Preferred vertebrates are mammals (including humans, rodents (such as mice etc.), birds and fish).

The "cell", "tissue" and "tissue culture" of present invention are derived from the organism or vertebrate se defined herebefore. Furthermore, the cell may be a lower eucaryotic cell such as yeast or a procaryotic cell such as *E. coli.*

A "tissue culture" according to the present invention refers to animal tissue, organ primordia, or the whole or part of an organ which is maintained or grown *in vitro* so as to preserve its architecture and/or function. Preferred tissue cultures in present invention are from vertebrate tissue. Furthermore, said tissue culture is preferably a primary tissue culture.

A "cell culture" according to the present invention includes isolated cells which are cultured *in vitro.* These cells can be immortalized by fusion with tumor cells (tumor derived cells) or obtained directly from their native source (primary cell culture). They may be transformed or untransformed.

"Neurodegenerative diseases" (synonymous to "neurodegenerative disorders") are a group of mostly progressive neurological disorders caused by the irreversible deterioration of essential cell and tissue components of the nervous system. They are characterized by an irreversible loss of neurons resulting in an impairment of motion and/or cognitive abilities. Said diseases include AD, PD, Parkinsonism, neurodegenerative dementia, neurodegenerative motion impairment, prion diseases, spinocerebellar ataxies and Chorea Huntington. In the context of present invention, the term "neurodegenerative disorders" preferably addresses Parkinsonism and dementia, more preferably PD and AD. This is due to the fact that ATP13A2 may not only be correlated to PD, Parkinsonism and dementia, but also to other neurodegenerative diseases, especially to Alzheimer's disease (AD). Genome-wide linkage disequilibrium mapping of late-onset Alzheimer's Disease revealed that the locus 1p36.12, which is located in the same region on the short arm of chromosome 1 as the KRS locus, is associated with AD (Hiltunen, M. et al., Neurology 57:1664 (2001)).

"Parkinsonism" is a generic term referring to all disorders whose symptoms are the same as symptoms of PD, without necessarily being PD. The cardinal symptoms are tremor, rigidity, bradykinesia and postural instability. "Parkinson's disease" is the most prevalent kind of Parkinsonism. It is a neurodegenerative disease of the SN which involves a progressive deterioration of the extrapyramidal system.

### Detailed Description of the Invention

The method of embodiment (1) is useful for diagnosis, prognosis and/or monitoring of a disorder, preferably of a neurodegenerative disorder. More preferably, said disorder is Parkinsonism or dementia, most preferably is PD or AD.

The method of embodiment (1) may be based on detection of the ATP13A2 protein, gene or mRNA by standard immunological or molecular biology methods like ELISA, PCR, Western blots, hybridization of DNA probes, DNA electrophoresis, differential display, direct sequencing etc..

In one aspect of embodiment (1) the ATP13A2 activity, may it be WT ATP13A2 or mutant ATP13A2 activity, is detected. In another aspect, mutants of ATP13A2 and/or *ATP13A2* are detected by standard molecular biology methods after protein and/or nucleic acid isolation from the sample. The latter method is preferred for prognosis and diagnosis of hereditary forms of neurodegenerative disorders correlated to ATP13A2 dysfunction.

In a further preferred aspect of the method according to embodiment (1), the detection step comprises an immunoassay, preferably an immunoassay utilizing at least one antibody specific for (a) the wild type ATP13A2, (b) the mutant ATP13A2, and/or (c) one or more of the C-terminal transmembrane (TM) domains of wild type ATP13A2, more preferably an antibody specific for the transmembrane domains of ATP13A2.

In even a further preferred aspect, the method (1) comprises a hybridisation assay or PCR-based assay which is apt for specific detection of mutations in the *ATP13A2* nucleic acid sequence, preferably for detection of deletion mutants, more preferably for detection of one of the DNA sequences as represented by SEQ ID NOs:4, 6 and 8.

Embodiment (2) of present invention encompasses mutant forms of ATP13A2 which have partially or completely lost their native function ("mutant ATP13A2"). This loss-of-function leads in one preferred aspect of embodiment (2) to a neurodegenerative disease as defined above. In a very special aspect, said disease is early-onset PD which may additionally be connected with dementia.

The mutant ATP13A2 of present invention owe their loss of function to one or more mutations in the *ATP13A2* gene which preferably lead to one or more of the group consisting of splicing errors, protein misfolding, erroneous posttranslational modifications, translocation errors, and impairment of enzyme function. The most prominent errors in connection with the mutant ATP13A2 are translocation errors leading to a mislocalization, i.e. the mutant ATP13A2 is not localized in the destination of the WT ATP13A2 (the lysosome) *in vivo.* Thus, in a preferred aspect of embodiment (2), the mutant ATP13A2 is not localized in the lysosome of the cell of its origin *in vivo* and *in vitro.* In another preferred aspect, it is localized in the ER *in vivo* and *in vitro.*

Another prominent error in connection with the mutant ATP13A2 is truncation of the WT ATP13A2. Thus, in another preferred aspect of embodiment (2), said mutant ATP13A2 are characterized in that they are truncated in comparison to WT ATP13A2, preferably by at least partial loss of at least one of the TM domains. More preferably they lack more than one of the TM domains completely. Most preferred are mutant ATP13A2 which lack at least the three TM domains at the C-terminus of the WT ATP13A2, even more preferably the C-terminal six TM domains.

The truncation of the mutant ATP13A2s is causative for a mislocalization of the mutant ATP13A2, especially for localization to the ER. In turn, said localization allows a subsequent proteasomal degradation. This leads to a reduced *in vivo* stability of the mutant ATP13A2 in comparison to the wild type ATP13A2. Thus, reduced *in vivo* stability is another preferred aspect of embodiment (2) of present invention.

In another preferred aspect of embodiment (2), the mutant ATP13A2 has lost part or all of its native function not due to truncation of the transmembrane domain, but due to one of the other errors listed above. Thus, in one sub-aspect said mutant ATP13A2 is a substitution, deletion and/or addition mutant of WT ATP13A2. Preferably, the function and/or conformation of the ATP13A2 is affected by the substitution(s), deletion(s) and/or addition(s). In case of amino acid substitutions, said substitutions are preferably non-conservative. The ratio of substituted, added or deleted amino acids in comparison to the native sequence is preferably more than 10 % of the total amino acids in the sequence, more preferably more than 30 %.

Embodiment (2) even encompasses fragments of mutant ATP13A2. Said fragments must still possess the immunologic properties of the full length mutant ATP13A2 of which they are derived. Thus, the minimum length of said fragment is determined by its ability to be recognized and bound by an antibody which is specific for the full length mutant ATP13A2.

In another preferred aspect, the mutant ATP13A2 is fusioned with at least one second protein or non-proteinaceous component. The latter may be selected from non-nateve chemical components (such as polyethers including PEG, polyesters, alkylation products etc.), polysaccarides and lipids. The second protein is preferably a marker protein (like a tag, GFP, etc.).

There are three mutant ATP13A2 which are especially preferred, namely the proteins which correspond to the DNA level mutations c.1306+5G>A, c.1632_1653dup22, and c.3057deIC, respectively. They have a predicted molecular weight of 125, 85, and 111 kDa and the amino acid sequences SEQ ID NOs:5, 7 and 9, respectively. The ER-associated degradation is especially pronounced for the protein resulting from mutation c.1306+5G>A, in which in-frame skipping of exon 13 and removal of half of TM domain M3 obviously lead to a severe protein folding defect. Thus, mutant ATP13A2 containing one of SEQ ID NOs:5, 7 and 9 are especially preferred aspects of embodiment (2).

The mutant ATP13A2s according to embodiment (2) are detectable by routine molecular genetic methods, e.g. nucleic acid sequencing, immunological detection, detection by hybridization with ATP13A2 specific probes, PCR with WT and/or mutant ATP13A2 specific primers, etc..

The mutant ATPase according to embodiment (2) of the invention is a vertebrate ATPase, preferably a mammalian ATPase, more preferably a human ATPase.

The nucleic acid sequence of embodiment (3) which encodes the mutant ATP13A2 is a mutant sequence of the WT gene or cDNA. It is preferably expressed in neurons, more preferably in brain, even more preferably in the substantia nigra.

The nucleic acid sequence of embodiment (3) preferably differs from the native *ATP13A2* in at least one nucleotide exchange, deletion or addition which leads to a frameshift, exon skipping and/or to a premature stop-codon. In one especially preferred aspect of embodiment (3), the nucleic acid sequence differs from the native *ATP13A2* by exchange of single nucleotides. In another especially preferred aspect, it contains one or more added nucleotides, esepcially duplicated regions of from 2 to 50 nt.

In one preferred aspect of embodiment (3), the nucleic acid sequence is a sequence encoding a truncated ATP13A2, preferably a truncated ATP13A2 as outlined above for embodiment (2). This truncation is preferably due to said frameshift, exon skipping and/or premature stop codon.. As outlined above, the transcription product in one aspect lacks at least one TM domain of the WT protein.

A preferred nucleotide exchange leading to exon skipping is a guanine to adenine transition at position +5 of the donor splice-site of exon 13 of the WT gene (c.1306+5G>A, Fig. 1B left side; SEQ ID NO:3, nt 15392). This mutation of a highly conserved splice-site residue leads to an in-frame skipping of exon 13 (removing 111 nucleotides) in translation.

Alternatively most preferred is a homozygous 22 bp duplication in exon 16 (c.1632_1653dup22 or p.552LfsX788, Fig. 1C; SEQ ID NO:3, nt 18027 to 18048) leading to a frameshift and stop-codon after 236 extraneous amino-acids.

An additional mutation may be a deletion of a single cytosine at nucleotide position 3057 in exon 26 of ATP13A2 leading to a frameshift and stop-codon after two extraneous amino acids (c.3057deIC or p.1019GfsX1121, Fig. 1B right side; SEQ ID NO:3, nt 24701).

Said frameshift mutations remove the C-terminal three and six TM domains, respectively, which is expected to cause a loss-of-function (the predicted topology model and location of mutations are shown in Fig. 1E). Furthermore, the in-frame skipping of exon 13 removes 37 amino-acids including half of the TM domain M3 (Fig. 1E). The remaining hydrophobic residues of this domain are not able to span the membrane which also leads to a profound distortion of transmembrane topology and loss of protein function.

Thus, in a preferred aspect of embodiment (3) for a nucleic acid sequence encoding the mutant ATP13A2 of present invention, said nucleic acid sequence comprises one of the DNA sequences as represented by SEQ ID NOs:4, 6 and 8.

In another preferred aspect of embodiment (3), the nucleic acid sequence encodes a substitution, deletion and/or addition mutant ATP13A2, an ATP13A2 fragment or an ATP13A2 fusion protein as outlined above.

Embodiment (8) of present invention makes use of embodiment (7) in a method for testing candidate active agents for their therapeutic potential in diseases correlated to ATP13A2, preferably in neurodegenerative diseases, more preferably in Parkinsonism and dementia. Said method comprises the steps
(a) administering the candidate agent to a culture of the cell of embodiment (5), to a non-human organism a tissue or tissue culture of embodiment (6); and
(b) determining the effect of the candidate agent on said cell, non-human organism or tissue.

The method of embodiment (8) is not only apt for identification of active agents which interact directly with ATP13A2, but also for identification of active agents which influence the regulatory network controlling ATP13A2. Furthermore, said method allows identification of agents which substitute ATP13A2. Such ATP13A2 substitutes can be identified using cells/tissue/non-human transgenic organisms which do not produce ATP13A2 (like the knock-out mutants of embodiment (5)) or cells/tissue/non-human organisms who contain a partial or complete loss-of-function ATP13A2 mutant. The latter are preferably cells or primary tissue isolated from patients suffering from ATP13A2 loss of function, or cells/tissue derived from said isolated material, e.g. by immortalization.

Furthermore, the method of embodiment (8) is useful for the search of components of the regulatory network influencing ATP13A2 activity *in vivo.*

Whether a loss of ATP13A2 function *per se* leads to a significant lysosomal dysfunction can be determined by studies in immortalized or primary cell lines from KRS patients, knockout cells or knockout animal models. The present invention links ATP13A2 to the networks implicated in the etiology of parkinsonism and dementia. Moreover, it is the first finding of a etiologcal role of type 5 P type ATPases in pathogenesis.

Knockout cells, knockout tissue and knockout animals deficient of ATP13A2, especially knock-out mice, provide a unique *in vitro* and *in vivo* model for neurodegenerative disorders. These models can be used for testing candidate agents for therapy of said diseases. They can be produced by treatment of the cell, tissue or organism with an agent suppressing or reducing ATP13A2 expression or interfering with *ATP13A2,* including transformation or transfection with heterologous DNA, RNAi, gene targeting including conditional and inducible gene knock out, chemical and radioation mutagenesis. In case of non-human animals or tissue therefrom, the ATP13A2 deficiency is preferably generated by constitutive or inducible RNAi or by transgene or conditional gene knockout.

The antibody of embodiment (9) may be mono- or polyclonal. In a preferred aspect, the antibody is monoclonal. It can be prepared by textbook methods for antibody production, such as immunization of non-human animals and - if a monoclonal antibody is desired - subsequent formation of hybridoma cells. Such hybridoma cells producing said antibody of embodiment (9) are also embodiments of present invention.

The antibody of embodiment (9) is specific for the WT ATP13A2 and/or the mutant ATP13A2. In case it is specific for the WT and mutant ATP13A2, it is preferably binding to epitopes common to both forms, more preferably to epitopes in the N-terminal part of WT ATP13A2.

The antibody of embodiment (9) is in another aspect specific for the transmembrane domain(s) of ATP13A2, preferably for the transmembrane domain(s) lacking in certain mutant ATP13A2 of embodiment (2). Thus, it is useful for differentiating between the WT and the truncated ATP13A2 in immunological methods.

The pharmaceutical or diagnostic composition of embodiment (10) is useful for diagnosis, prevention or treatment of neurodegenerative diseases, more preferably of Parkinsonism and dementia, more preferably of Parkinson's disease and Alzheimer's disease. A method for preventing or treating neurodegenerative diseases, more preferably Parkinsonism and dementia in a patient, which method comprises administering to the patient an effective dose of one or more of the ATPase of embodiment (1), the nucleic acid of (3), the vector of (4), and the antibody of (9), or a pharmaceutically acceptable salt thereof is therefore a further aspect of present invention.

A therapy using the composition of embodiment (10) may be gene therapy; pharmacological modification of protein function, of splicing and/or of gene expression; and/or modification of gene expression by RNA interference.

Present invention is based on the finding that loss-of-function mutations in *ATP13A2* cause hereditary early-onset parkinsonism with dementia by mislocalization of truncated proteins to the ER and proteasomal degradation. The following paragraphs give a description of said finding:

A large non-consanguineous Chilean family was identified with early-onset PD strongly resembling the original Kufor-Rakeb family (example 1, table 1). By mutation screening (example 4) and linkage analysis (example 3) the known autosomal recessive PD genes/loci *Parkin*/PARK2 (Kitada, T. et al., Nature 392:605 (1998)), *DJ-*1/PARK7 (Bonifati, V. et al., Neurol. Sci. 24:159 (2003)), and *PINK1*/PARK6 (Valente, E.M. et al., Science 304:1158 (2004)) were excluded as causative for PD (data not shown). However, between the loci for PARK7 and PARK6 a linkage in a 23 cM spanning region bordered by microsatellite markers *D1S2736* and *D1S2644* was found (Fig. 1A). The maximum two-point and multipoint lod-scores were 2.68 (Tab. 2, example 3) and 3.64, respectively. This locus overlaps with the originally defined PARK9 locus and reduces the critical KRS interval to a 6.6 cM region between *D1S436* and *D1S2644.* As there was no obvious functional candidate gene among the nearly 40 known genes in this 3.2 MB region, genes were prioritized according to their expression in the brain (using the GNF Gene Expression Atlas 2, http://genome.ucsc.edu/). Mutation screening by PCR amplification with intronic primer pairs and direct sequencing of all 29 exons and splice sites of the predicted gene *ATP13A2,* which is located in the critical KRS-interval between *D1S2697* and *D1S3669* (Fig. 1A), revealed two different mutations in a compound heterozygous state in all affected family members. The first mutation (also present in the mother, I:2 in Fig. 1A) is a deletion of a single cytosine at nucleotide position 3057 in exon 26 leading to a frameshift and stop-codon after two extraneous amino-acids (c.3057delC or p.1019GfsX1121, Fig. 1B right side). The second mutation (inherited from the father, I:1 Fig. 1A) is a guanine to adenine transition at position +5 of the donor splice-site of exon 13 (c.1306+5G>A, Fig. 1B left side). This mutation of a highly conserved splice-site residue is predicted to cause a severe impairment of splicing efficacy, which drops from a score of 0.98 to 0.11 as assessed by a bioinformatical splice-site prediction (http://www.fruitfly.org). Direct cDNA amplification with primers located in exons 12 and 14 on blood samples of several family members revealed an aberrant smaller fragment in the father and two affected children which was not present in the mother or in control cDNA from human brain (Fig. 1D). Direct sequencing of the aberrant band demonstrated an in-frame skipping of exon 13 (removing 111 nucleotides) caused by the splice-site mutation (Fig. 1D). To confirm the causative role of *ATP13A2* mutations in KRS, mutation screening in the originally described Jordanian KRS family was performed. Analysis of exon 16 showed a homozygous 22 bp duplication in all affected family members (c.1632_1653dup22 or p.552LfsX788, Fig. 1C) leading to a frameshift and stop-codon after 236 extraneous amino-acids. All mutations cosegregated with the disease (data not shown) and were not found in 100 control chromosomes from German descent.

*ATP13A2* codes for a novel member of the P-type ATPase superfamily (Axelsen, K.B. and Palmgren, M.G., J. Mol. Evol. 46:84 (1998); Kuhlbrandt, W., Nature Rev. Mol. Cell. Biol. 5:282 (2004)) and shares the characteristic functional A- and P-domains as well as the predicted 10-transmembrane (TM) domain topology with other members of this class (Fig. 1E). The two frameshift mutations remove the C-terminal three and six TM domains, respectively, which is expected to cause a loss-of-function (the predicted topology model and location of mutations are shown in Fig. 1E). The in-frame skipping of exon 13 removes 37 amino-acids including half of TM domain M3 (Fig. 1E). The remaining hydrophobic residues of this domain will not be able to span the membrane which should also lead to a profound distortion of transmembrane topology and loss of protein function.

For present invention, the human gene sequence *ATP13A2* was determined by cDNA amplification and RACE-PCR. The open reading frame of the main isoform consists of 3543 nucleotides (SEQ ID NO:1) coding for a protein of 1180 amino acids (SEQ ID N0:2), the observed sequence is identical to the GenBank entry NM_022089. Several splice variants were identified with a region of complex splicing behaviour between exons 5 and 6 (data not shown). On the genomic level, the gene (SEQ ID NO:3) covers 26 kb and the coding region is divided into 29 exons.

To study the tissue distribution, a human multiple tissue northern blot was analyzed. *ATP13A2* is ubiquitously expressed as an approximately 3.8 kb transcript. The strongest expression is in brain, CNS and neurons (Fig. 2A), and of these the strongest expression is in brain, especially in the SN. Even more pronounced is the expression in DA neurons. The expression in brain is similar to the expression pattern in the mouse (Schultheis, P.J. et al., Biochem. Biophys. Res. Commun. 323:731 (2004)).

Dot blot analysis confirmed the predominant expression in human adult brain and demonstrated high expression in fetal brain and all tested subregions of the adult human central nervous system (Fig. 2B) including the SN (signal A3 in Fig. 2B). To elucidate the spatial expression pattern in brain, *in situ* hybridization in adult mouse brain sections was performed. Again, a broad neuronal expression including e. g. the cortex and the thalamus was found (Fig. 2C).

Because of the essential role of dopaminergic (DA) midbrain neurons for functions like voluntary movements and working memory and their crucial involvement in the etiology of parkinsonism (Liss, B. and Roeper, J., Trends Neuroscience 27(8):475-481 (2004)), the *ATP13A2* expression in individual DA neurons was studied (Fig. 2D). Using a patch-clamp based harvesting or photo-ablation and laser-microdissection system, single DA neurons were isolated from mouse midbrain slices or cryosections, respectively. The identity of individual neurons was confirmed by electrophysiology and molecular fingerprinting using single-cell PCR as previously described (Liss, B. et al., J. Neurosci. 19:8839 (1999); Liss, B. et al., Embo J. 20:5715 (2001); Liss, B., Nucleic Acids Res. 30:e89 (2002)). The expression profiles of marker genes were used to classify neurons as dopaminergic [positive for tyrosine hydroxylase *(Th)* and dopamine transporter *(Dat)]* and to exclude a possible contamination by glia cells [glial fibrillary acidic protein *(Gfap)]* and GABAergic neurons [glutamate decarboxylase *(Gad65*/*67)].* DA neurons were further subdivided into calbindin (Cbd28k)-negative SN cells, which represent the main site of neurodegeneration in PD, and calbindin-positive DA neurons from the neighbouring VTA. Qualitative PCR showed *Atp13a2* expression in pools of both SN and VTA dopaminergic neurons (Fig. 2D). In additional quantitative real-time RT-PCR experiments on human mRNAs from whole brain (WB), substantia nigra (SN), and cerebellum (CB) there were furthermore demonstrated higher *ATP13A2* mRNA levels in SN when compared to WB and even more pronounced to CB (Fig. 2E). This statistically significant increase was evident when expression levels were normalized to beta-actin (n=4; p=0.0006 SN/WB; p=0.00052 SN/CB) and to neuron-specific enolase (n=4; p=0.0014 SN/WB; p= 0.0005 SN/CB). This high level of *ATP13A2* expression in SN neurons points to an important functional role of ATP13A2 in the dopaminergic system and is in accordance with the parkinsonism observed in KRS.

To study their subcellular localization, ATP13A2 (encoded by SEQ ID NO: 1) and the KRS-mutants (encoded by SEQ ID NO:4, 6 and 8, respectively) were heterologously expressed in COS7 cells (example 8, Fig. 3). Visualization of ATP13A2 was achieved by a V5-antibody directed against an N-terminal V5-epitope tag. After transient transfection, an intracellular vesicular staining pattern for the WT-construct was observed (Fig. 3A). Co-staining with antibodies directed against different intracellular organelles revealed a specific co-localization with the lysosomal membrane proteins Lamp2 (Fig. 3A) and Lamp1 (data not shown). In contrast, no co-localization was observed with markers for mitochondria (MitoTracker^{™} , Fig. 3A), endosomes, the exocytotic pathway, and the Golgi apparatus (data not shown). Confocal microscopy of a GFP-tagged WT-construct and co-staining with the intralysosomal LysoTracker^{™} independently confirmed the localization of ATP13A2 in the lysosomal membrane in COS7 cells (Fig. 3B). In contrast, none of the three mutants did show a vesicular staining pattern. Instead they were localized in a network-like structure around the nucleus (Fig. 3C). By co-staining with an antibody against protein disulfide isomerase (PDI), a marker for the ER, the accumulation of KRS mutants in the ER was demonstrated (Fig. 3C). This retention of truncated and most probably misfolded proteins in the ER confirmed the pathogenic nature of identified KRS-mutations on a cellular level.

Mutated and misfolded proteins can be identified by the ER quality control machinery and are degraded by the ubiquitin-proteasome pathway (Rao, R.V. and Bredesen, D.E., Curr. Opin. Cell. Biol. 16:653 (2004)). To study a possible involvement of the ER-associated degradation (ERAD) pathway, the amount of WT and mutant proteins was analyzed by Western blotting with transiently transfected COS7 cells (example 11). The WT protein showed a strong signal with highest intensity in the range of 130-140 kDa which is in good accordance with its predicted molecular weight of 129 kDa (Fig. 4A). Also the three mutants showed bands in their predicted size ranges (predicted molecular weight is 125, 85, and 111 kDa for c.1306+5G>A, c.1632_1653dup22, and c.3057delC, respectively) confirming the effects of genetic alterations on the protein level (Fig. 4A). However, the amount of mutants was severely diminished when compared to WT and actin protein levels, which demonstrated the instability of all KRS mutants. This effect was especially pronounced for c.1306+5G>A, in which in-frame skipping of exon 13 and removal of half of TM domain M3 obviously lead to a severe protein folding defect and to a strong activation of ERAD. Inhibition of the proteasome by MG-132 (5 µM) enhanced the stability of KRS mutants although this stabilization was less effective in c.1306+5G>A (Fig. 4B). Interestingly, also the WT protein was stabilized by the treatment with MG-132 which was not seen in DMSO-treated control cells after 12 hours (Fig. 4B). This relative instability of ATP13A2 is supported by bioinformatical analysis, which shows an increased instability index (http://ca.expasy.org/cgi-bin/protparam). In summary, mislocalized KRS-mutants show an increased instability and are degraded by the proteasome confirming their causative involvement in a severe form of human neurodegeneration.

In addition to enhanced oxidative stress and mitochondrial dysfunction, one unifying theme in the pathogenesis of neurodegeneration is the disturbance of protein folding/aggregation and degradation (Cookson, M.R., Annu. Rev. Biochem. 74:29 (2005); Dawson, T.M. and V.L., Science 302:819 (2003); Selkoe, D.J., Nature Cell Biol. 6:1054 (2004)). Two major pathways are involved in proteolysis, namely the lysosome and the ubiquitin-proteasome system (Ciechanover, A., Nature Rev. Mol. Cell. Biol. 6:79 (2005)). Proteasomal dysfunction is a known cause of neurodegenerative disorders and mutations in parkin (Kitada, T. et al., Nature 392:605 (1998)), which acts as an E3 ubiquitin ligase, and in the ubiquitin C-terminal hydrolase gene (Leroy, E. et al., Nature 395, 451 (1998)) cause monogenic forms of PD. Also KRS-mutations in *ATP13A2* lead to protein retention in the ER and enhanced proteasomal degradation. This may at least in part explain the neurodegeneration in KRS e. g. by proteasomal dysfunction due to overload with mutant ATP13A2. Alternatively, insufficient proteolysis after many years of disease process may cause toxic protein aggregations, which - because of the especially high expression of *ATP13A2* in the SN - may first manifest as parkinsonism. Additionally, lysosomal dysfunction, which may be present in KRS due to the loss of ATP13A2 function, might lead to insufficient lysosomal protein degradation. In fact, there is increasing evidence for an important role of the lysosome in the etiology of PD as demonstrated by the degradation of alpha-synuclein by autophagy (Lee, H.J. et al., J. Neurosci. 24:1888 (2004); Webb, J.L. et al., J. Biol. Chem. 278:25009 (2003)) - and its impairment by PD-associated alpha-synuclein mutations (Cuervo, A.M. et al., Science 305:1292 (2004)) - or the identification of mutations in the lysosomal glucocerebrosidase gene in PD patients (Aharon-Peretz, J. et al., New Engl. J. Med. 351:1972 (2004); Goker-Alpan, O. et al., J. Med. Genet. 41:937 (2004)).

The present invention is described in more detail by reference to the following examples. It should be understood that these examples are for illustrative purpose only and are not to be construed as limiting the invention.

### Examples

In the following examples, standard techniques of recombinant DNA technology were used that were described in various publications, e.g. Sambrook et al. (1989), Molecular Cloning: A Laboratory Manual, Cold Spring Harbor Laboratory, or Ausubel et al. (1987), Current Protocols in Molecular Biology 1987-1988, Wiley Interscience. Unless otherwise indicated, all enzymes, reagents and kits were used according to the manufacturers' instructions.

### Example 1: Patients

A non-consanguineous Chilean family with 4 affected and 7 healthy children was investigated. All family members and the parents as legal guardians of the affected individuals gave their written informed consent. The study was approved by the local ethics committee of the University of Bonn. Both parents were healthy. All affected members but one (II-11) were normal at birth. The onset of the disease was around the age of 16 years (18, 17, 15 years for II-8, II-9, and II-10). Due to perinatal ischemia, II-11 developed an additional diffuse hypoxic cerebral damage, which made it difficult to ascertain the exact onset of PD symptoms. A summary of the neurological symptoms of affected members is shown in table 1.

**Tab. 1: Clinical phenotype of the four family members with Kufor-Rakeb disease in the chilean family**

| number in pedigree | age of onset | initial symptoms | parkinsonism | additional symptoms | mental status | ocular symptoms |
|---|---|---|---|---|---|---|
| II:8 | 18^{a} | school problems, slowing of movements | rigidity, tremor, bradykinesia, hypomimia | hyperreflexia, muscle cloni after testing of reflexes, enuresis, no sensory or cerebellar symptoms | dementia, hallucinations | upgaze paresis |
| II:9 | 17^{a} | slowing of movements | rigidity, tremor (inconstant and discreet), bradykinesia | hyperreflexia, muscle cloni after testing of reflexes, spacticity, hearing loss, no sensory or cerebellar symptoms | dementia, hallucinations | no |
| II:10 | 15^{a} | school problems, slowing of movements | rigidity, tremor (discreet), bradykinesia | hyperreflexia, muscle cloni after testing of reflexes, spacticity, no sensory or cerebellar symptoms | dementia, aggressive behaviour | upgaze paresis |
| II: 11^{b} | perinatal ischemia | not possible to be exactly defined | mild, difficult to assess | hyperreflexia, spastic progressive tetraplegia, choreoathetoid movements | mental retardation | no |

| | | | | | | |
|---|---|---|---|---|---|---|
| ^{a}pregnancy and birth as well as motoric and mental development was uneventful before this age ^{b}pregnancy was complicated, perinatal ischemia with subsequent hemiparesis and developmental retardation; later on he developed additional symptoms of a progressive neurodegeneration | | | | | | |

### Example 2: Genotyping

Genomic DNA was extracted from EDTA blood samples according to standard protocols. Highly polymorphic microsatellite markers (list of markers see Fig. 1A) were selected from candidate regions according to the Genethon and Marshfield maps and public data from the human genome project (http://www.ensembl.org/ and http://genome.ucsc.edu/). PCR amplification was performed using standard protocols and published microsatellite specific markers according to GDB (http://www.gdb.org). For microsatellite analyses equal volumes of PCR products and loading buffer were electrophorized for 2.5 h at 70 W on a 6% polyacrylamide gel. Gels were stained with AgN0₃ and analyzed by at least two independent persons.

### Example 3: Linkage analysis

The disease was analyzed as an autosomal recessive trait with a mutant allele frequency of 0.001. Two-point logarithm of odds (LOD) scores were calculated under the assumption of equal allele frequencies and using the computer MLINK program from the FASTLINK package version 5.1. (http://linkage.rockefeller.edu/soft/). Recombination frequencies were assumed to be equal in male and female subjects. Allele frequencies were assumed to be 1/N (N = number of different alleles observed on the pedigree). Multipoint LOD scores were calculated using GENEHUNTER.

The lod-score for microsatellite markers tested in the Kufor-Rakeb region of the Chilean familiy is shown in Tab. 2.

**Tab. 2: Two-point lod-score for different θ values for microsatellite markers tested in the Kufor-Rakeb region**

| Marker^{a} | 0.000 | 0.001 | 0.010 | 0.050 | 0.100 | 0.150 | 0.200 | 0.300 | 0.400 |
|---|---|---|---|---|---|---|---|---|---|
| D1S2736 | -∞ | -0.822 | 0.141 | 0.681 | 0.780 | 0.749 | 0.664 | 0.417 | 0.142 |
| D1S2667 | 1.175 | 1.173 | 1.155 | 1.073 | 0.963 | 0.845 | 0.718 | 0.437 | 0.149 |
| D1S228 | 2.680 | 2.675 | 2.624 | 2.394 | 2.101 | 1.800 | 1.492 | 0.860 | 0.274 |
| D1S507 | 2.680 | 2.675 | 2.624 | 2.394 | 2.101 | 1.800 | 1.491 | 0.858 | 0.271 |
| D1S436 | 2.680 | 2.675 | 2.628 | 2.414 | 2.137 | 1.849 | 1.550 | 0.921 | 0.309 |
| D1S2697 | 2.680 | 2.675 | 2.628 | 2.414 | 2.137 | 1.849 | 1.550 | 0.921 | 0.309 |
| D1S3669 | 2.680 | 2.675 | 2.632 | 2.434 | 2.174 | 1.899 | 1.608 | 0.985 | 0.349 |
| D1S1592 | 0.874 | 0.873 | 0.861 | 0.805 | 0.726 | 0.639 | 0.542 | 0.325 | 0.107 |
| D1S2644 | - ∞ | -0.023 | 0.927 | 1.404 | 1.420 | 1.303 | 1.125 | 0.679 | 0.222 |
| D1S552 | 0.874 | 0.873 | 0.861 | 0.805 | 0.726 | 0.639 | 0.542 | 0.325 | 0.107 |
| D1S199 | - ∞ | -2.722 | -0.769 | 0.415 | 0.740 | 0.806 | 0.760 | 0.502 | 0.171 |
| D1S2843 | 0.675 | 0.674 | 0.664 | 0.618 | 0.553 | 0.481 | 0.403 | 0.232 | 0.072 |
| D1S2732 | - ∞ | -5.721 | -2.762 | -0.852 | -0.188 | 0.092 | 0.213 | 0.220 | 0.087 |

| | | | | | | | | | |
|---|---|---|---|---|---|---|---|---|---|
| ^{a} relative order of markers according to the UCSC Human Genome Browser (http://genome.ucsc.edu) | | | | | | | | | |

### Example 4: Mutation screening and co-segregation analysis

Using public databases, candidate genes were selected from the critical region. Their genomic structure was delineated and a PCR-based testing strategy on genomic DNA was established. All exons were amplified by standard PCR on genomic DNA of one patient and one of the parents; primers see Tab. 3.

**Tab. 3: genomic primers**

| **Primer name** | **SEQ ID NO:** | **Primer sequence** |
|---|---|---|
| ATP1F | 10 | GGA CTG CGG CAG TGT CGG AGC C |
| ATP1sF | 11 | GAG CCT GGT GGC CCA GGT GC |
| ATP1sR | 12 | CGC TTC CTG GGC TCG CGA CC |
| ATP1R | 13 | GCT GCG AGC GGC GGC GCC ATC C |
| ATP2F | 14 | AGC AAG AGG AGA AGG TGC CTC C |
| ATP3R | 15 | GGT TGG CAC CCA AGC ATC CTC C |
| ATP4F | 16 | CCT TGA TTT CTG CCT TCT CCA GC |
| ATP5R | 17 | GCA CAG ATC ATG AAA CCG AGG C |
| ATP6F | 18 | CAG CAT AAG CAC ACT GCT TCT GG |
| ATP6R | 19 | CAT GCC ATG CCA CCG TCT GTG C |
| ATP7F | 20 | ATA GTC TCC AGC CTG CAC AGG G |
| ATP8R | 21 | CTG GTT GCC ACC GTA AAG TGG C |
| ATP9F | 22 | CAC AGG AGA CCC AGC CTG TGG C |
| ATP11R | 23 | CCT GGA CAA CAG AGT GAA ACT CCG |
| ATP12F | 24 | CCT CTC TGA GCC TCA GTG TTC C |
| ATP12R | 25 | GGC CCA ACT TCT GCT AAG ATG GG |
| ATP13F | 26 | TTG CAA CTC CAG AGG CAC GGG TCG |
| ATP15R | 27 | TAG ACA GGA CCT GGC ATC CTG TGG |
| ATP16F | 28 | AGA GGG AAT CAC ATG TGC CAA GG |
| ATP16sF | 29 | GCC CAG CTC AGC AGG GTA CC |
| ATP16sR | 30 | TGG GCA TAA TAA GAG ACC ACC C |
| ATP16R | 31 | CGT TAC AGG TGT GAG CCA CTG C |
| ATP17F | 32 | ACG TAT TCC AGG TCA CCC AAC AGC |
| ATP19R | 33 | ACC AGG CAG GGC ATC TTC CTT GG |
| ATP20F | 34 | CCA AGG AAG ATG CCC TGC CTG G |
| ATP20R | 35 | ATG GGT TCC TTG GAA GTC ACT CC |
| ATP21F | 36 | GGC AGA CGG ATT TCA TCC AGC C |
| ATP23R | 37 | GGA CAA GCT CAG TCT CCG CAG G |
| ATP24F | 38 | CCC AGC TGT CAT CAT ATT CTG CC |
| ATP25R | 39 | CCC ACG TCA TCT ATT CTG GGA CC |
| ATP26F | 40 | GGG CCT CTG CTA TCA GAC TAG G |
| ATP27R | 41 | GTG CAC GCC AGT CTT CCA CTC G |
| ATP28F | 42 | CGA GTG GAA GAC TGG CGT GCA C |
| ATP29R | 43 | TGG CTC AGA GGC AGG GAG TTC C |

Mutation analysis was performed by direct sequencing of each amplicon. The genomic structure of the *ATP13A2* gene was deduced using the human cDNA GenBank number NM_022089. Mutation numbering is based on the cDNA sequence of the GenBank entry NM_022089.1, position +1 corresponds to the A of the ATG translation initiation codon. The mutations found in this gene were tested for co-segregation in the corresponding family. The c.3057delC and c.1306+5G>A mutation in the Chilean family were tested by restriction enzyme analysis with *Rsa*I and Single-Strand Conformational Polymorphisms *(SSCP),* respectively. The c.1632_1653dup22 in the Jordanian family was tested on a 10% polyacrylamide gel to resolve both bands. A control sample consisting of 50 DNAs from healthy German individuals was tested for each of the mutations in the *ATP13A2* gene. In order to confirm the predicted splicing effect of the c.1306+5G>A mutation, total RNA from leucocytes was isolated from both parents of the Chilean family, one unaffected family member and two affected individuals using TRIzol reagent (Invitrogen) according to the manufacturer's recommendations. Approximately 1 µg of RNA was used to perform one step RT-PCR experiments (QIAGEN) according to the protocol supplied by the manufacturer. The cDNA primers were located in the flanking exons (cDNA primers 12F and 15R, see Tab. 4 in example 5).

### Example 5: Cloning of full length cDNA of ATP13A2

The putative coding sequence of *ATP13A2* was predicted using the public data from the human genome project (http://genome.ucsc.edu/). Overlapping primers were designed according to the predicted cDNA sequence (primer sequences see Tab. 4); PCR experiments on brain cDNA were performed.

**Tab. 4: cDNA primers**

| **Primer name** | **SEQ ID NO:** | **Primer sequence** |
|---|---|---|
| cATP1F | 44 | CAC GGA GGG ACT GCG GCA GTG TCG |
| cATP2F | 45 | TGC TGC GGT ATT ACC TCT TCC AGG |
| cATP3R | 46 | AGC GGT GGA CGT CGT CAC AAG AGC |
| cATP4F | 47 | CCT GGT GAG CTC CAT CTT GCA CC |
| cATP5R | 48 | GGA TGA AGA TGC TGT AGA TGG TGC |
| cATP6F | 49 | ATA CAG CTG CTG GCT ACC GTG TCG |
| cATP7R | 50 | GGC TTC AGT AGG TTC CTC ATG ACC |
| cATP8F | 51 | GGC AGA AGC CTC AGT GGT CTC ACC |
| cATP9R | 52 | GAA CTG GGT CAG GCT GTA CAG AGC |
| cATP10R | 53 | TCC AGT TGG TGG CTC AGA GGC AGG |
| cATP12F | 54 | GTG CTG AAG ACG GCA CTG CC |
| cATP15R | 55 | ACA CAC CAG CTG CAG CTT GC |

The amplicons obtained were directly sequenced in order to confirm the coding sequence and to detect possible splicing variants or polymorphisms. To confirm the putative start methionine, 5'-RACE (Rapid Amplification of cDNA Ends) experiments on Marathon-Ready cDNA (BD Biosciences Clontech) were performed according to the manufacturer's instructions.

### Example 6: Tissue distribution of ATP13A2

*ATP13A2* expression was first assayed by hybridization of a gene-specific probe located in the 3' region of the cDNA. The probe was radiolabeled by random priming with a-[³²]dCTP with the kit Ready-to-Go (Amersham Biosciences) using the protocol suggested by the manufacturer. The labelled probe was purified by a ProbeQuant G-50 Micro Column (Amersham Biosciences). The probe was obtained from human brain cDNA by using the primer pair primers cATP11F/ATP29R, which produced a PCR product of 420 bp. Hybridizations of the 12-Lane Human Multiple Tissue Northern (MTN) Blot (Clontech Laboratories) and Human Multiple Tissue Expression (MTE) Array (Clontech Laboratories) were performed according to the MTE protocol provided by the manufacturer. Briefly, both membranes were prehybridized in ExpressHyb hybridization solution (CLONTECH Laboratories Inc.) containing denatured salmon testes DNA for 30 min at 65 °C, then hybridized for 16 h at 65 °C with the labeled probe denatured in Cot-1 DNA (150 µg/ml; Roche Molecular Biochemicals), salmon testes DNA (750 µg/ml), 4x SSC at 95°C, then added to ExpressHyb. The MTE and MTN array were washed four times with 2x SSC, 1% SDS at 65 °C, twice with 0.1× SSC, 0.5% SDS at 55 °C, and then developed on x-ray film at -70°C for 1 and 4 days.

### Example 7: In situ hybridization

1 µg of a pCR-II TOPO Dual (Invitrogen) containing a 554 bp *Atp13a2* PCR fragment, corresponding to the 3'-end of the mouse cDNA, was linearized with *Bam*HI or *Eco*RV to generate sense or antisense cRNA probes. *In vitro* transcription was performed using the DIG RNA labeling Kit (Roche, Germany) following the manufacturer's recommendations. Integrity of the cRNA was verified by electrophoresis on agarose gels. *In situ* hybridization was performed on paraffin-embedded 7 µm brain coronal sections from C57BL/6J mice. After deparaffinization and hydration of sections, tissue was post-fixed to ensure attachment of sections to slides and incubated in 1% H₂O₂ to block endogenous peroxidase. Tissues were permeabilized by proteinase K digestion, Triton^{®} X-100 and HCl treatments. Following triethanolamine treatment, samples were hybridized with 1 µl of probe/ml of hybridization buffer at 70 °C overnight. Specimens were then subjected to stringent post-hybridization washings. DIG detection was performed using alkaline phosphatase-conjugated anti-DIG antibodies (Roche, Germany) and BCIP/NBT.

### Example 8: Laser-microdissection

A photo-ablation and laser-microdissection system with fluorescence option (PALM, Bernried, Germany) was used. 8-12 µm serial coronal cryosections of mouse midbrains were cut (Leica cryostat CM1850) and mounted on RNAse free UV-treated membrane slides (1 mm PEN-membrane, PALM). Sections were fixed with ethanol (75 %, 95% 100%, 100%), stained with cresyl violet in 100% ethanol, and dried. Neurons were visualized (63-fold objective), marked, cut and catapulted under brightfield (DIC). Individual neurons were catapulted directly into an adhesive cap (PALM). Mixture for cell-lysis (in 0.5% NonidetP40, Roche Diagnostics, and 20U SUPERaseIn, Ambion) and cDNA synthesis was added directly into the lid, the tube was incubated upside down for 90 s at 65 °C, and quickly cooled on ice prior to adding reverse transcriptase.

### Example 9: Real time-PCR

Electrophysiology, cytoplasm harvest and cDNA-synthesis, as well as qualitative and quantitative real-time single cell PCR were performed as described (Liss, B. et al., Embo J. 20:5715 (2001); Liss, B., Nucleic Acids Res. 30:e89 (2002)). For multiplex PCR, HotstarTaq (Qiagen), for nested PCR, RedTaq (Sigma) was used. Primer sequences for calbindin (Cb) D28k, tyrosine hydroxylase (Th), Gfap, and Gad65/67 were as described before (Liss, B. et al., J. Neurosci. 19:8839 (1999)), primers for mouse *Atp13a2* (Accession-No: NM_029097.1) were:
F1: TGACGACAGGGACGTCAAT, SEQ ID NO:56
R1: GGACGTCATCACAGGTACGA, SEQ ID NO:57 (PCR product 521 bp)
F2: CTCCTCAGCTTCATCCGTG, SEQ ID NO:58
R2: TTTCCATCCAGACGTAGCG, SEQ ID NO:59 (PCR-product 329 bp).
Primers for Gad65/67 (NM_008078.1) were:
F1: CATACGCAGACAGCACGTTT, SEQ ID NO:60
R1: AAAAGATTCCATCGCCAGAG, SEQ ID NO:61 (PCR product 904 bp)
F2: GGGATGTCAACTACGCGTTT, SEQ ID NO:62
R2: CACAAATACAGGGGCGATCT, SEQ ID NO:63 (PCR-product 388 bp).
Primers for dopamine transporter (Dat) (AF109072) were:
F1: TTCTCCTCTGGCTTCGTTGT, SEQ ID NO:64
R1: TGGATTGGGTGTGAACAGTC, SEQ ID NO:65 (PCR product 961 bp)
F2: CAACCTGTACTGGCGGCTAT, SEQ ID NO:66
R2: GCATAGGCCAGTTTCTCTCG, SEQ ID NO:67 (PCR-product 233 bp).

Human real-time PCR assays "by demand" (Applied Biosystems): beta-actin (ID 4326315E), VIC/MGB-labelled, Applied Biosystems primers and probes; human ATP13A2 (Hs00223032_m1, Accession: NM_022089) FAM/NFQ-labelled, Applied Biosystems primers and probes; ENO-2 (NM_001975.2): forward: GGCACTCTACCAGGACTTTGTCA (SEQ ID NO:80), reverse: GATCCCTACATTGGCT GTGAACTT (SEQ ID NO:81), probe (FAM/TAMRA): ACTATCCTGTGGTCTCCATT GAGGACCCATT (SEQ ID NO:82). Human first-strand cDNA (whole brain, substantia nigra, cerebellum) was purchased from Clontech, 10 µl of a 1:100 dilution was used for real-time PCR (50 µl reactions).

Statistics: All data are given as mean±S.E.M. To evaluate statistical significance (normal parametric data), appropriate Student's t-tests were performed. A value of p < 0.05 was considered to be statistically significant and indicated by asterics in Fig. 2E.

### Example 10: Generation of plasmid constructs

The approximately 3.6 kb coding sequence of human ATP13A2 was amplified from brain cDNA using the primer pair ATP1sF (GAGCCTGGTGGCCCAGGTGC; SEQ ID NO:11) and ATP29R (TGGCTCAGAGGCAGGGAGTTCC; SEQ ID N0:43) by AccuTaq LA DNA Polymerase (SIGMA). This fragment, including the stop-codon, was ligated into the mammalian expression vector pcDNA3.1/V5&His-TOPO (Invitrogen) and transformed into competent *Escherichia coli* TOP10 cells according to the manufacturer's instructions (Invitrogen). The stop-codon of the cDNA was deleted through recombinant PCR. Therefore, 2 mutagenesis primers were designed to modify the corresponding codon. Therewith a first round of two PCRs was made: the first one used the mutagenesis primer ATPdelstR (CGTGGGCCTGCACAACCTCAGG; SEQ ID NO:68) together with the cDNA primer cATP11F (AGTACCTCATCCTGGCTGCAGC; SEQ ID NO:69). The second one used the other mutagenesis primer V1+delstop (CCTGAGGTTGTGCAGGCCCACGAAGGGCAATTCTGCAGATATCC; SEQ ID NO:70) and BGHrev (TAGAAGGCACAGTCGAGG; SEQ ID NO:71). In this manner, two PCR fragments were obtained, both of them carrying the modified stop-codon and containing an overlapping region. Taking advantage of this overlap, in a second round of PCR both fragments were ligated. To do so, both amplicons were used as a template together with the primer pair cATP11F and BGHrev. Thus, the resulting amplicon could be inserted in the expression vector containing the *ATP13A2* with the stop-codon after restriction enzyme digestion of the vector and PCR fragment with *SbfI* and *SacII.* In this way, the cDNA coding for *ATP13A2* was in-frame with the V5 and *His* epitope for later experiments.

Concerning the disease causing mutations, recombinant PCR was used to introduce them into the V5 and *His* epitope tagged wild type (WT) construct. Briefly, for the generation of the c.1306+5G>A mutation again a first round of two PCR amplifications was made. The first one used the mutagenesis primer cATP12R (CTGTGCGGGTCACCACTGCCAGG; SEQ ID NO:72) together with the cDNA primer cATP12F (CCTCTCTGAGCCTCAGTGTTCC; SEQ ID NO:54); the second one used the mutagenesis primer cATP14F (CCTGGCAGTGGTGACCCGCACAGCTCTCCTCGG CACCATCTACAGC; SEQ ID NO:73) and the cDNA primer cATP7R (GGCTTCA GTAGGTTCCTCATGACC; SEQ ID NO:50). In the next step, both PCR fragments thus generated were used as a template for the second PCR using the primer pair cATP12F and cATP7R. The resulting amplicon, as well as the expression vector containing the tagged *ATP13A2,* were digested with the restriction enzymes *SgrAI* and *PshAI.* The selected restriction sites flanked the location of exon 13 of the *ATP13A2.* In this way, the mutation was inserted into the coding sequence of the construct.

For the generation of the c.1632_1653dup22 mutation again a first round of two PCR amplifications was made. The first one used the mutagenesis primer ATPins16R (GGCCCCACAGGCAGGCGGCAGGGGCCCCACAGGCAGGCGGCGAGG; SEQ ID NO:74) together with the cDNA primer cATP12F (CCTCTCTGAGCCTCAGTGTTCC; SEQ ID NO:54); the second one used the mutagenesis primer ATPins16F (GCCCCTGCCGCCTGCCTGTGGGGCCCCTGCTCCGAGCACTGGCCACC; SEQ ID NO:75) and the cDNA primer cATP7R (GGCTTCAGTAGGTTCCTCATGACC; SEQ ID NO:50). In the next step, both PCR fragments thus generated were used as a template for the second PCR using the primer pair cATP12F and cATP7R. The resulting amplicon, as well as the expression vector containing the tagged *ATP13A2,* were digested with the restriction enzymes *SgrAI* and *PshAI.* The selected restriction sites flanked the location of exon 16 of the *ATP13A2.* In this way, the mutation was inserted into the coding sequence of the construct.

For the generation of the c.3057deIC mutation (deletion of one Cytosine in exon 26) again a first round of two PCR amplifications was made. The first one used the mutagenesis primer ATP26delcR (GGTCAGGAAGTACCCCCTAGCTGC; SEQ ID N0:76) together with the cDNA primer cATP6F (ATACAGCTGCTGGCTACCGTGTCG; SEQ ID N0:49); the second one used the mutagenesis primer ATP26delcF (GCAGC TAGGGGGTACTTCCTGACC; SEQ ID N0:77) and the cDNA primer ATPdelstR (CGTG GGCCTGCACAACCTCAGG; SEQ ID NO:68). In the next step, both PCR fragments thus generated were used as a template for the second PCR using the primer pair cATP6F and ATPdelstopR. The resulting amplicon, as well as the expression vector containing the tagged *ATP13A2,* were digested with the restriction enzymes *BsrGI* and *SbfI.*

Because the c.1632_1653dup22 and c.3057delC mutations produced a frameshift in the open reading frame of *ATP13A2,* an early stop codon was produced in both constructs leaving both of them without an inframe-tag for detection. Therefore, recombinant PCR was performed in order to introduce the V5 epitope in-frame after the initial methione. To generate these constructs a first round of two PCR amplifications was made. The first one used the mutagenesis primer V5NHrev (CGTAGAATCGAGACCGAGGAGAGGGTTAGGGATAGGCTTACCCATGCCGGCTCCTCGCGCT CATCG; SEQ ID NO:78) together with the cDNA primer ATP1sF (GAGCCT GGTGGCCCAGGTGC; SEQ ID NO:11); the second one used the mutagenesis primer V5NHforw (GGTAAGCCTATCCCTAACCCTCTCCTCGGTCTCGATTCTACGAGCGCAGACAGCA GCCCTCTCGTGG; SEQ ID NO:79) and the cDNA primer cATP12R (CTGTG CGGGTCACCACTGCCAGG; SEQ ID NO:72). In the next step, both PCR fragments thus generated were used as a template for the second PCR using the primer pair ATP1sF and cATP12R. The resulting amplicon, as well as the expression vectors containing each of both mutant constructs, were digested with the restriction enzymes *NheI* and *SgrAI.* The selected restriction sites flanked the beginning of the coding sequence of the *ATP13A2.* In this way, the V5 epitope was inserted in the coding sequence of the construct.

Transformants bearing plasmids containing the full length WT *ATP13A2* cDNA fragment and all mutants were identified by restriction endonuclease analysis. Integrity of all inserts was confirmed by direct sequence analysis. All plasmid constructs were maintained in the *E. coli* (Invitrogen) transformants under ampicillin selection.

### Example 11: Cell culture and transfection

COS7 cells were maintained on Petri dishes or glass coverslips as monolayer cultures in DMEM medium (Dulbecco's modified Eagle's medium containing 10% fetal calf serum [Life Technologies-BRL], 100 mg of penicillin/ml, 100 U of streptomycin/ml [Life Technologies-BRL], and amphotericin B [Fungizone]) at 37 °C in an atmosphere of 5% CO₂/95% air. Transfections were performed using LipofectAMINE° 2000 (Invitrogen) according to the manufacturer's protocol. Briefly, 24 h before transfection cells were splitted in 60 mm Petri dishes at confluence of 80-90% and kept in medium without antibiotics. The transfection was made using 6 µg DNA and 10 µl LipofectAMINE^{®} 2000, the mixture was added to the Petri dish. For immunofluorescence, transiently transfected cells were cultured on glass-coverslips from 24 to 48 h. The cells were fixed with ice-cold methanol:acetone (1:1) for 5 min at room temperature. Fixed cells were permeabilized with 0,1% Triton^{®} X-100 in PBS for 10 min at room temperature. Nonspecific binding was prevented with blocking solution (3% Bovine Serum Albumin in 1X PBS [Sigma]) for 30 min at room temperature. ATP13A2 was detected with anti-V5 antibody (Invitrogen) at a dilution of 1:500. ER and lysosomes were visualised through anti-PDI (Stressgen) and anti-H4B4 (Hybridome) antibodies respectively at a dilution of 1:250. Anti-V5 was directly labeled by Zenon Alexa Fluor 568 mouse IgG₂ₐ kit and anti-PDI/anti-H4B4 were labeled by Zenon Alexa Fluor 488 Mouse IgG₁ kit (Molecular Probes) according to the manufacturer's instructions. Briefly, antibodies were incubated with the fluorophore-labeled Fc-specific anti-mouse IgG Fab fragment for 5 min (Zenon labeling Complex formation). Not bound fragments were blocked by adding Zenon blocking reagent for 5 min. Freshly prepared Zenon labeling complex was diluted to a final dilution of 1:40 in blocking solution, which was directly applied to the coverslips and incubated at room temperature for 1 h. Coverslips were thoroughly washed in PBS for 5 min three times before fixation in 4% formaldehyde in 1X PBS for 10 min at room temperature. Coverslips were then washed twice with 1X PBS and mounted in antifade mounting medium containing DAPI (Vector Laboratories Inc.). Cells were imaged with a Zeiss Axioplan2 microscope with appropriate excitation/emission filter pairs. For mitochondrial labeling, cells were loaded before fixation with MitoTracker Orange CM-H₂TMROS (Molecular Probes) at a concentration of 0.5 ng/µl of growth media for 30 min at 37 °C. The staining for this coverslips was carried out as described above, but the V5-Antibody was labeled with the Zenon Alexa Fluor 488 Mouse IgG₂ₐ kit. For confocal imaging, cell were transiently transfected with 3 mg of ATP13A2-EGFP plasmid on glass coverslips and 24 h after transfection were mounted in a perfusion chamber for observation. The cells were incubated with 100 nM LysoTracker Red DND-99 (Molecular Probes) for 10 min at room temperature, washed and analyzed with a Zeiss model LSM 510 confocal microscope equipped with Ar (488 nm) and HeNe (543 nm) lasers. The excitation of EGFP and LysoTracker was done simultaneously by the 488 and 543 nm laser beam by single-track function. The images were processed using Huygens Professional deconvolution software.

### Example 12: Western Blot Analysis

Culture dishes were rinsed once with cold phosphate-buffered saline. Cells were collected into PBS and samples were centrifuged at 13000 rpm for 10 min at 4 °C. The supernatant was discarded and the resulting pellet was resuspended in an appropriate amount of loading buffer (2x NuPAGE LDS sample Buffer [Invitrogen] and 1x Complete Mini protease inhibitor cocktail [Roche]). Cell lyses were made using an ultrasonic bath. Volumes of 15 µl of each probe including 100 mM dithiothreitol (DTT) were boiled at 95 °C for 5 min and were subjected to SDS-PAGE. After protein separation on 3-8 % Tris-Acetate or 4-12 % Bis-Tris gels at 200 V for 45 min proteins were transferred to PVDF membranes (Hybond-P; Amersham Biosciences) for 1 h at 32 V. Nonspecific binding of antibody was prevented by blocking blots for 1 h with 2.5 % low-fat dry milk in PBS containing 0.1 % Tween 20 (PBST), followed by incubating in PBST with 2.5 % low-fat dry milk including the primary antibody of choice in an appropriate concentration according to the manufacturer's suggestion under continual rotation for 1 h at room temperature or overnight at 4 °C. The blots were washed for 15 min in PBST and subjected to PBST containing 2.5 % low-fat milk and anti-rabbit and anti-mouse horseradish peroxidase-conjugated secondary antibodies (dilution 1:5000). Incubation lasted 1 h, afterwards blots were washed 3 times, each wash 20 min, in PBST and visualized via an enhanced chemiluminescence kit (ECL) according to the manufacturer's suggested protocol (Pierce). Membranes were then exposed to x-ray film for 10 and 60 min. For semi-quantification of the amount of loaded protein, blots were incubated with anti-β-actin as a second primary antibody.

## Claims

1. A method for prognosing, diagnosing and/or monitoring a disorder **characterized by** changes in the expression and/or translation of the lysosomal type-5 P type ATPase ATP13A2 ("wild type ATP13A2") or by the presence of a loss-of-function mutant of ATP13A2 ("mutant ATP13A2") in a biological sample isolated from a human subject, which method comprises detection of
(i) the amount and/or activity of wild type ATP13A2 and/or of mutant ATP13A2 in the sample; and/or
(ii) the nucleic acid sequence coding for wild type ATP13A2 or for mutant ATP13A2 in the sample, and/or
(iii) mutations present in the ATP13A2 nucleic acid sequence of the sample in comparison to the wild type ATP13A2 nucleic acid sequence.

2. The method of claim 1, wherein the disorder is a neurodegenerative disease, more preferably is Parkinsonism or dementia, most preferably is Parkinson's disease or Alzheimer's disease.

3. The method of claim 1 or 2, wherein the detection is performed by
(i) a hybridisation assay or PCR-based assay apt for specific detection of *ATP13A2* mutations, preferably for detection of deletion mutants, more preferably for detection of one of the DNA sequences as represented by SEQ ID NOs:4, 6 and 8; or
(ii) an immunoassay using at least one antibody specific for (a) the wild type ATP13A2, (b) the mutant ATP13A2, and/or (c) one or more of the C-terminal transmembrane (TM) domains of wild type ATP13A2.

4. An isolated loss-of-function mutant protein of the human lysosomal type-5 P type ATPase ATP13A2 ("mutant ATP13A2"), which is causative for a disorder, especially for a neurodegenerative disease, more preferably for Parkinsonism or dementia, most preferably for Parkinson's disease or Alzheimer's disease.

5. The isolated mutant ATP13A2 of claim 4, which
(i) is localized in the endoplasmatic reticulum *in vivo* and *in vitro;* and/or
(ii) is not localized in the lysosome of the cell of its origin *in vivo* and *in vitro;* and/or
(iii) has a reduced *in vivo* stability in comparison to the wild type ATP13A2.

6. The isolated mutant ATP13A2 of claim 4 or 5, which
(i) is a deletion mutant of the wild type ATP13A2, preferably a deletion mutant wherein at least one of the C-terminal transmembrane (TM) domains is deleted, more preferably at least the C-terminal three TM domains are deleted, even more preferably the C-terminal six TM domains are deleted; and/or
(ii) contains one of the amino acid sequences as represented by SEQ ID N0s:5, 7 and 9.

7. An isolated nucleic acid sequence encoding the mutant ATP13A2 as defined in any one of claims 4 to 6.

8. The isolated nucleic acid sequence of claim 7 which
(i) is expressed in neurons, preferably in brain, more preferably in the substantia nigra; and/or
(ii) comprises one of the DNA sequences as represented by SEQ ID NOs:4, 6 and 8.

9. A vector comprising the nucleic acid sequence of any one of claims 7 or 8.

10. A cell which
(i) is transfected or transformed with the vector of claim 7 and/or
(ii) heterologously comprises the nucleic acid sequence of any one of claims 7 or 8 and/or
(iii) is capable of heterologously expressing a mutant protein as defined in claims 4 to 6; or
(iv) is a knock-out mutant not producing ATP13A2.

11. An isolated tissue, tissue culture or a non-human transgenic organism comprising one or more of the cells of claim 10.

12. Use of the cell of claim 10, or the non-human transgenic organism, the tissue or tissue culture of claim 11 as a test cell system, test tissue or test model organism in testing candidate active agents for their therapeutic potential in diseases correlated to ATP13A2, preferably in neurodegenerative diseases, more preferably in Parkinsonism and dementia, most preferably in Parkinson's disease and Alzheimer's disease.

13. A method for testing candidate active agents for their therapeutic potential in diseases correlated to ATP13A2, preferably in neurodegenerative diseases, comprising the steps
(a) administering the candidate agent to a culture of the cell of claim 10, or to a tissue or tissue culture or to the non-human transgenic organism of claim 11; and
(b) determining the effect of the candidate agent on said cell, tissue or non-human organism.

14. An antibody specific for (i) the wild type ATP13A2 , (ii) the mutant ATP13A2 of anyone of claims 4 to 6, or (iii) the transmembrane domain(s) lacking in the mutant protein of anyone of claims 4 to 6.

15. Use of one or more component(s) of the group consisting of wild type ATP13A2, a nucleic acid encoding said wild type ATP13A2, and/or the antibody of claim 14, and a pharmaceutically acceptable salt thereof, for preparing a medicament for prevention or treatment of neurodegenerative disorders.

16. A pharmaceutical or diagnostic composition comprising one or more of wild type ATP13A2, a nucleic acid encoding said wild type ATP13A2, and/or the antibody of claim 14, or a pharmaceutically or diagnostically acceptable salt thereof.

17. A kit for performing the method of claim 1, comprising anyone of the mutant ATP13A2 of claims 4 to 6, the nucleic acid of claim 7 or 8, the vector of claim 9, the cell of claim 10, the antibody of claim 14, and/or primers or probes for a hybridisation assay or PCR-based assay apt for specific detection of *ATP13A2* mutations.
